# EUROPEAN PATENT SPECIFICATION

(11) **EP 3 833 674 B1**
(45) Date of publication and mention of the grant of the patent: **07.10.2026**
(21) Application number: 19762483.6
(22) Date of filing: 09.08.2019
(51) Int. Cl.: C07K 7/06, A61K 8/64, A61Q 19/00, A61Q 19/08, A61K 9/00, A61K 9/107, A61K 9/06, A61K 47/10

(54) **COMPOUNDS USEFUL FOR THE TREATMENT AND/OR CARE OF THE SKIN, HAIR, NAILS AND/OR MUCOUS MEMBRANES**
VERBUNDSTOFFE, DIE ZUR BEHANDLUNG UND/ODER PFLEGE DER HAUT, HAARE UND/ODER SCHLEIMHÄUTE NÜTZLICH SIND
COMPOSÉS UTILES AU TRAITEMENT ET/OU AU SOIN DE LA PEAU, DES CHEVEUX, DES ONGLES ET OU DES MUQUEUSES

(30) Priority: 10.08.2018 EP 18382605
(43) Date of publication of application: 16.06.2021
(73) Proprietor: Lubrizol Advanced Materials, Inc., Cleveland, OH 44141-3247 (US)
(72) Inventor: DELGADO, Raquel, 088560 Gavà (ES); ALMIÑANA, Núria, 08030 Barcelona (ES); SOLEY, Albert, 08205 Barcelona (ES); LIDÓN, Maria del Carmen, 03349 Alicante (ES); RODRÍGUEZ, Catalina, 08004 Barcelona (ES); MOLA, Gemma, 08440 Barcelona (ES); VALERIO, Mauricio, 11405 Cádiz (ES); GARCIA, Consuelo, 30565 Murcia (ES)
(74) Representative: Bradley, Josephine Mary
(86) International application number: PCT/IB2019/056796
(87) International publication number: WO 2020/031146

(56) References cited:
- WO-A1-2009/155789
- WO-A1-2013/153196
- WO-A1-2017/100421
- CN-A- 109 908 024
- KR-A- 20120 099 550
- US-A1- 2010 069 264

## Description

### FIELD OF THE INVENTION

The invention relates to compounds useful for the treatment and/or care of the skin, hair, nails and/or mucous membranes. In particular, the compounds are useful for the prevention of skin aging and, in particular, for the treatment and/or prevention of skin wrinkles, the treatment and/or prevention of a sagging appearance of the skin, and/or the reduction and/or prevention of facial asymmetry. The invention extends to compositions comprising the compounds and methods of treatment using the compounds.

### BACKGROUND OF THE INVENTION

The effects of aging play a major role in skin appearance. The most striking signs of facial aging are wrinkles and a sagging appearance of the face which is related to the aging of muscle. With age, the epidermis and connective tissue of the skin become weak, facial muscular mass diminishes, the epidermis begins to loosen and drop, the natural folds in the cheek, neck and chin area change and there is a redistribution and loss of facial fat (adipose tissue).

Muscle aging is a well-known process which can start in humans when they are around 40 years old and it accelerates in later years. The resultant loss of muscle tone and thinning skin can give the face a loose, sagging appearance. The jawline loses its contour, and the profile of the face becomes less defined.

Formation of facial wrinkles is directly related to tensing of the muscles of the epidermis that act to drag the skin inwards. This muscular tension is the result of hyperactivity of the nerves innervating the facial muscles. Nerve hyperactivity is characterized by the uncontrolled and excessive release of neurotransmitters that excite muscle fibers. Neuronal exocytosis is a strongly regulated process in which, mainly, the formation of the protein complex known as SNARE complex is involved. The nucleus of said fusion complex is made up of the proteins SNAP-25 and syntaxin, located in the presynaptic plasma membrane, and the protein synaptobrevin (or VAMP), located in the vesicular plasma membrane. The main function of the fusion complex is to bring the vesicle loaded with neurotransmitter (acetylcholine) nearer to the presynaptic plasma membrane and put it in contact with the same. In this way, in response to an elevated concentration of calcium, the fusion of both plasma membranes is encouraged, thus producing the release of the neurotransmitter.

Since the release of neurotransmitters is associated with neuronal exocytosis, control of neuronal exocytosis can contribute to the relaxation of muscular tension and, thus, the reduction of wrinkles. Cleaving any of the proteins that make up the SNARE complex prevents its assembly, and therefore this is a key target method for controlling neuronal exocytosis. Botulinum toxins are a family of bacterial neurotoxins produced by *Clostridium botulinum.* Botulinum toxins are proteases that degrade neuronal proteins involved in the exocytosis mechanism activated by the calcium ion. For example, botulinum toxin A, the most commonly used clinically and cosmetically, cleaves the neuronal protein SNAP-25. Therefore, Botulinum toxins, particularly serotype A (BOTOX^{®}, Allergan Inc; Dysport^{™}, Ipsen Bipharm, Ltd. And Azzalure^{®}, Galderma, S.A), are widely used as an effective agent for reducing facial wrinkles and asymmetry. In fact, the administration of Botulinum toxin is the first effective non-surgical therapy to be used to eliminate the signs of aging. However, in the cosmetic field, there is an emerging belief that long-term use of Botulinum toxin could also have a cosmetically undesirable impact on facial muscles, which may lead to a sagging appearance of the skin and thus an increased senescent appearance of the face [Durand, PD. "Botulinum Toxin and Muscle Atrophy: A Wanted or Unwanted Effect", (2016), Aesthetic Surgery Journal, Vol 36(4), pp. 482-487].

It is known is the art that certain peptides can imitate the effects of the botulinum toxins in that they inhibit neuronal exocytosis, e.g. the peptides derived from the amino end of protein SNAP-25 disclosed in WO2000/64932A1. One such peptide is acetyl hexapeptide-3, commercialized under the tradename Argireline^{®} by Lipotec, S.A. Use of the Argireline^{®} peptide (Ac-Glu-Glu-Met-Gln-Arg-Arg) in skin whitening is disclosed in KR20120099550A. However, the peptide is erroneously assigned the sequence Arg-Arg-Gln-Met-Glu-Glu-acetyl in the abstract of KR20120099550A. Consistent with the usual convention for writing peptide sequences and, indeed, paragraph [0007] of KR20120099550A, this sequence should be read as Acetyl-Glu-Glu-Met-Gln-Arg-Arg.

WO 2013/153196 relates to compounds capable of inhibiting neuronal exocytosis and to the treatment of those conditions which require the inhibition of neuronal exocytosis.

There is a need to find novel active compounds that can alleviate or prevent the signs of skin aging. In particular, there is a need to find novel active compounds that can prevent or reduce skin wrinkles and/or a sagging appearance of the face. There is a need to find novel active compounds that can mimic the neuronal exocytosis inhibition behaviour of Botulinum toxins. Indeed there is a need to provide novel active compounds that mimic the neuronal exocytosis inhibition behaviour of Botulinum toxins better than those of known alternatives to Botulinum toxins such as the Argireline^{®} peptide.

The present invention sets out to meet some or all of these needs and to solve some or all of the above-identified problems.

### SUMMARY OF THE INVENTION

In a first aspect, the invention provides a compound represented by formula (I):

R₁-Wₘ-Xₙ-AA₁-AA₂-AA₃-AA₄-AA₅-AA₆-Yₚ-Z_{q}-R₂ (I),

its stereoisomers and/or its cosmetically acceptable salts, wherein:
AA₁ is Arg, Lys or no amino acid;
AA₂ is Arg or Lys;
AA₃ is Gln or Asp;
AA₄ is Met or Leu;
AA₅ is Glu, Asp or Gln;
AA₆ is Glu, Asp, Gln or no amino acid;
AA₁ is different from AA₆;
W, X, Y and Z are each independently an amino acid selected from the group consisting of Ala, Gly, Val and Ile;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl;
R₁ and R₂ are not amino acids; and
either (i) each of AA₁, AA₂, AA₃, AA₄, AA₅ and AA₆ is an L-amino acid, or (ii) 1, 2 or 3 of AA₃, AA₄ and AA₅ is a D-amino acid and the remaining of AA₁ to AA₆ are L-amino acids.

It has been found that compounds of formula (I) are effective in inhibiting noradrenaline release and increasing collagen synthesis in the skin. Further, compounds of the invention have been found to be effective in increasing the amount of lipids in adipose cells and thus effective in increasing facial volume. They are thus useful as skin antiaging agents and can mimic the inhibiton of neuronal exocytosis behaviour of Botulinum toxins used to treat the symptoms of skin aging. Furthermore, surprisingly, it has been found that certain compounds of the invention can upregulate expression of muscleblind-like1 (MBNL-1), a highly conserved RNA-binding protein which plays an important role in the process of trans-differentiation from fibroblasts to myofibroblasts. It follows that these peptides are particularly useful for preventing or alleviating cosmetic properties of the skin associated with loss of muscle tone due to muscle aging and, indeed, alleviating cosmetically undesirable side effects of anti-aging treatments involving Botulinum toxin injections. Compounds of the invention have been demonstrated to show improved performance over the known peptide alternative to Botulinum toxin, Argireline^{®}.

In another aspect, the invention provides a cosmetic composition comprising a compound of formula (I), its stereoisomers and/or its cosmetically acceptable salts, together with at least one cosmetically acceptable excipient or adjuvant.

In another aspect, the invention provides the use of a compound of formula (I), its stereoisomers and/or its cosmetically acceptable salts, or a composition comprising a compound of formula (I), its stereoisomers and/or its cosmetically acceptable salts, for the treatment and/or care of the skin, hair, nails and/or mucous membranes. In particular, the invention provides the use of a compound of formula (I), its stereoisomers and/or its cosmetically acceptable salts, or a cosmetic composition comprising a compound of formula (I), its stereoisomers and/or its cosmetically acceptable salts, for the cosmetic, non-therapeutic treatment and/or care of the skin, hair, nails and/or mucous membranes. The cosmetic, non-therapeutic treatment and/or care can be: the prevention or the treatment of the symptoms of skin aging; the treatment and/or prevention of skin wrinkles; the stimulation of collagen synthesis and/or prevention of collagen loss; the improvement or maintenance of skin firmness; the treatment and/or prevention of a sagging appearance of the skin; and/or the treatment or prevention of facial asymmetry. The cosmetic, non-therapeutic treatment and/or care can be the increase of the volume of adipose tissue and/or the prevention and/or alleviation of the effects of adipose tissue loss.

In another aspect, the invention provides a method of cosmetic, non-therapeutic treatment and/or care of the skin, hair, nails and/or mucous membranes in a subject comprising administering a cosmetically effective amount of a compound of formula (I), its stereoisomers and/or its cosmetically acceptable salts, or a cosmetic composition comprising same, to the subject. Typically, the compound will be administered topically. The cosmetic, non-therapeutic treatment and/or care can be: the prevention or the treatment of the symptoms of skin aging; the treatment and/or prevention of skin wrinkles; the stimulation of collagen synthesis and/or prevention of collagen loss; the improvement or maintenance of skin firmness; the treatment and/or prevention of a sagging appearance of the skin; and/or the treatment or prevention of facial asymmetry. The cosmetic, non-therapeutic treatment and/or care can be the increase of the volume of adipose tissue and/or the prevention and/or alleviation of the effects of adipose tissue loss.

In another aspect, the invention provides a kit for use in a cosmetic, non-therapeutic method of treatment and/or care of the skin comprising:
(i) a composition comprising Botulinum toxin;
(ii) optionally a composition comprising Ac-Glu-Glu-Met-Gln-Arg-Arg-NH₂, or H-Tyr-D-Ala-Gly-Phe-Leu-OH or combinations thereof; and
(iii) a cosmetic composition comprising a compound of formula (I).

### BRIEF DESCRIPTION OF THE DRAWINGS

Figure 1 shows the mean of percentage of noradrenaline (NA) release with respect to basal conditions in SH-SY5Y for 3 assays (Example 6).
Figure 2 shows the mean of percentage of collagen type I respect to basal conditions for 5 assays (Example 7).
Figure 3 shows the mean of percentage of MBNL1 respect to basal conditions in hSkMc for 3 assays (Example 8).
Figure 3a shows the mean of percentage of MBNL1 respect to basal conditions in hSkMc for 3 assays (Example 8). Abbreviations: BC = basal control (without treatment)
Figure 4 shows the percentage of muscle mass loss in human skeletal muscle cells measured using an immunofluorescence assay. The measurements are made on samples of human skeletal muscle cells treated with tumor necrosis factor alpha (TNFα) and either (i) treated with a compound of the invention or (ii) not treated with a compound of the invention. Results are also compared with human skeletal muscle cells that are neither treated with TNFα nor treated with a compound of the invention (Example 17). Abbreviations: BC+TNF = Basal control with 20mg/ml of TNFα; BC = Basal control.
Figure 5 shows levels of lipid accumulation in human subcutaneous pre-adipocytes as determined by fluorescent staining (Example 19). The measurements are made on samples of a coculture of young and old pre-adipocytes that have been treated with a compound of the invention or that have not been treated with a compound of the invention. Results are also compared with a culture of old pre-adipocytes. Abbreviations: % LA = Percentage of lipid accumulation; CC = Coculture control; YC = young control; OC = old control.

### DETAILED DESCRIPTION OF THE INVENTION

### Definitions

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes, mast cells, neurones and/or adipocytes among others. The term "skin" also comprises the scalp. The term "skin" includes the skin of mammals and includes human skin. Likewise, the terms "hair, nails and mucous membranes" include the hair, nails and mucous membranes of mammals, for example humans.

When the terms "treatment" and "care" are accompanied by the qualifications "cosmetic, non-therapeutic", it means that the treatment or care has the aim of improving or maintaining the aesthetic appearance of the skin, hair, nails and/or mucous membranes. In particular, the treatment can have the aim of improving cosmetic properties of the skin, hair, nails and/or mucous membranes such as, for example and not restricted to, the level of hydration, elasticity, firmness, shine, tone or texture, which properties affect the aesthetic appearance of the skin, hair, nails and/or mucous membranes. The term "care" in the context of this specification refers to the maintenance of properties of the skin, hair, nails and/or mucous membranes. Said properties are subject to being improved or maintained by cosmetic treatment and/or care of the skin, hair, nails and/or mucous membranes both in healthy subjects as well as in those which present diseases and/or disorders of the skin, hair, nails and/or mucous membranes.

The term "prevention", as used in this invention, refers to the ability of a compound of the invention to prevent, delay or hinder the appearance or development of a disease or disorder, or to prevent, delay or hinder the change in a cosmetic property of the skin, mucous membranes and/or hair. The term "prevention", as used in this invention, is interchangeable with the term "inhibition", i.e. it refers to the ability of a compound of the invention to inhibit the appearance or development of a disease or disorder, or to inhibit the change in a cosmetic property of the skin, hair, nails and/or mucous membranes.

In the context of this invention, the term "aging" refers to the changes experienced by the skin as the result of intrinsic aging process (i.e. chronoaging) or extrinsic skin aging process induced by environmental factors (i.e. through exposure to the sun (photoaging) or to environmental agents such as tobacco smoke, extreme climatic conditions of cold or wind, chemical contaminants or pollutants). In the context of the invention, aging includes all the external visible and/or perceptible changes through touch, such as and not restricted to, the development of discontinuities on the skin such as wrinkles, fine lines, expression lines, stretch marks, furrows, irregularities or roughness, increase in the size of pores, loss of hydration, loss of elasticity, loss of firmness, loss of smoothness, loss of the capacity to recover from deformation, loss of resilience, sagging of the skin such as sagging cheeks, the appearance of bags under the eyes or the appearance of a double chin, among others, changes to the color of the skin such as marks, reddening, bags or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and it presents the same physical characteristics as aging, such as and not restricted to, flaccidity, sagging, changes to the color or irregularities in the pigmentation, abnormal and/or excessive keratinization. The sum of various environmental factors such as exposure to tobacco smoke, exposure to pollution, and climatic conditions such as cold and/or wind also contribute to the aging of the skin.

In this description, the abbreviations used for amino acids follow the rules of IUPAC-IUB Commission of Biochemical Nomenclature specified in Eur. J. Biochem., (1984), 138, 9-37. Thus, for example, Gly represents NH₂-CH₂-COOH, Gly- represents NH₂-CH₂-CO-, -Gly represents -NH-CH₂-COOH and -Gly- represents -NH-CH₂-CO-. Therefore, the hyphen, which represents the peptide bond, eliminates the OH in the 1-carboxyl group of the amino acid (represented here in the conventional non-ionized form) when situated to the right of the symbol, and eliminates the H of the 2-amino group of the amino acid when situated to the left of the symbol; both modifications can be applied to the same symbol (see Table 1).

**Table 1**

| **Table 1. Structures of the amino acid residues, their nomenclature in three-letter code and nomenclature for the amino acids in one letter code** | | | |
|---|---|---|---|
| Name | Residue | Symbol | Residue |
| Arginyl | 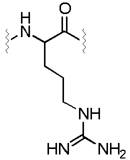 | Lysyl | 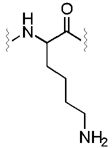 |
| -Arg- | | -Lys- | |
| R | | K | |
| Glutaminyl | 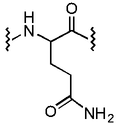 | Glutamyl | 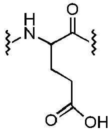 |
| -Gln- | | -Glu- | |
| Q | | E | |
| Asparaginyl | 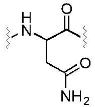 | Methionyl | 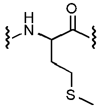 |
| -Asn- | | -Met- | |
| N | | M | |
| Leucyl | 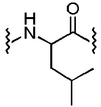 | Aspartyl | 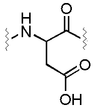 |
| -Leu- | | -Asp- | |
| L | | D | |
| Alanyl | 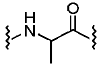 | Valyl | 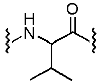 |
| -Ala- | | -Val- | |
| A | | V | |
| Isoleucyl | 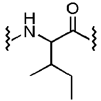 | Glycyl | 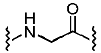 |
| -lle- | | -Gly- | |
| I | | G | |

As used herein, the term "non-cyclic aliphatic group" includes linear (i.e. straight and unbranched) or branched, saturated or unsaturated hydrocarbyl groups such as alkyl, alkenyl and alkynyl. The non-cyclic aliphatic group may be substituted (mono- or poly-) or unsubstituted.

As used herein, the term "alkyl" includes both saturated linear and branched alkyl groups, which may be substituted (mono- or poly-) or unsubstituted. The alkyl group is bound to the rest of the molecule by a single bond. The alkyl group has from 1 to 24, preferably from 1 to 16, more preferably from 1 to 14, even more preferably from 1 to 12, yet more preferably 1, 2, 3, 4, 5 or 6 carbon atoms. The term "alkyl" includes, for example, methyl, ethyl, isopropyl, isobutyl, *tert*-butyl, 2-methylbutyl, heptyl, 5-methylhexyl, 2-ethylhexyl, octyl, decyl, dodecyl, lauryl, hexadecyl, octadecyl and amyl.

As used herein, the term "alkenyl" refers to a group containing one or more double carbon-carbon bonds and which may be linear or branched and substituted (mono- or poly-) or unsubstituted. Preferably it has 1, 2 or 3 double carbon-carbon bonds. If more than one double carbon-carbon bond is present, the double bonds may be conjugated or not conjugated. Preferably the alkenyl group has from 2 to 24, preferably from 2 to 16, more preferably from 2 to 14, even more preferably from 2 to 12, yet more preferably 2, 3, 4, 5 or 6 carbon atoms. The alkenyl group is bound to the rest of the molecule by a single bond. The term "alkenyl" includes, for example, vinyl (-CH₂=CH₂), allyl (-CH₂-CH=CH₂), prenyl, oleyl, linoleyl groups and similar.

The term "alkynyl" refers to a group containing one or more triple carbon-carbon bonds and which may be linear or branched, and substituted (mono- or poly-) or unsubstituted. Preferably the alkynyl group has 1, 2 or 3 triple carbon-carbon bonds. The triple bonds may be conjugated or not conjugated. The alkynyl group has from 2 to 24, preferably from 2 to 16, more preferably from 2 to 14, even more preferably from 2 to 12, yet more preferably 2, 3, 4, 5 or 6 carbon atoms. The alkynyl group is bound to the rest of the molecule by a single bond. The term "alkynyl" includes, for example and not restricted to, ethynyl, 1-propynyl, 2-propynyl, 1-butynyl, 2-butynyl, 3-butynyl, pentynyl, such as 1-pentynyl, and similar. The alkynyl group can also contain one or more double carbon-carbon bonds, and alkynyl groups include, for example and not restricted to, but-1-en-3-ynyl and pent-4-en-1-ynyl groups, and similar.

The term "alicyclyl" is used herein to cover, for example and not restricted to, aliphatic cyclic (alicyclic) groups such as cycloalkyl or cycloalkenyl or cycloalkynyl groups. The term "alicyclyl" refers to a monoradical that contains one or more rings of carbon atoms, the rings may be saturated (e.g., cyclohexyl) or unsaturated (e.g., cyclohexenyl) provided that they are not aromatic. More specifically alicylic groups contain three or more, from 3 to 24, from 3 to 12, or from 6 to 12, ring carbon atoms. The alicyclic group may be a monocyclic, bicyclic, or tricyclic ring system and the rings may be, for example, fused or linked by a single bond or a linking group such as a methylene or other alkylene group. The alicyclic group may be substituted (mono- or poly-) or unsubstituted. In one embodiment, the alicyclyl group is a 6 to 12 membered ring system which consists of carbon atoms and optionally contains one or two double bonds.

The term "cycloalkyl" refers to a saturated mono- or polycyclic alkyl group which may be substituted (mono- or poly-) or unsubstituted. The cycloalkyl group has from 3 to 24, preferably from 3 to 16, more preferably from 3 to 14, even more preferably from 3 to 12, yet even more preferably 3, 4, 5 or 6 carbon atoms. The cycloalkyl group is bound to the rest of the molecule by a single bond. Cycloalkyl groups include, for example and not restricted to, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and similar.

The term "cycloalkenyl" refers to a non-aromatic mono- or polycyclic alkenyl group which may be substituted (mono- or poly-) or unsubstituted. The cycloalkenyl group has from 5 to 24, preferably from 5 to 16, more preferably from 5 to 14, even more preferably from 5 to 12, yet more preferably 5 or 6 carbon atoms. The cycloalkenyl group is bound to the rest of the molecule by a single bond. Preferably the cycloalkenyl group contains 1, 2 or 3 double carbon-carbon bonds. If more than one double carbon-carbon bond is present, the double bonds may be conjugated or not conjugated. Cycloalkenyl groups include, for example and not restricted to, the cyclopent-1-en-1-yl group and similar.

The term "cycloalkynyl" refers to a non-aromatic mono- or polycyclic alkynyl group which may be substituted (mono- or poly-) or unsubstituted. The cycloalkynyl group has from 8 to 24, preferably from 8 to 16, more preferably from 8 to 14, even more preferably from 8 to 12, yet even more preferably 8 or 9 carbon atoms and is bound to the rest of the molecule by a single bond. Preferably the cycloalkynyl group contains 1, 2 or 3 triple carbon-carbon bonds, conjugated or not conjugated. Cycloalkynyl groups include, for example and not restricted to, the cyclooct-2-yn-1-yl group and similar. Cycloalkynyl groups can also contain one or more double carbon-carbon bonds, including, for example and not restricted to, the cyclooct-4-en-2-ynyl group and similar.

As used herein, the term "heterocyclyl" or "heterocyclic" refers to a hydrocarbon ring system of 3 to 10 members, wherein one or more of the atoms in the ring or rings is a heteroatom (i.e. not a carbon atom). Thus "heterocyclyl" or "heterocyclic" refers a cyclic group in which the ring atoms consist of carbon and one or more heteroatoms. To satisfy valence, the heteroatom may be bonded to H or substituent groups. Preferably from 1, 2 or 3 of the ring carbon atoms are heteroatoms. Each heteroatom can be independently selected from the group consisting of O, N, S, P and B, or the group consisting of O, N, and S. The heterocyclyl group may be substituted (mono- or poly-) or unsubstituted. The heterocyclyl group may be a monocyclic, bicyclic, or tricyclic ring system and the rings may be, for example, fused or linked by a single bond or a linking group such as a methylene or other alkylene group. Nitrogen, carbon or sulfur atoms present in the heterocyclyl radical may be optionally oxidized and the nitrogen atom may be optionally quaternized. The heterocyclyl radical may be unsaturated or partially or fully saturated. The heterocyclyl radical may be aliphatic or aromatic. In one embodiment, the heterocyclyl is aliphatic (also known as heteroalicyclyl) and is a 3 to 10 membered ring system where the atoms of the ring or rings consist of carbon atoms and from 1 to 4, or 1, 2 or 3 heteroatoms. In one embodiment, the heterocyclyl group is a 6 to 10 membered ring system where the atoms of the ring or rings consist of carbon atoms and from 1 to 4 heteroatoms and where the ring system optionally contains one or two double bonds. In one embodiment, the heterocyclyl is aromatic (also known as heteroaryl) and is a 6 to 10 membered ring system where the atoms of the ring or rings consist of carbon atoms and from 1 to 4, or 1, 2 or 3 heteroatoms. The greatest preference is for the term heterocyclyl to refer to a ring of 5 or 6 members. Examples of saturated heteroalicyclyl groups are dioxane, piperidine, piperazine, pyrrolidine, morpholine and thiomorpholine. Examples of aromatic heterocyclyl groups are pyridine, pyrrol, furan, thiophene, benzofuran, imidazoline, quinolein, quinoline, pyridazine and naphthyridine.

The term "aryl group" refers to an aromatic group which has from 6 to 30, preferably from 6 to 18, more preferably between 6 and 10, yet even more preferably 6 or 10 carbon atoms. The aryl group can comprise 1, 2, 3 or 4 aromatic rings, which may be linked by a carbon-carbon bond or fused together and includes, for example and not restricted to, phenyl, naphthyl, diphenyl, indenyl, phenanthryl or antranyl among others. The aryl group may be substituted (mono- or poly-) or unsubstituted.

The term "aralkyl group" refers to an alkyl group substituted by an aromatic group, with from 7 to 24 carbon atoms and including, for example and not restricted to, -(CH₂)₁₋₆-phenyl, -(CH₂)₁₋₆-(1-naphthyl), -(CH₂)₁₋₆-(2-naphthyl), -(CH₂)₁₋₆-CH(phenyl)₂ and similar.

The term "heteroarylalkyl" refers to an alkyl group substituted by a heteroaryl (also known as aromatic heterocyclic) group as defined above, the alkyl group having from 1 to 6 carbon atoms and the heteroaryl group having from 2 to 24 carbon atoms and from 1 to 3 heteroatoms. Heteroarylalkyl groups include, for example and not restricted to, -(CH₂)₁₋₆-imidazolyl, -(CH₂)₁₋₆-triazolyl, -(CH₂)₁₋₆-thienyl, -(CH₂)₁₋₆-furyl, -(CH₂)₁₋₆-pyrr olidinyl and similar.

As is understood in this technical field, there may be a certain degree of substitution of the aforementioned groups. In particular, there can be substitution in any of the groups identified above where it is explicitly stated. The substituted groups (radicals) referred to above are groups (or radicals) which are substituted in one or more positions available by one or more substituents. Preferably substitution is in the 1, 2 or 3 positions, more preferably in the 1 or 2 positions, yet even more preferably in the 1 position. Suitable substituents include, for example and not restricted to: C₁-C₄ alkyl; hydroxyl; C₁-C₄ alkoxyl; amino; amino-C₁-C₄alkyl; C₁-C₄ carbonyloxyl; C₁-C₄ oxycarbonyl; halogen such as fluoride, chlorine, bromine and iodine; cyano; nitro; azide; C₁-C₄ alkylsulfonyl; thiol; C₁-C₄ alkylthio; aryloxy such as phenoxyl; -NR_{b}(C=NR_{b})NR_{b}R_{c}; wherein R_{b} and R_{c} are independently selected from the group formed by H, C₁-C₄ alkyl, C₂-C₄ alkenyl, alkynyl, C₅-C₄, cycloalkyl, C₆-C₁₈ aryl, C₇-C₁₇ aralkyl, heterocyclyl of 3-10 members or protective group of the amino group.

As used herein, the term "comprising", which is inclusive or open-ended and does not exclude additional unrecited elements or method steps, is intended to encompass as alternative embodiments, the phrases "consisting essentially of" and "consisting of" where "consisting of" excludes any element or step not specified and "consisting essentially of" permits the inclusion of additional unrecited elements or steps that do not materially affect the essential or basic and novel characteristics of the composition or method under consideration.

### Compounds of the invention

A first aspect of the invention relates to a compound of formula (I)

R₁-Wₘ-Xₙ-AA₁-AA₂-AA₃-AA₄-AA₅-AA₆-Yₚ-Z_{q}-R₂ (I),

a stereoisomer and/or cosmetically acceptable salt thereof, wherein:
AA₁ is Arg, Lys or no amino acid;
AA₂ is Arg or Lys;
AA₃ is Gln or Asp;
AA₄ is Met or Leu;
AA₅ is Glu, Asp or Gln;
AA₆ is Glu, Asp, Gln or no amino acid;
AA₁ is different from AA₆;
W, X, Y and Z are each independently an amino acid selected from the group consisting of Ala, Gly, Val and Ile;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl;
R₁ and R₂ are not amino acids; and
either (i) each of AA₁, AA₂, AA₃, AA₄, AA₅ and AA₆ is an L-amino acid, or (ii) 1, 2 or 3 of AA₃, AA₄ and AA₅ is a D-amino acid and the remaining of AA₁ to AA₆ are L-amino acids.

In particular, compounds of formula (I) have been found to have a greater capacity to inhibit noradrenaline release, and similar or greater capacity to stimulate collagen synthesis in the skin, than Agireline^{®}.

The compound of formula (I) is a peptide which comprises 5, 6, 7 or 8 amino acids linked in a chain. R₁ in bound to the amino terminal end (N-terminal) of the peptide and R₂ is bound to the carboxy-terminal end (C-terminal) of the peptide.

R₁ can be selected from the group consisting of H, a polymer derived from polyethylene glycol with a molecular weight comprised between 200 and 35000 Daltons and R₅CO, wherein R₅ is selected from the group consisting of C₁C₂₄ alkyl, C₂C₂₄ alkenyl, C₂C₂₄ alkynyl, C₃C₂₄ cycloalkyl, C₅C₂₄ cycloalkenyl, C₈C₂₄ cycloalkynyl, C₆-C₃₀ aryl, C₇-C₂₄ aralkyl, 3-10 membered heterocyclyl ring, and a heteroarylalkyl containing from 2 to 24 carbon atoms and from 1 to 3 heteroatoms, wherein the alkyl group has 1 to 6 carbon atoms.

R₁ can be selected from the group consisting of H and R₅-CO-, wherein R₅ is selected from the group consisting of C₁-C₁₈ alkyl, C₂-C₂₄ alkenyl, C₃-C₂₄ cycloalkyl or the group consisting of C₁-C₁₆ alkyl, C₂-C₁₈ alkenyl, C₃-C₇ cycloalkyl. The R₅-CO- group includes alkanoyl groups such as acetyl (CH₃-CO-, which is abbreviated herein as "Ac-"), myristoyl (CH₃-(CH₂)₁₂-CO-, which is abbreviated herein as "Myr-") and palmitoyl (CH₃-(CH₂)₁₄-CO-, which is abbreviated herein as "Palm-").

R₁ can be selected from the group consisting of H and acetyl, tert-butanoyl, prenyl, hexanoyl, 2-methylhexanoyl, cyclohexanecarboxyl, octanoyl, decanoyl, lauroyl, myristoyl, palmitoyl, stearoyl, oleoyl and linoleoyl.

R₁ can be selected from the group consisting of H and R₅-CO-, wherein R₅ is selected from the group consisting of C₁-C₁₆ alkyl or C₂-C₁₈ alkenyl.

R₁ can be selected from the group consisting of H and R₅-CO-, wherein R₅ is C₁-C₁₅ alkyl.

R₁ can be selected from the group consisting of H, acetyl and palmitoyl.

R₂ can be selected from the group consisting of -NR₃R₄, -OR₃, -SR₃, wherein R₃ and R₄ are independently selected from the group formed by H, a polymer derived from polyethylene glycol, C₁-C₂₄ alkyl, C₂-C₂₄ alkenyl, C₂-C₂₄ alkynyl, C₃-C₂₄ cycloalkyl, C₃-C₂₄ cycloalkenyl, C₈-C₂₄ cycloalkynyl, C₆-C₃₀ aryl, C₇-C₂₄ aralkyl, 3-10 membered heterocyclyl ring, and heteroarylalkyl containing from 2 to 24 carbon atoms and from 1 to 3 heteroatoms, wherein the alkyl group has 1 to 6 carbon atoms. Optionally, R₃ and R₄ can be joined by a saturated or unsaturated carbon-carbon bond, forming a ring with the nitrogen atom.

R₂ can be -NR₃R₄ or -OR₃. R₃ and R₄ can be independently selected from the group consisting of H, a polymer derived from polyethylene glycol with a molecular weight comprised between 200 and 35000 Daltons, methyl, ethyl, hexyl, dodecyl and hexadecyl. Alternatively, R₃ and R₄ can be independently selected from the group consisting of H and C₁-C₁₆ alkyl. In one embodiment, R₂ is not OR₃ where R₃ is a methyl group, i.e. R₂ is not OCH₃. In one embodiment R₃ is H and R₄ is selected from the group formed by H and C₁-C₁₆ alkyl, including methyl, ethyl, hexyl, dodecyl and hexadecyl.

R₂ can be selected from the group consisting of -OH, -NH₂ and -NHR₄ where R₄ is C₁-C₁₆ alkyl or C₁-C₃ alkyl or C₁-C₂ alkyl.

R₂ can be -OH or -NH₂.

R₁ can be selected from the group consisting of H and R₅-CO-, wherein R₅ is selected from the group consisting of C₁-C₁₈ alkyl, C₂-C₂₄ alkenyl, C₃-C₂₄ cycloalkyl; and R₂ is -NR₃R₄ or -OR₃ wherein R₃ and R₄ are independently selected from the group consisting of H and C₁-C₁₆ alkyl. In this embodiment, R₃ can be H and R₄ can be selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of H and acetyl, tert-butanoyl, prenyl, hexanoyl, 2-methylhexanoyl, cyclohexanecarboxyl, octanoyl, decanoyl, lauroyl, myristoyl, palmitoyl, stearoyl, oleoyl and linoleoyl; and R₂ is -NR₃R₄ or -OR₃ wherein R₃ and R₄ are independently selected from the group consisting of H and C₁-C₁₆ alkyl. In this embodiment, R₃ can be H and R₄ can selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of H and R₅-CO-, wherein R₅ is selected from the group consisting of C₁-C₁₆ alkyl or C₂-C₁₈ alkenyl; and R₂ is -NR₃R₄ or -OR₃ wherein R₃ and R₄ are independently selected from the group consisting of H and C₁-C₁₆ alkyl. In this embodiment R₃ can be H and R₄ can be selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of H, acetyl, myristoyl or palmitoyl; and R₂ is -NR₃R₄ or -OR₃ wherein R₃ and R₄ are independently selected from the group consisting of H and C₁-C₁₆ alkyl. In this embodiment R₃ can be H and R₄ can be selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of H and R₅-CO-, wherein R₅ is C₁-C₁₅ alkyl and R₂ is -NR₃R₄ or -OR₃ wherein R₃ and R₄ are independently selected from the group consisting of H and C₁-C₁₆ alkyl. In this embodiment R₃ can be H and R₄ can be selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of H, acetyl and palmitoyl and R₂ is -NR₃R₄ or -OR₃ wherein R₃ and R₄ are independently selected from the group consisting of H and C₁-C₁₆ alkyl. In this embodiment R₃ can be H and R₄ can be selected from the group formed by H, C₁-C₁₆ alkyl, C₁-C₃ alkyl and C₁-C₂ alkyl; for example, R₂ can be selected from the group consisting of -OH and -NH₂.

R₁ can be selected from the group consisting of a substituted non-cyclic aliphatic group, substituted alicyclyl, substituted heterocyclyl, substituted heteroarylalkyl, substituted aryl, substituted aralkyl and R₅-CO-, wherein R₅ is selected from the group consisting of a substituted non-cyclic aliphatic group, substituted alicyclyl, substituted aryl, substituted aralkyl, substituted heterocyclyl and substituted heteroarylalkyl; and/or R₂ is -NR₃R₄, wherein at least one of R₃ and R₄ is selected from the group consisting of a substituted non-cyclic aliphatic group, substituted alicyclyl, substituted heterocyclyl, substituted heteroarylalkyl, substituted aryl and substituted aralkyl, or R₂ is -OR₃, or - SR₃, wherein R₃ is selected from the group consisting of a substituted non-cyclic aliphatic group, substituted alicyclyl, substituted heterocyclyl, substituted heteroarylalkyl, substituted aryl and substituted aralkyl.

In accordance with another particular embodiment the most preferred structures of the polymer derived from polyethylene glycol are the group (-CH₂-CH₂-O)ᵣ-H in which r is a number comprised between 4 and 795 and the group 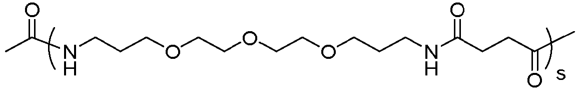 where s is a number comprised between 1 and 125.

The invention provides for a compound of formula (I), wherein at least one of: R₁ is not H; and R₂ is not OH. That is, the inventon provides for a compound of formula (I) where R₁ is not H and/or R₂ is not OH.

In the compound of formula (I): AA₁ is selected from the group consisting of Arg, Lys and no amino acid; AA₂ is selected from the group consisting of Arg and Lys; AA₃ is selected from the group consisting of Gln and Asp; AA₄ is selected from the group consisting of Met and Leu; AA₅ is selected from the group consisting of Glu, Asp and Gln; and AA₆ is selected from the group consisting of Glu, Gln and Asp and no amino acid. It is a requirement that AA₁ is different from AA₆. Thus, when AA₁ is no amino acid, i.e. when an amino acid AA₁ is not present, amino acid AA₆ must be present, i.e. AA₆ is selected from the group consisting of Glu, Asp and Gln. Further, when AA₆ is no amino acid, i.e. when an amino acid AA₆ is not present, amino acid AA₁ must be present, i.e. AA₁ is selected from the group consisting of Arg and Lys. In one embodiment of the compound of formula (I): AA₁ is Arg and/or AA₂ is Arg.

The invention provides for a compound of formula (I), wherein: AA₁ is selected from the group consisting of Arg, Lys and no amino acid; AA₂ is selected from the group consisting of Arg and Lys; AA₃ is selected from the group consisting of Gln and Asp; AA₄ is selected from the group consisting of Met and Leu; AA₅ is selected from the group consisting of Glu and Asp; and AA₆ is selected from the group consisting of Glu, Gln and no amino acid. In one embodiment of the compound of formula (I): AA₁ is Arg and/or AA₂ is Arg.

The invention provides for a compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₅ is selected from the group consisting of Glu and Asp; and AA₆ is selected from the group consisting of Glu and Gln. In one embodiment of the compound of formula (I): AA₁ is Arg and/or AA₂ is Arg.

The invention provides for a compound of formula (I), wherein: AA₁ is selected from the group consisting of Arg, Lys and no amino acid; AA₂ is selected from the group consisting of Arg and Lys; AA₃ is Gln; AA₄ is Met; AA₅ is Glu and Asp; and AA₆ is selected from the group consisting of Glu, Gln and no amino acid. In one embodiment of the compound of formula (I): AA₁ is Arg and/or AA₂ is Arg.

The invention provides for a compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₅ is selected from the group consisting of Glu, Asp and Gln; and AA₆ is selected from the group consisting of Glu, Gln and Asp. In this embodiment, preferably AA₁ is Arg and/or AA₂ is Arg.

The invention provides for a the compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₅ is selected from the group consisting of Glu, Asp and Gln; and AA₆ is selected from the group consisting of Glu, Gln and Asp. In this embodiment, preferably AA₁ is Arg and/or AA₂ is Arg.

The invention provides for a compound of formula (I), wherein: AA₁ is selected from the group consisting of Arg and Lys; AA₂ is selected from the group consisting of Arg and Lys; AA₃ is selected from the group consisting of Gln and Glu; AA₄ is selected from the group consisting of Met and Leu; AA₅ is selected from the group consisting of Glu, Asp and Gln; and AA₆ is selected from the group consisting of Glu, Gln and Asp. In this embodiment, preferably AA₁ is Arg and/or AA₂ is Arg.

The invention provides for a compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₅ is selected from the group consisting of Glu and Gln; and AA₆ is is selected from the group consisting of Glu and Gln.

The invention provides for a compound of formula (I), wherein: AA₁ is Arg; AA₂ is selected from the group consisting of Arg and Lys; AA₃ is selected from the group consisting of Gln and Asp; AA₄ is selected from the group consisting of Met and Leu; AA₅ is Glu; and AA₆ is Glu.

The invention provides for a compound of formula (I), wherein: AA₁ is selected from the group consisting of Arg, Lys and no amino acid; AA₂ is Arg; AA₃ is Gln; AA₄ is Met; AA₅ is selected from the group consisting of Glu and Asp; and AA₆ is selected from the group consisting of Glu, Gln and no amino acid.

The invention provides for a compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₅ is selected from the group consisting of Glu, Asp and Gln; and AA₆ is no amino acid. In this embodiment, preferably AA₁ is Arg and/or AA₂ is Arg.

The invention provides for a compound of formula (I), wherein: AA₁ is no amino acid; AA₂ is selected from the group consisting of Arg and Lys; AA₃ is selected from the group consisting of Gln, Glu, Asn and Asp or the group consisting of Gln and Asp; AA₄ is selected from the group consisting of Met and Leu; AA₅ is selected from the group consisting of Glu, Asp and Gln; and AA₆ is selected from the group consisting of Glu, Gln and Asp.

The invention provides for a compound of formula (I), wherein: AA₁ is Arg; AA₂ is Arg; AA₃ is Gln; AA₄ is Met; AA₅ is Glu; and AA₆ is Glu; and 0, 1, 2, 3 or 4 of AA₁, AA₂, AA₃, AA₄, AA₅ and AA₆ are replaced providing that: when AA₁ is replaced, it is replaced by Lys or no amino acid; when AA₂ is replaced, it is replaced by Lys; when AA₃ is replaced, it is replaced by Glu, Asn or Asp; when AA₄ is replaced, it is replaced by Leu; when AA₃ is replaced, it is replaced by Asp or Gln; and when AA₆ is replaced, it is replaced by Asp, Gln or no amino acid. In one embodiment, 0, 1, 2 or 3 of AA₁, AA₂, AA₃, AA₄, AA₅ and AA₆ are replaced. In one embodiment, 0 (none) of AA₁, AA₂, AA₃, AA₄, AA₅ and AA₆ are replaced. In one embodiment, one of AA₁, AA₂, AA₃, AA₄, AA₅ and AA₆ are replaced. In one embodiment, two of AA₁, AA₂, AA₃, AA₄, AA₅ and AA₆ are replaced. In one embodiment, three of AA₁, AA₂, AA₃, AA₄, AA₅ and AA₆ are replaced.

The invention provides for a compound of formula (I) in each of the embodiments described herein, wherein when AA₁ is no amino acid, W, X, Y and Z are each independently selected from the group consisting of Ala, Gly, Val and Ile.

The invention provides for a compound of formula (I) in each of the embodiments described herein, wherein when AA₁ is no amino acid, m, n, p and q are each 0.

The compounds of the invention can exclude Arg-Arg-Glu-Leu-Glu-Glu-Leu, i.e. the invention provides a compound of formula (I) as described above, wherein the compound is not Arg-Arg-Glu-Leu-Glu-Glu-Leu. The compounds of the invention can exclude Lys-Lys-Glu-Leu-Glu-Glu-Leu, i.e. the invention provides a compound of formula (I) as described above, wherein the compound is not Lys-Lys-Glu-Leu-Glu-Glu-Leu.

The invention provides for a compound of formula (I) as described above, wherein each of AA₁, AA₂, AA₃, AA₄, AA₅ and AA₆ in formula (I) is an L-amino acid. Compounds of this embodiment have been found to be particularly effective at inhibiting noradrenaline release and stimulating collagen synthesis in the skin. This embodiment includes a compound of formula (I) wherein: AA₁ is L-Arg; AA₂ is L-Arg; AA₃ is L-Gln; AA₄ is L-Met; AA₅ is L-Glu; and AA₆ is L-Glu.

The invention provides for a compound of formula (I), wherein 1, 2 or 3 of AA₁, AA₂, AA₃, AA₄, AA₅ and AA₆ in formula (I) is a D-amino acid, and the remaining of AA₁ to AA₆ are L-amino acids. It has been found that the compounds of formula (I) are particularly effective at upregulating the expression of muscleblind-like1 (MBNL-1), and are thus useful for preventing or alleviating cosmetic properties of the skin associated with loss of muscle tone due to muscle aging and, indeed, alleviating cosmetically undesirable side effects of anti-aging treatments involving Botulinum toxin injections.

This embodiment includes where 1 or 2 of AA₃, AA₄ and AA₃ are D-amino acids and the other amino acids, i.e the remaining of AA₁ to AA₆, are L-amino acids.

Thus the invention provides for a compound of formula (I), wherein: AA₁ is selected from the group consisting of Arg, Lys and no amino acid; AA₂ is selected from the group consisting of Arg and Lys; AA₃ is selected from the group consisting of Gln and Asp; AA₄ is selected from the group consisting of Met and Leu; AA₅ is selected from the group consisting of Glu and Asp; and AA₆ is selected from the group consisting of Glu, Gln and no amino acid, and wherein 1, 2, or 3 of AA₃, AA₄ and AA₅ are D-amino acids and the other amino acids, i.e. the remaining of AA₁ to AA₆, are L-amino acids. This embodiment includes where 1 or 2 of AA₃, AA₄ and AA₅ are D-amino acids and the other amino acids, i.e. the remaining of AA₁ to AA₆, are L-amino acids.

Thus the invention provides for a compound of formula (I), wherein: AA₁ is selected from the group consisting of Arg and Lys; AA₂ is selected from the group consisting of Arg and Lys; AA₃ is selected from the group consisting of Gln and Asp; AA₄ is selected from the group consisting of Met and Leu; AA₃ is selected from the group consisting of Glu and Asp; and AA₆ is selected from the group consisting of Glu and Gln, and wherein 1, 2, or 3 of AA₃, AA₄ and AA₅ are D-amino acids and the other amino acids, i.e. the remaining of AA₁ to AA₆, are L-amino acids. This embodiment includes where 1 or 2 of AA₃, AA₄ and AA₅ are D-amino acids and the other amino acids, i.e. the remaining of AA₁ to AA₆, are L-amino acids.

Thus the invention provides for a compound of formula (I), wherein: AA₁ is Arg; AA₂ is Arg; AA₃ is Gln; AA₄ is Met; AA₅ is Glu; and AA₆ is Glu; and 0, 1, 2, 3 or 4 of AA₁, AA₂, AA₃, AA₄, AA₅ and AA₆ are replaced providing that: when AA₁ is replaced, it is replaced by Lys or no amino acid; when AA₂ is replaced, it is replaced by Lys; when AA₃ is replaced, it is replaced by Asp; when AA₄ is replaced, it is replaced by Leu; when AA₃ is replaced, it is replaced by Asp or Gln; and when AA₆ is replaced, it is replaced by Asp, Gln or no amino acid, and wherein 1, 2, or 3 of AA₃, AA₄ and AA₅ are D-amino acids and the other amino acids, i.e. the remaining of AA₁ to AA₆, are L-amino acids. This embodiment includes where 1 or 2 of AA₃, AA₄ and AA₅ are D-amino acids and the other amino acids, i.e. the remaining of AA₁ to AA₆, are L-amino acids. 0, 1, 2 or 3 of AA₁, AA₂, AA₃, AA₄, AA₅ and AA₆ can be replaced. 0 (none) of AA₁, AA₂, AA₃, AA₄, AA₅ and AA₆ can be replaced. One of AA₁, AA₂, AA₃, AA₄, AA₅ and AA₆ can be replaced. Two of AA₁, AA₂, AA₃, AA₄, AA₅ and AA₆ can be replaced. Three of AA₁, AA₂, AA₃, AA₄, AA₅ and AA₆ can be replaced.

The invention provides for a compound of formula (I), wherein: AA₁ is selected from the group consisting of L-Arg, L-Lys and no amino acid; AA₂ is selected from the group consisting of L-Arg and L-Lys; AA₃ is selected from the group consisting of D-Gln and D-Asp; AA₄ is selected from the group consisting of L-Met and L-Leu; AA₅ is selected from the group consisting of L-Glu, L-Asp and L-Gln; and AA₆ is selected from the group consisting of L-Glu, L-Gln and L-Asp and no amino acid. In this embodiment, preferably AA₁ is L-Arg and AA₂ is L-Arg. This embodiment includes a compound of formula (I) wherein: AA₁ is L-Arg; AA₂ is L-Arg; AA₃ is D-Gln; AA₄ is L-Met; AA₅ is L-Glu; and AA₆ is L-Glu. The invention provides for a compound of formula (I), wherein: AA₁ is selected from the group consisting of L-Arg, L-Lys and no amino acid; AA₂ is selected from the group consisting of L-Arg and L-Lys; AA₃ is selected from the group consisting of L-Gln and L-Asp; AA₄ is selected from the group consisting of D-Met and D-Leu; AA₅ is selected from the group consisting of L-Glu, L-Asp and L-Gln; and AA₆ is selected from the group consisting of L-Glu, L-Gln and L-Asp and no amino acid. Compounds of this embodiment have been found to be particularly effective at inhibiting noradrenaline release and stimulating collagen synthesis in the skin. In this embodiment, preferably is AA₁ is L-Arg and AA₂ is L-Arg. This embodiment includes a compound of formula (I) wherein: AA₁ is L-Arg; AA₂ is L-Arg; AA₃ is L-Gln; AA₄ is D-Met; AA₅ is L-Glu; and AA₆ is L-Glu.

The invention provides for a compound of formula (I), wherein: AA₁ is selected from the group consisting of L-Arg, L-Lys and no amino acid; AA₂ is selected from the group consisting of L-Arg and L-Lys; AA₃ is selected from the group consisting of L-Gln and L-Asp; AA₄ is selected from the group consisting of L-Met and L-Leu; AA₅ is selected from the group consisting of D-Glu, D-Asp and D-Gln; and AA₆ is selected from the group consisting of L-Glu, L-Gln and L-Asp and no amino acid. In this embodiment, preferably is AA₁ is L-Arg and AA₂ is L-Arg. This embodiment includes a compound of formula (I) wherein: AA₁ is L-Arg; AA₂ is L-Arg; AA₃ is L-Gln; AA₄ is L-Met; AA₅ is D-Glu; and AA₆ is L-Glu.

The invention provides for a compound of formula (I), wherein: AA₁ is selected from the group consisting of L-Arg, L-Lys and no amino acid; AA₂ is selected from the group consisting of L-Arg and L-Lys; AA₃ is selected from the group consisting of L-Gln and L-Asp; AA₄ is selected from the group consisting of D-Met and D-Leu; AA₅ is selected from the group consisting of D-Glu, D-Asp and D-Gln; and AA₆ is selected from the group consisting of L-Glu, L-Gln and L-Asp and no amino acid. In this embodiment, preferably is AA₁ is L-Arg and AA₂ is L-Arg. This embodiment includes a compound of formula (I) wherein: AA₁ is L-Arg; AA₂ is L-Arg; AA₃ is L-Gln; AA₄ is D-Met; AA₅ is D-Glu; and AA₆ is L-Glu.

The invention provides for a compound of formula (I), wherein: AA₁ is selected from the group consisting of L-Arg, L-Lys and no amino acid; AA₂ is selected from the group consisting of L-Arg and L-Lys; AA₃ is selected from the group consisting of D-Gln and D-Asp; AA₄ is selected from the group consisting of D-Met and D-Leu; AA₅ is selected from the group consisting of L-Glu, L-Asp and L-Gln; and AA₆ is selected from the group consisting of L-Glu, L-Gln and L-Asp and no amino acid. In this embodiment, preferably is AA₁ is L-Arg and AA₂ is L-Arg. This embodiment includes a compound of formula (I) wherein: AA₁ is L-Arg; AA₂ is L-Arg; AA₃ is D-Gln; AA₄ is D-Met; AA₅ is L-Glu; and AA₆ is L-Glu.

Compounds of the invention include those selected from the group of amino acid sequences listed in Table 2a, in which their sequence identifier is detailed, and their cosmetically or pharmaceutically acceptable salts.

**Table 2a**

| Sequence | **Identifier** |
|---|---|
| L-Arg-L-Arg-L-Gln-L-Met-L-Glu-L-Glu | SEQ ID NO. 21 |
| L-Arg-L-Arg-L-Gln-D-Met-L-Glu-L-Glu | SEQ ID NO. 22 |
| L-Arg-L-Arg-L-Asp-D-Met-L-Glu-L-Glu | SEQ ID NO. 23 |
| L-Arg-L-Arg-L-Gln-D-Met-L-Asp-L-Glu | SEQ ID NO. 24 |
| L-Arg- L-Arg-L-Gln-D-Met-L-Glu-L-Gln | SEQ ID NO. 25 |
| L-Arg-L-Arg-L-Gln- L-Leu-L-Glu-L-Glu | SEQ ID NO. 26 |
| L-Arg-L-Arg- L-Gln-D-Met-L-Gln-L-Glu | SEQ ID NO. 27 |
| L-Arg-L-Arg- L-Gln-D-Met-L-Glu- L-Asp | SEQ ID NO. 28 |
| L-Arg-L-Lys-L-Gln-D-Met-L-Glu-L-Glu | SEQ ID NO. 29 |
| L-Lys-L-Arg-L-Gln-D-Met-L-Glu-L-Glu | SEQ ID NO. 30 |
| L-Arg-L-Arg-L-Gln-D-Met-L-Glu L-Glu-L-Ala | SEQ ID NO. 31 |
| L-Ala-L-Arg-L-Arg-L-Gln-D-Met-L-Glu-L-Glu-L-Ala | SEQ ID NO. 32 |
| L-Arg-L-Gln-D-Met-L-Glu-L-Glu | SEQ ID NO. 33 |
| L-Arg-L-Arg-L-Gln-D-Met-L-Glu | SEQ ID NO. 34 |
| L-Arg-L-Arg-D-Gln-L-Met-L-Glu-L-Glu | SEQ ID NO. 35 |
| L-Arg-L-Arg-L-Gln-D-Leu-L-Glu-L-Glu | SEQ ID NO. 36 |
| L-Arg-L-Arg-L-Gln-D-Met-D-Glu-L-Glu | SEQ ID NO. 37 |
| L-Arg-L-Arg-D-Gln-D-Met-L-Glu-L-Glu | SEQ ID NO. 38 |
| L-Arg-L-Arg-L-Asp-D-Met-L-Asp-L-Gln | SEQ ID NO. 39 |
| L-Arg-L-Arg-L-Asp-D-Leu-L-Asp-L-Gln | SEQ ID NO. 40 |

Compounds of the invention include each of the sequences of Table 2a in which one of amino acids AA₁ to AA₆ is replaced by a replacement amino acid, wherein the replacement amino acid is selected from the alternative amino acids listed for the amino acid being replaced in formula (I) above. The replacement amino acid is different from the amino acid that is being replaced. Thus the invention provides for each of the sequences of Table 2a in which one of amino acids AA₁ to AA₆ is replaced by an amino acid, wherein: when an AA₁ amino acid in one of the sequences in Table 2a is replaced it is replaced by Arg or Lys, with the proviso that if the AA₁ amino acid is Arg it is replaced by Lys and if the AA₁ amino acid is Lys it is replaced by Arg; when an AA₂ amino acid in one of the sequences in Table 2a is replaced it is replaced by Arg or Lys, with the proviso that if the AA₂ amino acid is Arg it is replaced by Lys and if the AA₂ amino acid is Lys it is replaced by Arg; when an AA₃ amino acid in one of the sequences in Table 2a is replaced it is replaced by Gln or Asp, with the proviso that if the AA₃ amino acid is Gln it is replaced by Asp, and if the AA₃ amino acid is Asp it is replaced by Gln; when an AA₄ amino acid in one of the sequences in Table 2a is replaced it is replaced by Met or Leu, with the proviso that if the AA₄ amino acid is Met it is replaced by Leu and if the AA₄ amino acid is Leu it is replaced by Met; when an AA₅ amino acid in one of the sequences in Table 2a is replaced it is replaced by Glu, Asp or Gln, with the proviso that if the AA₅ amino acid is Gln it is replaced by Glu or Asp, if the AA₅ amino acid is Glu it is replaced by Gln or Asp, and if the AA₅ amino acid is Asp it is replaced by Glu or Gln; and when an AA₆ amino acid in one of the sequences in Table 2a is replaced it is replaced by Glu, Asp or Gln, with the proviso that if the AA₆ amino acid is Gln it is replaced by Glu or Asp, if the AA₆ amino acid is Glu it is replaced by Gln or Asp, and if the AA₆ amino acid is Asp it is replaced by Glu or Gln.

In the amino acid sequences of Table 2a according to formula (I), R₁ and R₂ are H and OH, respectively. Compounds of the invention include each of the sequences of Table 2a with their N- and C- terminals modified by the other R₁ and R₂ groups, respectively, as defined herein for formula (I).

Thus, in particular, the invention provides for a compound according to formula (I), whrein the compound is an amino acid sequence selected from SEQ ID NO.s 21, 22, 23, 24, 25, 29, 30, 32, 33, 34, 35, 36 and 38, or from SEQ ID NO.s 21, 22, 35 and 38, and its cosmetically or pharmaceutically acceptable salts, wherein optionally, said sequence has its N-terminal amino acid modified by R₁ as defined above for formula (I), where R₁ is not H, and, alternatively or additionally, said sequence has its C-terminal amino acid modified by R₂ as defined above for formula (I) where R₂ is not OH. The amino acid sequence can be SEQ ID NO.s 21, 22, 23, 24, 25, 29, 30, 32, 33, 34, 35, 36,38. The amino acid sequence can be SEQ ID NO.s 21, 22, 35 or 38. The amino acid sequence can be SEQ ID NO. 21. The amino acid sequence can be SEQ ID NO. 22. The amino acid sequence can be SEQ ID NO. 35. The amino acid sequence can be SEQ ID NO. 38. The compounds of this invention can exist as stereoisomers or mixtures of stereoisomers; for example, the amino acids which comprise them can have the configuration L-, D-, or be racemic independently of each other. Therefore, it is possible to obtain isomeric mixtures as well as racemic mixtures or diastereomeric mixtures, or pure diastereomers or enantiomers, depending on the number of asymmetric carbons and on which isomers or isomeric mixtures are present. The preferred structures of the compounds of the invention are pure isomers, i.e., enantiomers or diastereomers. For example, when it is stated that AA₂ can be Arg, it is understood that, unless otherwise specified, AA₂ is selected from L-Arg, D-Arg or mixtures of both, racemic or nonracemic. The preparation procedures described in this document enable the person skilled in the art to obtain each of the stereoisomers of the compound of the invention by choosing the amino acid with the right configuration.

In the context of this invention, the term "amino acids" includes the amino acids encoded by the genetic code as well as non-encoded amino acids, whether they are natural or not. Examples of non-encoded amino acids are, without restriction, citrulline, ornithine, sarcosine, desmosine, norvaline, 4-aminobutyric acid, 2-aminobutyric acid, 2-aminoisobutyric acid, 6-aminohexanoyc acid, 1-naphthylalanine, 2-naphthylalanine, 2-aminobenzoic acid, 4-aminobenzoic acid, 4-chlorophenylalanine, 2,3-diaminopropionic acid, 2,4-diaminobutyric acid, cycloserine, carnitine, cystine, penicillamine, pyroglutamic acid, thienylalanine, hydroxyproline, allo-isoleucine, allothreonine, isonipecotic acid, isoserine, phenylglycine, statin, β-alanine, norleucine, N-methyl amino acids, α-amino acids and β-amino acids, among others, as well as their derivatives. A list of non-natural amino acids can be found in the article *"*Unusual amino acids in peptide synthesis" by D.C. Roberts and F. Vellaccio, in The Peptides, Vol. 5 (1983), Chapter VI, Gross E. and Meienhofer J., Eds., Academic Press, New York, USA or in the commercial catalogues of the companies specialized in the field.

In the context of this invention, when W, X, Y and/or Z are present, i.e. at least one of n, m, p or q is not 0, it is understood that the nature of W, X, Y and/or Z does not hinder the activity of the compound of the invention, and, instead, contributes to it or has no effect on it. W, X, Y and Z can each be independently selected from the group consisting of Ala, Gly, Val and Ile. W, X, Y and Z can each be independently selected from the group consisting of Ala, Gly and Val. W, X, Y and Z can each be Ala.

Each of m, n, p and q can be 0, i.e. the compound of formula (I) is a peptide which comprises 5 or 6 amino acids, AA₁ to AA₅, AA₂ to AA₆ or AA₁ to AA₆, linked in a chain. Alternatively, the sum of m, n, p and q can be 1, i.e. the compound of formula (I) is a peptide which comprises 6 or 7 amino acids linked in a chain. Alternatively, the sum of m, n, p and q can be 2, i.e. the compound of formula (I) is a peptide which comprises 7 or 8 amino acids linked in a chain.

In particular, the compound of the invention can be selected from the group of compounds listed in Table 3a, and their cosmetically acceptable salts.

**Table 3a**

| **Compound** | **Identifier** |
|---|---|
| Ac-L-Arg-L-Arg-L-Gln-L-Met-L-Glu-L-Glu-NH₂ | PEP21 |
| Ac-L-Arg-L-Arg-D-Gln-L-Met-L-Glu-L-Glu-NH₂ | PEP22 |
| Ac-L-Arg-L-Arg-L-Gln-D-Met-L-Glu-L-Glu-NH₂ | PEP23 |
| Ac-L-Arg-L-Arg-D-Gln-D-Met-L-Glu-L-Glu-NH₂ | PEP24 |
| Palm-L-Arg-L-Arg-L-Gln-D-Met-L-Glu-L-Glu-NH₂ | PEP25 |
| Ac-L-Arg-L-Arg-L-Gln-D-Met-L-Glu-L-Glu-OH | PEP26 |
| H-L-Arg-L-Arg-L-Gln-D-Met-L-Glu-L-Glu-NH₂ | PEP27 |
| Palm-L-Arg-L-Arg-L-Gln-D-Met-L-Glu-L-Glu-OH | PEP28 |
| Ac-L-Arg-L-Arg-L-Asp-D-Met-L-Glu-L-Glu-OH | PEP29 |
| Ac-L-Arg-L-Arg-L-Gln-D-Met L-Asp-L-Glu-NH₂ | PEP30 |
| Ac-L-Arg-L-Arg-L-Gln-D-Met-L-Glu-L-Gln-NH₂ | PEP31 |
| Ac-L-Arg-L-Arg-L-Gln-L-Leu-L-Glu L-Glu-NH₂ | PEP32 |
| Ac-L-Arg-L-Arg-L-Gln-D-Met-L-Gln-L-Glu-NH₂ | PEP33 |
| Ac-L-Arg-L-Arg-L-Gln-D-Met-L-Glu-L-Asp-NH₂ | PEP34 |
| Ac-L-Arg-L-Lys-L-Gln-D-Met-L-Glu-L-Glu-NH₂ | PEP35 |
| Ac-L-Lys-L-Arg-L-Gln-D-Met-L-Glu-L-Glu-NH₂ | PEP36 |
| Ac-L-Arg-L-Arg-L-Gln-D-Leu-L-Glu-L-Glu-NH₂ | PEP37 |
| Ac-L-Arg-L-Arg-L-Gln-D-Met-D-Glu-L-Glu-NH₂ | PEP38 |
| Ac-L-Arg-L-Arg-L-Asp-D-Met-L-Asp-L-Gln-NH₂ | PEP39 |
| Ac-L-Arg-L-Arg-L-Asp-D-Leu-L-Asp- L-Gln-NH₂ | PEP40 |

**Table 3b**

| **Compound** | **Identifier** |
|---|---|
| Ac-L-Arg-L-Arg-L-Asp-D-Met-L-Glu-L-Glu-NH₂ | PEP41 |
| Ac-L-Ala-L-Arg-L-Arg-L-Gln-D-Met-L-Glu-L-Glu-L-Ala-NH₂ | PEP42 |
| Ac-L-Arg-L-Gln-D-Met-L-Glu-L-Glu-NH₂ | PEP43 |
| Ac-L-Arg-L-Arg-L-Gln-D-Met-L-Glu- NH₂ | PEP44 |

Compounds of the invention include each of the compounds of Tables 3a and 3b in which one of amino acids AA₁ to AA₆ is replaced by a replacement amino acid, wherein the replacement amino acid is selected from the alternative amino acids listed for the amino acid being replaced in formula (I) above. The replacement amino acid is different from the amino acid that is being replaced. Thus the invention provides for each of the compounds of Tables 3a and 3b in which one of amino acids AA₁ to AA₆ is replaced by an amino acid, wherein: when an AA₁ amino acid in one of the sequences in Tables 3a and 3b is replaced it is replaced by Arg or Lys, with the proviso that if the AA₁ amino acid is Arg it is replaced by Lys and if the AA₁ amino acid is Lys it is replaced by Arg; when an AA₂ amino acid in one of the sequences in Tables 3a and 3b is replaced it is replaced by Arg or Lys, with the proviso that if the AA₂ amino acid is Arg it is replaced by Lys and if the AA₂ amino acid is Lys it is replaced by Arg; when an AA₃ amino acid in one of the sequences in Tables 3a and 3b is replaced it is replaced by Gln or Asp, with the proviso that if the AA₃ amino acid is Gln it is replaced by Asp, and if the AA₃ amino acid is Asp it is replaced by Gln; when an AA₄ amino acid in one of the sequences in Tables 3a and 3b is replaced it is replaced by Met or Leu, with the proviso that if the AA₄ amino acid is Met it is replaced by Leu and if the AA₄ amino acid is Leu it is replaced by Met; when an AA₅ amino acid in one of the sequences in Tables 3a and 3b is replaced it is replaced by Glu, Asp or Gln, with the proviso that if the AA₅ amino acid is Gln it is replaced by Glu or Asp, if the AA₅ amino acid is Glu it is replaced by Gln or Asp, and if the AA₅ amino acid is Asp it is replaced by Glu or Gln; and when an AA₆ amino acid in one of the sequences in Tables 3a and 3b is replaced it is replaced by Glu, Asp or Gln, with the proviso that if the AA₆ amino acid is Gln it is replaced by Glu or Asp, if the AA₆ amino acid is Glu it is replaced by Gln or Asp, and if the AA₆ amino acid is Asp it is replaced by Glu or Gln.

The invention provides for a compound according to formula (i), wherein the compound is selected those in Tables 3a and 3b and, in particular can be selected from PEP21, PEP22, PEP23, PEP24, PEP26, PEP27, PEP30, PEP31, PEP35, PEP36, PEP37, PEP41, PEP42, PEP43 and PEP44, or from PEP21, PEP22, PEP23, and PEP24, and its cosmetically or pharmaceutically acceptable salts. The compound can be PEP21, PEP22, PEP23, PEP24, PEP26, PEP27, PEP30, PEP31, PEP35, PEP36, PEP37, PEP41, PEP42, PEP43 or PEP44. The compound can be PEP21, PEP22, PEP23 or PEP24. The compound can be PEP21. The compound can be PEP22. The compound can be PEP23. The compound can be PEP24.

The cosmetically acceptable salts of the compounds provided by the present invention are also found within the field of this invention. The term "cosmetically acceptable salt" means a salt recognized for its use in animals, for example, in mammals, and more specifically in human beings, and includes salts used to form base addition salts, either they are inorganic, for example and not restricted to, lithium, sodium, potassium, calcium, magnesium, manganese, copper, zinc or aluminium among others, or they are organic, for example and not restricted to, ethylamine, diethylamine, ethylenediamine, ethanolamine, diethanolamine, arginine, lysine, histidine or piperazine among others, or acid addition salts, either they are organic, for example and not restricted to, acetate, citrate, lactate, malonate, maleate, tartrate, fumarate, benzoate, aspartate, glutamate, succinate, oleate, trifluoroacetate, oxalate, pamoate or gluconate among others, or inorganic, for example and not restricted to, chloride, sulfate, borate or carbonate, among others. The nature of the salt is not critical, provided that it is cosmetically or pharmaceutically acceptable. The cosmetically acceptable salts of the compounds of the invention can be obtained by the conventional methods, well known in the prior art *[*Berge S.M. et al., "Pharmaceutical Salts", (1977), J. Pharm. Sci., 66, 1-19*].*

The invention also provides for a combination of the compound of the invention, its stereoisomers, and/or its cosmetically acceptable salts, in any of the embodiments described above, with: a Botulinum toxin; Ac-Glu-Glu-Met-Gln-Arg-Arg-NH₂; or H-Tyr-D-Ala-Gly-Phe-Leu-OH; or combinations thereof.

### Preparation procedures of the compounds of the invention

Synthesis of the compounds of the invention, their stereoisomers, mixtures thereof and/or their cosmetically acceptable salts can be carried out according to conventional methods, known in the prior art, such as solid phase peptide synthesis methods *[*Stewart J.M. and Young J.D., "Solid Phase Peptide Synthesis, 2nd edition", (1984), Pierce Chemical Company, Rockford, Illino*is;* Bodanzsky M. and Bodanzsky A., "The practice of Peptide Synthesis", (1994), Springer Verlag, Berl*in;* Lloyd-Williams P. et al., "Chemical Approaches to the Synthesis of Peptides and Proteins", (1997), CRC, Boca Raton, FL, USA*],* synthesis in solution, enzymatic synthesis *[*Kullmann W. "Proteases as catalysts for enzymic syntheses of opioid peptides", (1980), J.Biol.Chem., 255(17), 8234-8238*]* or any combination thereof. The compounds can also be obtained by fermentation of a bacterial strain, modified or unmodified by genetic engineering with the objective of producing the desired sequences, or by controlled hydrolysis of proteins with animal or plant origins, preferably plant, which results in free peptide fragments that contain the desired sequence.

For example, a method of obtaining the compounds of formula (I), their stereoisomers and mixtures thereof comprises the stages of:
- coupling of an amino acid, with the *N*-terminal end protected and the *C*-terminal end free, with an amino acid with the *N*-terminal end free and the *C*-terminal end protected or bound to a solid support;
- elimination of the protective group of the *N*-terminal end;
- repetition of the coupling sequence and elimination of the protective group of the *N*-terminal end until the desired peptide sequence is obtained;
- elimination of the protective group of the *C*-terminal end or cleavage of the solid support.

Preferably, the *C*-terminal end is bound to a solid support and the process is carried out in solid phase and, therefore, comprises the coupling of an amino acid with the *N*-terminal end protected and the *C*-terminal end free, with an amino acid with the *N*-terminal end free and the *C*-terminal end bound to a polymeric support; elimination of the protective group of the *N*-terminal end; and repetition of this sequence as many times as is necessary to thus obtain the compound of desired length, finally followed by the cleavage of the synthesized compound from the original polymeric support.

The functional groups of the side chains of the amino acids are maintained conveniently protected with temporary or permanent protective groups throughout synthesis, and can be unprotected simultaneously or orthogonally to the process of cleavage of the peptide from the polymeric support.

Alternatively, solid phase synthesis can be carried out using a convergent strategy coupling a peptide with the polymeric support or with a peptide or an amino acid previously bound to the polymeric support. Convergent synthesis strategies are widely known by persons skilled in the art and are described in Lloyd-Williams P. et al., "Convergent Solid-Phase Peptide Synthesis", (1993), Tetrahedron, 49(48), 11065-11133*.*

The process can comprise the additional stages of deprotection of the *N*-terminal and *C*-terminal ends and/or cleavage of the peptide from the polymeric support in an indiscriminate order, using standard procedures and conditions known in the prior art, after which the functional groups of these ends can be modified. The optional modification of the *N*-terminal and *C*-terminal ends can be carried out with the peptide of formula (I) anchored to the polymeric support or once the peptide has been separated from the polymeric support.

Optionally, R₁ can be introduced by the reaction of the *N*-terminal end of the compound of the invention with a R₁-X compound through a nucleophilic substitution reaction, in the presence of an adequate base and solvent, wherein the fragments that have the functional groups not involved in the N-C bond formation are suitably protected with temporary or permanent protective groups. R₁ is as defined above and X is a leaving group, for example and not restricted to, the tosyl group, the mesyl group and halogen groups among others.

Optionally and/or additionally, the R₂ radicals can be introduced by the reaction of a compound HR₂ with a complementary fragment which corresponds to the peptide of formula (I) in which R₂ is -OH in the presence of an adequate solvent and a base such as *N,N*-diisopropylethylamine (DIEA) or trimethylamine, or an additive such as 1-hydroxybenzotriazole (HOBt) or 1-hydroxyazabenzotriazole (HOAt), and a dehydrating agent such as a carbodiimide, a uronium salt, a phosphonium salt or amidinium salt, among others, or by prior formation of an acyl halide with, for example, thionyl chloride, and thereby obtaining a peptide according to the invention of formula (I), wherein the fragments that have the functional groups not involved in the N-C bond formation are suitably protected with temporary or permanent protective groups. Alternatively other R₂ radicals may be introduced by simultaneous incorporation to the peptide cleavage process from the polymeric carrier. R₂ is -OR₃, -NR₃R₄ or -SR₃, where R₃ and R₄ are as defined above.

A person skilled in the art would easily understand that the deprotection/cleavage steps of the *C*-terminal and *N*-terminal ends and their subsequent derivatization can be performed in a different order, according to the processes known in the prior art.

The term "protective group" relates to a group which blocks an organic functional group and which can be removed in controlled conditions. The protective groups, their relative reactivities and the conditions in which they remain inert are known to the person skilled in the art.

Examples of representative protective groups for the amino group are amides, such as amide acetate, amide benzoate, amide pivalate; carbamates such as benzyloxycarbonyl (Cbz or Z), 2-chlorobenzyl (CIZ), *para*-nitrobenzyloxycarbonyl (pNZ), *tert*-butyloxycarbonyl (Boc), 2,2,2-trichloroethyloxycarbonyl (Troc), 2-(trimethylsilyl)ethyloxycarbonyl (Teoc), 9-fluorenylmethyloxycarbonyl (Fmoc) or allyloxycarbonyl (Alloc), trityl (Trt), methoxytrityl (Mtt), 2,4-dinitrophenyl (Dnp), *N*-[1-(4,4-dimethyl-2,6-dioxocyclohex-1-ylidene)ethyl (Dde), 1-(4,4-dimethyl-2,6-dioxo-cyclohexylidene)-3-methylbutyl (ivDde), 1-(1-adamantyl)-1-methylethoxycarbonyl (Adpoc), among others, preferably Boc or Fmoc.

Examples of representative protective groups for the carboxyl group are esters, such as the *tert*-butyl ester (tBu), allyl ester (All), triphenylmethyl ester (Trt tester), cyclohexyl ester (cHx), benzyl ester (Bzl), *ortho-*nitrobenzyl ester, *para*-nitrobenzyl ester, *para-*methoxybenzyl ester, trimethylsilylethyl ester, 2-phenylisopropyl ester, fluorenylmethyl ester (Fm), 4-(*N*-[1-(4,4-dimethyl-2,6-dioxocyclohexylidene)-3-methylbutyl]amino) benzyl ester (Dmab), among others; preferred protective groups of the invention are the All, tBu, cHex, Bzl and Trt esters.

The side chains of trifunctional amino acids can be protected during the synthetic process with temporary or permanent protective groups orthogonal to the protective groups of the *N*-terminal and *C*-terminal ends.

The hydroxyl group of the tyrosine side chain can be protected with the 2-bromobenzyloxycarbonyl group (2-BrZ), tBu, All, Bzl or 2,6-dichlorobenzyl (2,6-diCIZ) among others. In a preferred embodiment, the protective group strategy used is the strategy wherein the amino groups are protected by Boc, the carboxyl groups are protected by Bzl, cHx or All esters and the tyrosine side chain is protected with 2-BrZ or Bzl. In another preferred embodiment, the protective group strategy used is the strategy wherein the amino groups are protected by Fmoc, the carboxyl groups are protected by tBu, All or Trt esters, the tyrosine side chain is protected by tBu.

The amino group of the tryptophan side chain can be protected, for example, by the formyl group (For) or Boc. In one embodiment, when the amino group is protected by Fmoc, and the tryptophan side chain can be: unprotected, i.e. the amino acid is incorporated as Fmoc-Trp-OH; protected by Boc, i.e. the amino acid is incorporated as Fmoc-Trp(Boc)-OH; or protected by For, i.e. the amino acid is incorporated as Fmoc-Trp(For)-OH. In one embodiment, the amino group is protected by Boc, and the tryptophan side chain can be protected by For, i.e. the amino acid is incorporated as Boc-Trp(For)-OH.

Examples of these and other protective groups, their introduction and removal, can be found in the literature *[*Atherton B. and Sheppard R.C., "Solid Phase Peptide Synthesis: A practical approach", (1989), IRL Oxford University Press*].* The term "protective groups" also includes the polymeric supports used in solid phase synthesis.

When synthesis takes place totally or partially in solid phase, the possible solid supports used in the process of the invention involve polystyrene support, polyethylene glycol grafted to polystyrene and similar, for example and not restricted to, *p-*methylbenzhydrylamine resins (MBHA) *[*Matsueda G.R. et al., "A p-methylbenzhydrylamine resin for improved solid-phase synthesis of peptide amides", (1981), Peptides, 2, 45-50*],* 2-chlorotrityl resins *[*Barlos K. et al., "Darstellung geschützter Peptid-Fragmente unter Einsatz substituierter Triphenylmethyl-Harze", (1989), Tetrahedron Lett., 30, 3943-3946; Barlos K. et al., "Veresterung von partiell geschützten Peptid-Fragmenten mit Harzen. Einsatz von 2-Chlorotritylchlorid zur Synthese von Leu1-Gastrin I", (1989), Tetrahedron Lett., 30, 3947-3951*],* TentaGel˙ resins (Rapp Polymere GmbH), ChemMatrix˙ resins (Matrix Innovation, Inc) and similar, which may or may not include a labile linker, such as 5-(4-aminomethyl-3,5-dimethoxyphenoxy) valeric acid (PAL) *[*Albericio F. et al., "Preparation and application of the 5-(4-(9-fluorenylmethyloxycarbonyl) aminomethyl-3,5-dimethoxy-phenoxy)valeric acid (PAL) handle for the solid-phase synthesis of C-terminal peptide amides under mild conditions", (1990), J. Org. Chem., 55, 3730-3743*],* 2-[4-aminomethyl-(2,4-dimethoxyphenyl)] phenoxyacetic acid (AM) *[*Rink H., "Solid-phase synthesis of protected peptide fragments using a trialkoxy-diphenyl-methylester resin" , (1987), Tetrahedron Lett., 28, 3787-3790*], [*Wang S.S., "p-Alkoxybenzyl Alcohol Resin and p-Alkoxybenzyloxycarbonylhydrazide Resin for Solid Phase Synthesis of Protected Peptide Fragments", (1973), J.Am.Chem.Soc., 95, 1328-1333*]* and similar, which enable simultaneous deprotection and cleavage of the compound from the polymeric support.

### Applications

The present invention is based on the finding that compounds of formula (I) (the compounds of the invention) are useful in the treatment of the skin, hair, nails and/or mucous membranes. In particular, it has been found that the compounds of the invention can inhibit noradrenaline release and increase collagen synthesis in skin and are thus are useful in the prevention or treatment of the symptoms of skin aging including skin wrinkles, a sagging appearance of the skin and a loss of firmness and for treating or preventing facial asymetry. Further, it has been found that compounds of the invention can increase the lipid content of adipose cells and thus can cause an increase in the volume of adipose tissue. Thus the compounds of the invention can be used to increase facial volume, for example, in the cheek area. These effects further indicate the usefulness of the compounds in the prevention or treatment of the symptoms of skin aging. Thus the compounds of formula (I) are useful in the cosmetic, non-therapeutic treatment of the skin, hair, nails and/or mucous membranes.

The inhibition of noradrenaline release is an indication of inhibiton of neuronal exocytosis, similar to that of the Botulinum toxins. In the neuromuscular junctions, the release of neurotransmitters from peripheral neurons to skeletal muscle allows muscle contraction. Facial muscles are also subjected to these contractions. These contractions are more frequent around the eyes and mouth and in the forehead. With age, continuous release of neurotransmitters into neuromuscular junctions and decrease of elasticity, contributes to the increase of facial wrinkles and permanent expression lines. Therefore, inhibiting noradrenaline release is considered to be beneficial to the reduction these symptoms of aging. Increasing collagen synthesis helps counteract the effects of the reduction of collagen synthesis in the skin which accompanies the aging process.

Collagen is the most abundant protein in skin connective tissue; it forms a mesh-like structure that helps to support new cells as they grow while providing needed flexibility. Type I collagen (Collagen I) is the principal collagen of skin and is responsible for the strength and resiliency of this tissue. One of well-recognized characteristics of aging is sagging of the skin. This is due to a number of factors including loss of elasticity and firmness of the skin, the effect of gravity, the loss of skeletal support of the face, as well as loss of subcutaneous adipose tissue support in the face. An increase in collagen synthesis is considered to be beneficial to the reduction these symptoms of aging.

Adipose tissue or body fat is a connective tissue comprising cells called adipocytes which accumulate lipids. Advantageously, compounds of the invention have been found to be effective in increasing the lipid content of adipose cells and thus are useful in treatments to increase of the volume of adipose tissue, and to prevent and/or alleviate of effects of adipose tissue loss. The compounds of the invention can be used to increase facial volume, for example, in the cheek area.

Further, in some embodiments, compounds of the invention have been found to upregulate the expression of musclebind-like1 (MBNL-1). Without being bound into theory it is believed that an increase of MBNL1 protein contributes to the the slowing down of and/or avoidance of muscle mass loss due to the activation of atrophic processes, which can be due to muscle aging. Muscle aging can be also exacerbated by Botulinum toxin treatments. Thus, in these embodiments the compounds of the invention are especially useful in maintaining or improving skin firmness, preventing sagging appearance of the skin, and/or reducing facial asymmetry.

In one aspect, the invention provides the use of the compound of the invention, a stereoisomer and/or cosmetically acceptable salt thereof, or a cosmetic composition comprising the compound of the invention, a stereoisomer and/or a cosmetically acceptable salt thereof, in the cosmetic, non-therapeutic treatment and/or care of the skin, hair, nails and/or mucous membranes. In particular, the cosmetic, non-therapeutic treatment and/or care is that of the skin. In the context of this invention, skin includes the skin of the whole body including the skin of the face (including skin around the eyes), neckline, neck, décolletage, arms, hands, legs, feet, thighs, hips, buttocks, stomach and torso.

The compounds of the invention are useful in the cosmetic, non-therapeutic treatment and/or care of the skin, including: the treatment and/or prevention of the aging of the skin, the treatment and/or prevention of skin wrinkles; maintaining and improving skin firmness; the stimulation of collagen synthesis and/or prevention of collagen loss; the treatment and/or prevention of a sagging appearance of the skin; and/or the reduction and/or prevention of facial asymmetry; the increase of the volume of adipose tissue; and/or the prevention and/or alleviation of the effects of adipose tissue loss.

The compounds of the invention are useful in the cosmetic, non-therapeutic treatment and/or care of the skin, including: the treatment and/or prevention of the aging of the skin, the treatment and/or prevention of skin wrinkles; maintaining and improving skin firmness; the stimulation of collagen synthesis and/or prevention of collagen loss; the treatment and/or prevention of a sagging appearance of the skin; and/or the reduction and/or prevention of facial assymmetry.

The cosmetic, non-therapeutic treatment and/or care of the skin can be: the treatment and/or prevention of skin wrinkles; maintaining and improving skin firmness; the stimulation of collagen synthesis and/or prevention of collagen loss.

The cosmetic, non-therapeutic treatment and/or care can involve the inhibition of noradrenaline, the stimulation of the synthesis of collagen and/or the upregulation of MBNL-1. Thus, the cosmetic, non-therapeutic treatment and/or care of the skin, hair, nails and/or mucous membranes can be associated with inhibiting noradrenaline release, increasing collagen synthesis and/or upregulating expression of muscleblind-like1 (MBNL-1).

In one embodiment, there is provided the use of the compound of the invention, a stereoisomer and/or cosmetically acceptable salt thereof, or a cosmetic composition comprising the compound of the invention, a stereoisomer and/or cosmetically acceptable salt thereof, for the treatment and/or prevention of the aging of the skin. The treatment and/or prevention is skin aging includes the alleviation and/or the prevention of symptoms of skin aging. The symptoms of skin aging include the appearance of wrinkles and the loss of skin biomechanical properties such as firmness. The loss of firmness can be due to the reduction in collagen production in the skin or the loss of muscle tone with age. Particularly, the loss of muscle tone refers to cutaneous muscles, more particularly to cutaneous facial muscles. The symptoms of skin aging include a loss of volume due to a loss of of adipose tissue.

In one embodiment, there is provided the use of the compound of the invention, a stereoisomer and/or cosmetically acceptable salt thereof, or a cosmetic composition comprising the compound of the invention, a stereoisomer and/or cosmetically acceptable salt thereof, for the treatment and/or prevention of skin wrinkles. Skin wrinkles include expression wrinkles, also commonly referred as expression lines.

In one embodiment, there is provided the use of the compound of the invention, a stereoisomer and/or cosmetically acceptable salt thereof or a cosmetic composition comprising the compound of the invention, a stereoisomer and/or cosmetically acceptable salt thereof, for maintaining and/or improving skin firmness.

In one embodiment, there is provided the use of the compound of the invention, a stereoisomer and/or cosmetically acceptable salt thereof, or a cosmetic composition comprising the compound of the invention, a stereoisomer and/or cosmetically acceptable salt thereof, for the stimulation of collagen synthesis and/or prevention of collagen loss.

In one embodiment, there is provided the use of the compound of the invention, stereoisomer and/or cosmetically acceptable salt thereof, or a cosmetic composition comprising the compound of the invention, a stereoisomer and/or cosmetically acceptable salt thereof, for increasing the volume of adipose tissue; and/or preventing and/or alleviating the effects of adipose tissue loss. The adipose tissue can be subcutaneous adipose tissue and can be subcutaneous adipose tissue of the face, hands and/or neck (particularly the part of the neck). Increasing the volume of adipose tissue in the skin will cause an increase in volume of the skin. In particular, the adipose tissue loss is due to ageing.

In one embodiment, there is provided the use of the compound of the invention, a stereoisomer and/or cosmetically acceptable salt thereof, or a cosmetic composition comprising the compound of the invention, a stereoisomer and/or cosmetically acceptable salt thereof, for the treatment and/or prevention of a sagging appearance of the skin. Sagging appearance can be caused by loss of muscle tone, particularly cutaneous muscle tone, more particularly facial muscles.

In one embodiment, there is provided the use of the compound of the invention, a stereoisomer and/or cosmetically acceptable salt thereof, or a cosmetic composition comprising the compound of the invention, a stereoisomer and/or cosmetically acceptable salt thereof, for the reduction and/or prevention of facial assymmetry.

The invention also extends to the use of combination of the compound of the invention with a Botulinum toxin, Ac-Glu-Glu-Met-Gln-Arg-Arg-NH₂, or H-Tyr-D-Ala-Gly-Phe-Leu-OH or combinations thereof, in the treatment and/or care of the skin, hair, nails and/or mucous membranes as described above in relation to the applications (uses) of the compounds of the invention.

In another aspect, the invention provides a cosmetic, non-therapeutic method of treatment and/or care of the skin, hair, nails and/or mucous membranes in a subject comprising administering a cosmetically effective amount of a compound of the invention, a stereoisomer and/or cosmetically acceptable salt thereof or a cosmetic composition comprising a cosmetically effective amount of the compound of the invention, a stereoisomer and/or a cosmetically acceptable salt thereof, to the subject. The method can be for the treatment and/or care of the skin, hair, nails and/or mucous membranes as described above in relation to the applications (uses) of the compounds and compositions of the invention. In particular, the cosmetic, non-therapeutic method of treatment and/or care is that of the skin. The administration can be topical or, for example, transdermal. In this aspect of the invention, the compound of the invention may be present in a cosmetic composition such as the cosmetic composition as described herein. In one embodiment, the method involves administering the compound or administering the composition using microneedles.

The invention also extends to a method of treatment and/or care of the skin, hair, nails and/or mucous membranes of a subject comprising administering a combination of the compound of the invention, a stereoisomer and/or cosmetically or pharmaceutically acceptable salt thereof with a Botulinum toxin, Ac-Glu-Glu-Met-Gln-Arg-Arg-NH₂, or H-Tyr-D-Ala-Gly-Phe-Leu-OH or combinations thereof, to the subject. The method can be for the treatment and/or care of the skin, hair, nails and/or mucous membranes as described above in relation to the applications (uses) of the compounds of the invention. For example, the method of treatment can comprise administering a Botulinum toxin and a compound of the invention, a stereoisomer and/or cosmetically or pharmaceutically acceptable salt thereof to the subject. For example, the method of treatment can comprise administering: Ac-Glu-Glu-Met-Gln-Arg-Arg-NH₂, or H-Tyr-D-Ala-Gly-Phe-Leu-OH or combinations thereof; and a compound of the invention, a stereoisomer and/or cosmetically acceptable salt thereof, to the subject. Preferably, this method of treatment is a skin antiaging treatment.

The Botulinum toxin, Ac-Glu-Glu-Met-Gln-Arg-Arg-NH₂, or H-Tyr-D-Ala-Gly-Phe-Leu-OH or combinations thereof and the compound of the invention can be administered simultaneously (at the same time) or administered one after the other. When the Botulinum toxin, Ac-Glu-Glu-Met-Gln-Arg-Arg-NH₂, or H-Tyr-D-Ala-Gly-Phe-Leu-OH or combinations thereof, and the compound or composition of the invention are administered at the same time, they can be administered as a separate dosage forms or as a part of a single composition. When the products are administered in separate dosage forms, the dosage forms can be in the same or different containers.

The above methods of treatment include the cosmetic, non-therapeutic treatment and/or care of the skin, including: the treatment and/or prevention of the aging of the skin, the treatment and/or prevention of skin wrinkles; maintaining and improving skin firmness; the stimulation of collagen synthesis and/or prevention of collagen loss; the treatment and/or prevention of a sagging appearance of the skin; the reduction and/or prevention of facial asymmetry; the increase of the volume of adipose tissue; and/or the prevention and/or alleviation of the effects of adipose tissue loss. The above methods of treatment include the cosmetic, non-therapeutic treatment and/or care of the skin, including: the treatment and/or prevention of the aging of the skin, the treatment and/or prevention of skin wrinkles; maintaining and improving skin firmness; the stimulation of collagen synthesis and/or prevention of collagen loss; the treatment and/or prevention of a sagging appearance of the skin; and/or the reduction and/or prevention of facial assymmetry. In one embodiment, the cosmetic, non-therapeutic method of treatment and/or care of the skin is a skin antiaging treatment.

For the above described methods of the invention, topical or transdermal application can be carried out by iontophoresis, sonophoresis, electroporation, mechanical pressure, osmotic pressure gradient, occlusive cure, microinjections, by needle-free injections by means of pressure, by microelectric patches, face masks or any combination thereof.

For the above described methods of the invention, the frequency of application or administration can vary greatly, depending on the needs of each subject, with a recommendation of an application from once a month to ten times a day, preferably from once a week to four times a day, more preferably from three times a week to twice a day, even more preferably once a day. For example the frequency of the administration according to the method of treatment and/or care of the skin, hair, nails and/or mucous membranes of a subject comprising administering a combination of the compound of the invention, a stereoisomer and/or cosmetically or pharmaceutically acceptable salt thereof with a Botulinum toxin, Ac-Glu-Glu-Met-Gln-Arg-Arg-NH₂, or H-Tyr-D-Ala-Gly-Phe-Leu-OH or combinations thereof, to the subject, can vary widely, depending on the need of each subject. In one embodiment, method of the invention comprises the administration of Botulinum toxin, followed by the administration of the compound or compositions of the invention. In a particular embodiment, after the administration of the Botulinum toxin, the compound or compositions of the invention are administered at least once a day for at least one week. More particularly, the compound or compositions of the invention are administered at least once a day until the next administration of Botulinum toxin.

In another aspect, the invention provides a method for selecting a compound useful for the prevention or the treatment of the symptoms of skin aging, the improvement or maintenance of skin firmness, the treatment and/or prevention of a sagging appearance of the skin, and/or the treatment or prevention of facial asymmetry comprising determining the ability of the compound to upregulate MLBN-1 in human skeletal muscle cells.

### Compositions of the invention

The compounds of the invention can be administered for their application by any means that causes contact between the compounds and the site of action in a subject's body, preferably that of a mammal, preferably a human, and in the form of a composition which contains them.

In another aspect, the invention provides a composition comprising a compound according to formula (I), a stereoisomer and/or a cosmetically or pharmaceutically acceptable salt thereof.

In particular, the invention provides a cosmetic composition comprising a compound according to formula (I), a stereoisomer and/or a cosmetically acceptable salt thereof, together with at least one cosmetically acceptable excipient or adjuvant. These compositions can be prepared by conventional means known to persons skilled in the art *["*Harry's Cosmeticology", Seventh edition, (1982), Wilkinson J.B., Moore R.J., ed. Longman House, Essex, GB*].*

The compounds of this invention have variable solubility in water, according to the nature of their amino acid sequence or any possible modifications in the N-terminal and/or C-terminal ends. Therefore, the compounds of this invention can be incorporated into the compositions by aqueous solution, and those which are not soluble in water can be solubilized in cosmetically acceptable conventional solvents such as and not restricted to, ethanol, propanol, isopropanol, propylene glycol, glycerin, butylene glycol or polyethylene glycol or any combination thereof.

The cosmetically effective amount of the compounds of the invention which should be administered, as well as their dosage, will depend on numerous factors, including age, state of the patient, the nature or severity of the condition to be treated and/or cared for, the route and frequency of administration and of the particular nature of the compounds to be used.

The term "cosmetically effective amount" is understood to mean a non-toxic but sufficient amount of the compound or compounds of the invention to provide the desired effect. The compounds of the invention are used in the cosmetic compositions of this invention at cosmetically effective concentrations to achieve the desired effect; for example in amounts with respect to the total weight of the composition of: from 0.00000001% (in weight) to 20% (in weight); from 0.000001% (in weight) to 15% (in weight), from 0.00001% (in weight) to 10% (in weight); or from 0.0001% (in weight) to 5% (in weight).

The compounds of formula (I), their stereoisomers, mixtures thereof and/or their cosmetic acceptable salts, can also be incorporated into cosmetic delivery systems and/or sustained release systems.

The term "delivery system" relates to a diluent, adjuvant, excipient or carrier with which the compound of the invention is administered. These cosmetic carriers can be liquids, such as water, oils or surfactants, including those of petroleum, animal, plant or synthetic origin, for example and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin and similar. A person skilled in the art knows the diluents, adjuvants or excipients which can be used in the different delivery systems in which the compound of the invention can be administered.

The term "sustained release" is used in a conventional sense relating to a delivery system of a compound which provides the gradual release of this compound during a period of time and preferably, although not necessarily, with relatively constant compound release levels over a period of time.

Examples of delivery or sustained release systems include, without restriction, liposomes, mixed liposomes, oleosomes, niosomes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as in microemulsions and nanoemulsions, which can be added to achieve a greater penetration of the active principle and/or improve its pharmacokinetic and pharmacodynamic properties. Preferred delivery or sustained release systems are liposomes, surfactant-phospholipid mixed micelles, microemulsions, more preferably water-in-oil microemulsions with an internal structure of reverse micelle and nanocapsules containing microemulsions.

In one embodiment, the invention provides a cosmetic composition comprising a compound of formula (I) and a cosmetically acceptable carrier selected from the group consisting of creams, emulsions, gels, liposomes, nanoparticles and ointments.

The sustained release systems can be prepared by methods known in the prior art, and the compositions which contain them can be administered, for example, by topical or transdermal administration, including adhesive patches, non-adhesive patches, occlusive patches and microelectric patches, or by systemic administration, for example and not restricted to, oral or parenteral route, including nasal, rectal or subcutaneous implantation or injection, or direct implantation or injection into a specific body part, and preferably should release a relatively constant quantity of the compounds of the invention. The amount of compound contained in the sustained release system will depend, for example, on where the composition is to be administered, the kinetics and duration of the release of the compound of the invention, as well as the nature of the condition to be treated and/or cared for.

The compounds of this invention can also be adsorbed on solid organic polymers or solid mineral supports such as and not restricted to, talc, bentonite, silica, starch or maltodextrin among others.

The compositions which contain the compounds of formula (I), their stereoisomers, mixtures thereof and/or their cosmetically acceptable salts can also be incorporated into fabrics, non-woven fabrics and medical devices which are in direct contact with the skin, thus releasing the compounds of the invention whether by biodegradation of the binding system to the fabric, non-woven fabric or medical device, or by friction between them and the body, due to bodily moisture, the skin's pH or body temperature. Furthermore, the compounds of the invention can be incorporated into the fabrics and non-woven fabrics used to make garments that are in direct contact with the body.

Examples of fabrics, non-woven fabrics, garments, medical devices and means for immobilizing the compounds to them, among which are the delivery systems and/or the sustained release systems described above, can be found in literature and are known in the prior art *[*Schaab C.K. (1986) HAPPI May 1986; Nelson G., "Application of microencapsulation in textiles", (2002), Int. J. Pharm., 242(1-2), 55-62; *"*Biofunctional Textiles and the Skin" (2006) Curr. Probl. Dermatol. v.33, Hipler U.C. and Elsner P., eds. S. Karger AG, Basel, Switzerl*and;* Malcolm R.K. et al., "Controlled release of a model antibacterial drug from a novel self-lubricating silicone biomaterial", (2004), J. Cont. Release, 97(2), 313-320*].* The preferred fabrics, non-woven fabrics, garments and medical devices are bandages, gauzes, t-shirts, socks, tights, underwear, girdles, gloves, diapers, sanitary napkins, dressings, bedspreads, wipes, adhesive patches, non-adhesive patches, occlusive patches, microelectric patches and/or face masks.

The cosmetic compositions which contain the compounds of the invention, their stereoisomers, mixtures thereof and/or their cosmetically or pharmaceutically acceptable salts, can be used in different types of compositions for topical or transdermal application which optionally include cosmetically or pharmaceutically acceptable excipients necessary for formulating the desired administration form.

The compositions for topical or transdermal application can be produced in any solid, liquid or semisolid formulation, such as and not restricted to, creams, multiple emulsions such as and not restricted to, oil and/or silicone in water emulsions, water-in-oil and/or silicone emulsions, water/oil/water or water/silicone/water type emulsions and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These topical or transdermal application formulations can be incorporated using techniques known by the person skilled in the art into different types of solid accessories for example and not restricted to, bandages, gauzes, t-shirts, socks, tights, underwear, girdles, gloves, diapers, sanitary napkins, dressings, bedspreads, wipes, adhesive patches, non-adhesive patches, occlusive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders among others.

The cosmetic compositions of the invention may include agents which increase the percutaneous absorption of the compounds of the invention, for example and not restricted to, dimethylsulfoxide, dimethylacetamide, dimethylformamide, surfactants, azone (1-dodecylazacycloheptane-2-one), alcohol, urea, ethoxydiglycol, acetone, propylene glycol or polyethylene glycol, among others. Furthermore, the cosmetic or pharmaceutical compositions of this invention can be applied to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the peptide of the invention. The application area will be determined by the nature of the condition to be treated and/or cared for.

Furthermore, the compositions containing the compounds of formula (I), their stereoisomers, mixtures thereof and/or their cosmetically acceptable salts can be used in different types of formulations for oral administration, preferably in the form of oral cosmetics such as and not restricted to, capsules, including gelatin capsules, soft capsules, hard capsules, tablets, including sugar coated tablets, tablets, pills, powders, granules, chewing gum, solutions, suspensions, emulsions, syrups, elixirs, polysaccharide films, jellies or gelatins, and any other form known by the person skilled in the art. In a particular embodiment, the compounds of the invention can be incorporated into any form of functional food or fortified food, such as and not restricted to, dietary bars or compact or non-compact powders. These powders can be dissolved in water, soda, dairy products, soy derivatives or can be incorporated into dietary bars. The compounds of this invention can be formulated with common excipients and adjuvants for oral compositions or food supplements, for example and not restricted to, fat components, aqueous components, humectants, preservatives, texturizing agents, flavors, aromas, antioxidants and colorants common in the food industry.

Cosmetic compositions containing the compounds of formula (I), their stereoisomers, mixtures thereof and/or their cosmetically acceptable salts can also be administered, as well as by topical or transdermal route, by any other appropriate route, such as oral or parenteral route, for which they will include the cosmetically acceptable excipients necessary for the formulation of the desired administration form. In the context of this invention, the term "parenteral" includes nasal, auricular, ophthalmic, rectal, urethral, vaginal, subcutaneous, intradermal route, intravascular injections, such as intravenous, intramuscular, intraocular, intravitreous, intracorneal, intraspinal, intramedullary, intracranial, intracervical, intracerebral, intrameningeal, intraarticular, intrahepatic, intrathoracic, intratracheal, intrathecal and intraperitoneal, and any another similar injection or infusion technique. A person skilled in the art knows the different means by which the cosmetic compositions which contain the compounds of the invention can be administered.

Among the cosmetically acceptable adjuvants contained in the cosmetic or pharmaceutical compositions described in this invention are additional ingredients commonly used in cosmetic or pharmaceutical compositions, for example and not restricted to anti-wrinkle agents, botox-like agents and/or anti-aging agents; (ii) firming agents, skin elasticity agents and/or restructuring agents; moisturizing agents; (iv) anti-photoaging agents, and/or blue-light protector agents; DNA protecting agents, DNA repair agents, and/or stem cell protecting agents; free radical scavengers and/or anti-glycation agents, detoxifying agents, antioxidant and/or anti-pollution agents; antiperspirant agents; melanin synthesis stimulating or inhibiting agents; whitening or depigmenting agents; propigmenting agents; self-tanning agents; lipolytic agents or agents stimulating lipolysis, adipogenic agents, etc. Additional examples can be found in *CTFA International Cosmetic Ingredient Dictionary & Handbook, 12th Edition (2008).*

In one embodiment, the invention provides a cosmetic composition comprising a compound of formula (I) and a pharmaceutically or cosmetically effective amount of an adjuvant selected from the group consisting of: (i) anti-wrinkle-agent, botox-like agent and/or anti-aging agent; (ii) firming agent, skin elasticity agent and/or restructuring agent; (iii) moisturizing agent; (iv) anti-photoaging agent, and/or blue-light protector agent; (v) DNA protecting agent, DNA repair agent, and/or stem cell protecting agent; (vi) free radical scavengers and/or anti-glycation agent, detoxifying agent, antioxidant and/or anti-pollution agents; and/or combinations thereof.

In a particular embodiment, the anti-wrinkle agent, botox-like agent and/or anti-aging agent is selected from thee group consisting of Matrixyl^{®} [INCI: Palmitoyl Pentapeptide-4], Matrixyl^{®} 3000^{®} [INCI: Palmitoyl Tetrapeptide-7, Palmitoyl Oligopeptide], Matrixyl^{®} Synthe'6 [INCI: Glycerin, Water, Hydroxypropyl Cyclodextrin, Palmitoyl Tripeptide-38], Matrixyl^{®} Morphomics^{™} [INCI: Pentylene Glycol, Caprylyl Glycol], Essenskin^{™} [INCI: calcium hydroxymethionine], Renovage [INCI: Teprenone], Dermaxyl^{®} [INCI: Palmitoyl Oligopeptide], Calmosensine [INCI: Butylene Glycol, Acetyl Dipeptide-1 Cetyl Ester], Volulip [INCI: Cetearyl Ethylhexanoate, Sorbitan Isostearate, Portulaca Pilosa Extract, Sucrose Cocoate, Palmitoyl Tripeptide-38], Subliskin [INCI: Sinorhizobium Meliloti Ferment, Cetyl Hydroxyethyl Cellulose, Lecithin], Biopeptide CL [INCI: Palmitoyl Oligopeptide], Biopeptide EL [INCI: Palmitoyl Oligopeptide], Rigin [INCI: Palmitoyl Tetrapeptide-3], Biobustyl [INCI: Glyceryl Polymethacrylate, Rahnella/Soy Protein Ferment, Palmitoyl Oligopeptide], Dynalift [INCI: Sodium Polystyrene Sulfonate, Sorghum Bicolor Stalk Juice, Glycerin], Idealift [INCI: Acetyl Dipeptide-1 Cetyl Ester], Siegesbeckia [INCI: Siegesbeckia Orientales Extract], Ovaliss [INCI: Coco-glucoside, Caprylyl Glycol, Alcohol, Glaucine], Juvinity^{™} [INCI: Geranylgeranyisopropanol], Prolevis [INCI: Hydrolyzed Vegetable Protein], Idealift^{™} [INCI: Hydroxyethylcellulose, Acetyl Dipeptide-1 cetyl ester], Beautifeye^{™} [INCI: Albizia Julibrissin Bark Extract, Darutoside], Chromocare^{™} [INCI: Sigesbeckia Orientalis Extract, Rabdosia Rubescens Extract] or Resistem^{™} [INCI proposed: Globularia Cordifolia Ferment] marketed by Sederma/Croda. Vialox^{®} [INCI: Pentapeptide-3], Syn^{®}-Ake^{®} [INCI: Dipeptide Diaminobutyroyl Benzylamide Diacetate], Syn^{®}-Coll [INCI: Palmitoyl Tripeptide-5], Phytaluronate [INCI: Ceratonia Siliqua (Carob) Gum], Preregen^{®} [INCI: Glycine soja (Soybean) Protein, Oxido Reductases], Pepha-Nutrix [INCI: Natural Nutrition Factors], Pepha-Tight [INCI: Algae Extract, Pullulan], Pentacare-NA [INCI: Hydrolyzed Wheat Gluten, Ceratonia Siliqua Gum], Syn^{®}-Tacks [INCI: Glycerin, Palmitoyl Dipeptide-5 Diaminobutyloyl Hydroxythreonine, Palmitoyl Dipeptide-6 Diaminohydroxybutyrate], BeauActive MTP [INCI: Hydrolyzed milk protein], Syn^{®}-TC [INCI: Tetradecyl Aminobutyroylvalylaminobutyric Urea Trifluoroacetat, Palmitoyl Tripeptide-5, Palmitoyl Dipeptide-5 Diaminobutyroyl Hydroxythreonine], Syn^{®}-Hycan [INCI: Tetradecyl Aminobutyroylvalylaminobutyric Urea Trifluoroacetate], Syn^{®}-Glycan [INCI: Tetradecyl Aminobutyroylvalyl-aminobutyric Urea Trifluoroacetate], Regu-Age [INCI: Hydrolyzed Rice Bran Protein, Oxido Reductases, Glycine Soja Protein], Pepha-Timp [INCI: Human oligopeptide-20], Pepha-Age [INCI: Dunaliella Salina Extract], Colhibin [INCI: Hydrolyzed Rice Protein], Elhibin [INCI: Glycine Soja Protein, Disodium cocoamphodiacetate] or All-Q^{™} Plus [INCI: Ubiquinone, Tocopheryl Acetate] marketed by Pentapharm/DSM; Myoxinol^{™} [INCI: Hydrolyzed Hibiscus esculentus Extract], Myoxinol^{™} LS 9736 [INCI: Hydrolyzed Hibiscus esculentus Extract, Dextrin], Syniorage^{™} [INCI: Acetyl Tetrapeptide-11], Dermican^{™} [INCI: Acetyl Tetrapeptide-9], DN-AGE^{®} LS [INCI: Cassia alata leaf Extract], Hyalufix GL [INCI: Alpinia Galanga Leaf Extract], Neurobiox [INCI: Achillea Millefolium Extract,], Deliner [INCI: Zea Mays (Corn) Kernel Extract], Lys'lastine V [INCI: Peucedanum Graveolens (Dill) Extract], Extracellium [INCI: Hydrolyzed Potato Protein], Proteasyl TP LS 8657 [INCI: Pisum Sativum Extract], Flavagrum PEG [INCI: PEG-6 Isostearate, Hesperetin Laurate], Micromerol [INCI: Pyrus Malus Fruit Extract], Extracellium [INCI: Hydrolyzed Potato Protein], Marine Filling Spheres [INCI: Pentaerythrityl Tetraisostearate, Silica Dimethyl Silylate, Sodium Chondroitin Sulfate, Atelocollagen], Triactigen [INCI: Mannitol, Cyclodextrin, Yeast Extract, Disodium Succinate], Eterniskin [INCI: Grifola Frondosa Fruiting Body Extract, Maltodextrin], Ascotide [INCI: Ascorbyl Phosphate Succinoyl Pentapeptide-12], Hyalurosmooth [INCI: Cassia Angustifolia Seed Polysaccharide], Indinyl CA [INCI: Cassia Angustifolia Seed Polysaccharide], Arganyl [INCI: Argania Spinosa Leaf Extract], Sphingoceryl Veg [INCI: Phyto-ceramides], Vit-ALike [INCI: Vigna Acontifolia Seed Extract], Peptiskin [INCI: Arginine/Lysine polypeptide], Prodejine [INCI: Mannitol, Cyclodextrin, Yeast Extract, Disodium Succinate], Aqu'activ [INCI: Behenyl Alcohol, Glyceryl Oleate, Cocamide MIPA, Calcium Citrate], Elestan [INCI: Glycerin, Manilkara Leaf Extract], Hibiscin HP [INCI: Hibiscus Esculentus Seed Extract], Collalift^{®}18 [INCI: Khaya Senegalensis Bark], Collrepair^{™} DG [INCI: Hexylene Glycol, Niacin] or Litchiderm [INCI: Litchi Chinensis Pericarp Extract] marketed by Laboratoires Sérobiologiques/Cognis/BASF; Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18], Inyline^{®} [INCI: Acetyl Hexapeptide-30], Aldenine^{®} [INCI: Hydrolized Wheat Protein, Hydrolized Soy Protein, Tripeptide-1], Preventhelia^{®} [INCI: Diaminopropionoyl Tripeptide-33], Decorinyl^{®} [INCI: Tripeptide-10 Citrulline], Decorinol^{®} [INCI: Tripeptide-9 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Eyeseryl^{®} [INCI: Acetyl Tetrapeptide-5], Peptide AC29 [INCI: Acetyl Tripeptide-30 Citrulline], Relistase^{®} [INCI: Acetylarginyltriptophyl Diphenylglycine], Thermostressine^{®} [INCI: Acetyl Tetrapeptide-22], Lipochroman^{™} [INCI: Dimethylmethoxy Chromanol], Chromabright^{®} [INCI: Dimethylmethoxy Chromanyl Palmitate], Antarcticine^{®} [INCI: Pseudoalteromonas Ferment Extract], dGlyage^{®} [INCI: Lysine HCl, Lecithin, Tripeptide-9 Citrulline], Vilastene^{™} [INCI: Lysine HCl, Lecithin, Tripeptide-10 Citrulline], Hyadisine^{®} [INCI: Pseudoalteromonas Ferment Extract], Hyanify^{™} [INCI: Saccharide Isomerate], Diffuporine^{®} [INCI: Acetyl Hexapeptide-37], Silusyne^{®} [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39], Adifyline^{®} [INCI: Acetyl Hexapeptide-38], Delisens^{™} [INCI: Acetyl Hexapeptide-46], Telangyn^{™} [INCI: Acetyl Tetrapeptide-40], Reproage^{™} peptide [INCI: Acetyl Hexapeptide-8], Cellynkage^{™} marine ingredient [INCI: Saccharide Isomerate], Eyedeline^{™} marine ingredient [INCI: Plankton Extract], uplevity^{™} [INCI: Acetyl Tetrapeptide-2], Seacode^{™} marine ingredient [INCI: Pseudoalteromonas Ferment Extract] or Serilesine^{®} peptide solution [INCI: Hexapeptide-10] marketed by Lipotec/Lubrizol; Sirtalice^{™} [INCI: Bacillus Ferment], Epitensive^{™} [INCI: Nicotiana Benthamiana Hexapeptide-40 SH-Oligopeptide-1], Scelleye^{™} [INCI: Nicotiana Benthamiana SH-Oligopeptide-2], Seadermium [INCI: Aqua, Glycerin, Bacillus Ferment], Pauseile [INCI: Aqua, Glycerin, Bacillus Ferment] or Neoclair pro [INCI: Aqua, Glycerin, Caprylyl Glycol, Acetyl Tetrapeptide-2] marketed by Lipotrue; Collaxyl^{®} IS [INCI: Hexapeptide-9], Laminixyl IS^{™} [INCI: Heptapeptide], Orsirtine^{™} GL [INCI: Oryza sativa (Rice) Extract], D'Orientine^{™} IS [INCI: Phoenix dactylifera (Date) Seed Extract], Phytoquintescine^{™} [INCI: Einkorn (Triticum monococcum) Extract], Quintescine^{™} IS [INCI: Dipeptide-4], Peptide Vinci 01 [INCI: Penta-decapeptide-1], Peptide Vinci 02^{™} [INCI: Hexapeptide-3], Aquarize IS^{™} [INCI: Hydrolyzed Rice Extract], Lanablue [INCI: Algae extract], Ederline^{™} [INCI: Pyrus Malus (Apple) Seed Extract], Dynachondrine^{™} ISR [INCl:Hydrolized Soy Protein], Prolixir S20^{™} [INCI: Dimer Tripeptide-43], Phytocohesine^{™} PSP [INCI: Sodium Beta-Sitosteryl Sulfate, Beta-Sitosterol], Perenityl^{™} IS [INCI: Pyrus Communis (Pear) Seed Extract], Caspaline 14^{™} [INCl:Hexapeptide-42], Peptide Q10^{™} [INCl:Pentapeptide-34 Trifluoroacetate], Survixyl IS^{™} [INCI: Pentapeptide-31], ChroNOgen^{™} [INCI: Tetrapeptide-26], Elixiance [INCI: Schinus Molle Extract], Harmoniance^{™} [INCI: Nelumbo Nucifera Flower Extract], Serenityl [INCI: Marsdenia Condurango Bark Extract], Natriance Wrinkle-less [INCI: Hydrolyzed Corn Protein], Phytoneomatrix [INCI: Hydrolyzed Soybean Extract], Prolixir ICE [INCI: Hydrolyzed Rice Protein], PhytoRNx Baobab^{™} [INCI: Hydrolyzed Adansonia Digitata Extract], Natriance Renovate Extract [INCI: Hydrolyzed Linseed Extract], Natriance Self-Hydrate Extract [INCI: Pisum Sativum Extract], Actopontine YST [INCI: Hydrolyzed Yeast Protein] or Telosense^{™} [proposed INCl: Hydrolized Soy Protein, Hydrolized Yeast Protein] marketed by Vincience/ISP/Ashland; BONT-L-Peptide [INCI: Palmitoyl Hexapeptide-19], TIMP Peptide [INCI: Acetylhexapeptide-20], ECM Moduline [INCI: Palmitoyl Tripeptide-28], Renaissance [INCI: Hydrolyzed Wheat Protein, Palmitoyl Decapeptide-21, Decapeptide-22, Oligopeptide-78, Zinc Palmitoyl Nonapeptide-14] or X50 Antiaging [INCI: Lactic Acid/glycolic Acid Copolymer, Polyvinyl Alcohol, Copper Palmitoyl Heptapeptide-14, Heptapeptide-15 Palmitate] marketed by Infinitec Activos; EquiStat [INCI: Pyrus malus Fruit Extract, Glycine soja Seed Extract], Juvenesce [INCI: Ethoxydiglicol and Caprylic Triglycerid, Retinol, Ursolic Acid, Phytonadione, Ilomastat], Ursolisome [INCI: Lecithin, Ursolic Acid, Atelocollagen, Xanthan Gum, Sodium chondroitin sulfate], Basaline [INCI: Hydrolyzed Malt Extract], Phytokine [INCI: Hydrolyzed Soy Protein], marketed by Coletica/Engelhard/BASF; Ameliox [INCI: Carnosine, Tocopherol, Silybum marianum Fruit Extract] or PhytoCellTec Malus Domestica [INCI: Malus domestica Fruit Cell Culture], Lipobelle Soyaglicane [INCI: Soy Isoflavones], RoyalEpigen P5 [INCI: Butyrospermum Parkii BUtter, Hydrogenated Lecithin, Maltodextrin, Pentapeptide-48, Phenethyl Alcohol, Ethylhexylglycerin, Glycerin, Aqua] or DermCom [INCI: Crocus Chrysanthus Bulb Extract, Acacia Senegal Gum, Aqua/Water] marketed by Mibelle Biochemistr;ActiMatrix [INCI: Peptide based mushroom Extract], Peptamide 6 [INCI: Hexapeptide-11] marketed by Active Organics/Arch; and combinations thereof.

In another embodiment, the firming agent, skin elasticity agent and/or restructuring agent is selected, from the group consisting of Argassential [INCI: C10-16 Alkyl Glucoside, Dicaprylyl Ether, Glycerin] or Replexium BC [INCI: Dimethyl Isosorbide, Polysorbate 20, Aqua, Acetyl Tetrapeptide-11, Acetyl Tetrapeptide-9] marketed by BASF; Prolevis [INCI: Hydrolyzed Vegetable Protein] or Poretect [INCI: Caprylic/capric Triglyceride, Sorbitan Trioleate, Apium Graveolens Seed Extract, Linum Usitatissimum Seed Extract] marketed by Sederma/Croda; Actifirm Ultra Advanced botanical ingredient [INCI: Centella Asiatica Extract, Rosmarinus Officinalis Leaf Extract, Dipropylene Glycol, Alcohol, Echinacea Angustifolia Leaf Extract] or Actifcol Advanced botanical ingredient [INCI: Aqua, Glycerin, Sodium Citrate, Lentinus Edodes Extract, Potassium Sorbate, Sodium Benzoate, Phytic Acid] marketed by Lipotec/Lubrizol; Densorphin^{™} [INCI: Vitex Agnus Castus Extract, Aqua, Maltodextrin] or PhytoCellTec^{™} nunatak^{®} [INCI: Isomalt, Aqua, Saponaria Pumila Callus Culture Extract, Lecithin] marketed by Mibelle; and combinations thereof.

In another embodiment, the moisturizing agent is selected from the group consisting of qua Shuttle [INCI: Sorbitol, Laminaria Digitata Extract, Diatomaceous Earth] marketed by Infinitec; Aqua-Osmoline^{™} [INCI: Ceratonia Siliqua (Carob) Seed Extract] marketed by Vincience/ISP/Ashland; Hydralphatine^{™} Asia [INCI: Hydrogenated Starch Hydrolysate, Panthenol, Bambusa Vulgaris Shoot Extract, Nelumbo Nucifera Flower Extract, Nymphaea Alba Root Extract] or Hydraporine^{™} [INCI: Betaine, Hydrogenated Lecithin, Honey, Pectin] marketed by Lucas Meyer Cosmetics/Unipex; PatcH2O^{™} [INCI: Trehalose, Urea, Serine, Glyceryl Polyacrylate, Algin, Sodium Hyaluronate, Pullulan], Aqu'activ^{™} [INCI: Behenyl Alcohol, Glyceryl Oleate, Cocamide MIPA], Irwinol^{®} [INCI: Octyldodecanol, Irvingia Gabonensis Kernel Butter, Hydrogenated Coco-Glycerides], Lipodermol^{®}) [INCI: Octyldodecanol, Arachidyl Propionate, Tocopheryl Acetate, Retinyl Palmitate, Ethyl Linoleate, Ethyl Linolenate] or Seanamin^{®} SU [INCI: Sorbitol, Algae Extract, Chrondrus Crispus (Carrageenan), Fucus Vesiculosus Extract, Algin] marketed by L. Serobiologiques/Cognis/BASF; Snow Algae Powder [INCI: Coenochloris Signiensis Extract] marketed by Mibelle; Hyasol BT [INCI: Sodium Hyaluronate], Syn-Up^{™} [INCI: Benzylsulfonyl D-Seryl Homophenylalanine Amidinobenzamide Acetate] or Pentavitin^{®} [INCI: Saccharide Isomerate] marketed by Pentapharm/DSM; • Aqualance^{™} [INCI: Erythritol, Homarine HCl], Hydraprotectol^{™} [INCI: Glyceryl Polymethacrylate, Aleuritic Acid, Yeast Extract (Faex), Glycoprotein], Moist 24^{™} [INCI: Imperata Cylindrica Root Extract], Optim Hyal^{™} [INCI: Hydrolyzed Yeast Extract, Cetyl Hydroxyethylcellulose, Polyglucuronic Acid], Osmocide^{®} 4 [INCI: Glycerin, Acrylates/C10-30 Alkyl Acrylate Crosspolymer] or Revidrate^{™} [INCI: Ethylhexyl Palmitate, Sorbitan Oleate, Sorbitan Laureate, Myristyl Malate Phosphonic Acid] marketed by Sederma/Croda; Xpertmoist^{®} molecular film [INCI: Glycerin, Pseudoalteromonas Ferment Extract, Xanthan Gum, Proline, Alanine, Serine, Ethylhexylglycerin, Caprylyl Glycol] or Actizyme GL advanced botanical ingredient [INCI: Glycerin, Mucor miehei extract, Aqua, Sodium Citrate, Potassium Sorbate, Sodium Benzoate, Phytic Acid] marketed by Lipotec/Lubrizol; and combinations thereof.

In another embodiment, the anti-photoaging agent, and/or blue-light protector agent is selected from the group consisting of Algaktiv Genofix CPD [INCI: Plankton Extract, Aqua, Lecithin] marketed by Greenaltech; Blumilight^{™} Biofunctional [INCI proposed: Water/Aqua (and) Butylene Glycol (and) Theobroma Cacao (Cocoa) Seed Extract] marketed by Ashland; Lys'Sun [INCI: Hamamelis Virginiana Leaf Extract, Aqua, Pentylene Glycol, Caprylyl Glycol, Xanthan Gum] marketed by BASF; Vitachelox [INCI: Vitis Vinifera Seed Extract, Camellia Sinensis Leaf Extract, Quercus Robur Wood Extract] marketed by Indena; L-VCG [INCI: Ascorbyl Glucoside]marketed by Freshine Bio-technology; Lumicease blue ingredient [INCI: Glycerin, Aqua, Hydrolyzed Pea Protein, Glucose, Sodium Chloride] marketed by Lipotec/Lubrizol; Lightwaves Defense [JS+M] [INCI: Jasminum Sambac Leaf Cell Extract] marketed by Naolys; Blue Oleoactif [INCI: Glycine Soja Oil, Polyglyceryl-3 Diisostearate, Oryza Sativa Germ Extract, Oryza Sativa Extract] marketed by Oleos-Hallstar; Majestem [INCI: Glycerin, Leontopodium Alpinum Callus Culture Extract, Xanthan Gum] or Senestem [INCI: Glycerin, Plantago Lanceolata Leaf Extract, Xanthan Gum] marketed by Sederma; Blueshield [INCI: Glycerin, Capsicum Annuum Fruit Extract, Xanthan Gum] marketed by Solabia; and combinations thereof.

In another embodiment, a DNA protecting agent, DNA repair agent, and/or stem cell protecting agent is selected from the group consisting of; GP4G SP [INCI: Aqua, Glycerin, Aretmia Extract], Heliostatine [INCI: Aqua, Glycerin, Pisum Sativum Extract], Orsirtine [INCI: Aqua, Glycerin, Oryza Sativa Extract], Chronogen [INCI: Aqua, Butylene Glycol, Tetrapeptide (INCI proposed)], Survixyl IS [INCI: Water, Butylene Glycol, Pentapeptide-31] and Chrondricare [INCI: Aqua, Butylene Glycol Pentapeptide-28] marketed by Vincience/ISP/Ashland; Lanacityn^{®} [INCI: Glycerin, Aqua, Alteromonas ferment extract, Chysanthellum indicum extract] or Melinoil [INCI: Isopropyl Palmitate, Lecithin, Aqua, Acetyl Hexapeptide-1] marketed by Atrium Innovations/Lucas Meyer Cosmetics; Repair Complex [INCI: Bifida Ferment Lysate] marketed by CLR; Phycojuvenine [INCI: Laminaria Digitata] marketed by Codif; Unirepair T-43 [INCI: Butylene Glycol, Acetyl Tyrosine, Proline, Hydrolyzed Vegetable Protein, Adenosine Triphosphate] marketed by Induchem; Dragosine [INCI: Carnosine] marketed by Symrise; DN-Age [INCI: Cassia Alata Leaf Extract] marketed by Laboratories Serobiologiques/Cognis/BASF; Helioguard [INCI: Porphyra Umbilicalis encapsulated into liposomes], PhytoCellTec Malus Domestica [INCI: PhytoCellTec Malus Domestica] or PhytoCellTec Argan [INCI: Argania Spinosa Sprout Cell Extraxt, Isomalt, Lecithin, Sodium Benzoate, Aqua] marketed by Mibelle Biochemistry; Pepha-Protect [INCI: Water Melon Extract] marketed by Pentapharm/DSM; Celligent [INCI: Helianthus Annuus Seed Oil, Ethyl Ferulate, Polyglyceryl-5 Trioleate, Rosmarinus Officinalis Leaf Extract, Aqua, Disodium Uridine Phosphate] or Defensil [INCI: Octyl Dodecanol, Echium Plantagineum Seed Oil, Cardiospermum Halicacabum Extract, Helianthus Annuus Seed Oil Unsaponifiables] marketed by Rahn; Venuceane [INCI: Thermus Thermophilus Ferment, Glycerin], UV-Soft [INCI: Yeast Extract], Renovage [INCI: Caprylic/Capric Triglyceride, Teprenone], Juvinity [INCI: Caprylic/Capric Triglyceride, Geranylgeranylpropanol (proposed)], Phytessence Holyherb [INCI: Butylene Glycol, Eriodictyon Californicum (Holyherb) Flower/Leaf/Stem Extract] or Resistem [INCI: Glycerin, Globularia Cordifolia Ferment] marketed by Sederma/Croda; Infraguard [INCI: Caesalpinia Spinosa Fruit Pod Extract, Propylene Glycol, Aqua, Helianthus Annuus Sprout Extract, Sodium Benzoate, Phenoxyethanol] marketed by Mibelle; Heliomoduline [INCI: Low molecular weight peptides from cottonseed] or Stem-C-Guard [Hydrolyzed Pea] marketed by Silab; and combinations thereof..

In another embodiment, the reactive carbonyl species scavenger, free radical scavengers and/or anti-glycation agent, detoxifying agent, antioxidant and/or anti-pollution agent is selected, for example and not restricted to, from the group formed by carnosine and its derivatives; GHK [INCI: Tripeptide-1] and its salts and/or derivatives or Quintescine IS [INCI: Dipeptide-4] marketed by Vincience/ISP/Ashland; Preregen [INCI: Glycine Soja (Soybean) Protein, Oxido Reductases], Edelweiss GC [INCI: Leontopodium Alpinum Extract], Lipogard [INCI: Squalane, Ubiquinone], Nectapure [INCI: Buddleja Davidii Extract, Thymus Vulgaris Extract], Alpaflor Nectapure [INCI: Buddleja Davidii Extract, Thymus Vulgaris Extract, Glycerin, Water] or Dismutin-BT [INCI: Highly purified SOD from a natural yeast strain of Saccharomyces cerevisiae] marketed by Pentapharm/DSM; Preventhelia^{®} [INCI: Diaminopropionoyl Tripeptide-33], Aldenine^{®} [INCI: Hydrolized Wheat Protein, Hydrolized Soy Protein, Tripeptide 1], Lipochroman^{™} [INCI: Dimethylmethoxy Chromanol], Thermostressine^{®} [INCI: Acetyl Tetrapeptide-22] Pollushield^{™} functional ingredient [INCI: Diisopropyl Adipate, Lecithin, Acrylic Acid/Acrylamidomethyl Propane Sulfonic Acid Copolymer, Dimethylmethoxy Chromanol, Xanthan Gum] or Bodyfensine^{®} [INCI: Acetyl Dipeptide-3 Aminohexanoate] marketed by Lipotec/Lubrizol; unactyl [INCI: Mannitol, Pisum Sativum Extract, Histidine HCl, Arginine, Cyclodextrin, Dextrin, Yeast Extract, Acetyl Trysoine, Pyridoxine HCl, Khaya Senegalensis Bark Extract, Nicotinamide, Adenine Dinucleotide, Disodium Succinate, Aspartic Acid], Imidinyl [INCI: Tamarindus Indica Seed Polysaccharide], Phystrogene [INCI: Butylene Glycol, Malva Sylvestris (Mallow) Extract, Xanthan Gum] or Purisoft [INCI: Moringa Pterogysperma Seed Extract] marketed by Laboratoires Sérobiologiques/Cognis/BASF; AquaCacteen [INCI: Glycerin, Opuntia Ficus Indica Stem Extract, Phenoxyethanol, Aqua], Trimoist (KMF) [INCI: Sodium Stearoyl Lactylate, Cetyl alcohol, Olus Vegetable oil, Tocopheryl acetate, Glycerin, Glycine soja sterol, Sodium lactate, Sodium barboxymethyl betaglucan, Carnosine, Lactic Acid], MelanoBronze [INCI: Vitex Agnus Castus Extract (Monk's pepper berries extract (phyto-endorphins)), Acetyl Tyrosine], CM-Glucan [INCI: Sodium Carobxymethyl Betaglucan, Phenoxyethanol, SunActin [INCI: Helianthus Annuus (Sunflower) Sprout Extract, Tocopherols, Glycerin, Lecithin, Phenoxyethanol, Aqua], GSP-T skin [INCI: Glycerin, Alcohol, Aqua, PEG-40 Hydrogenated Castor Oil, Vitis Vinifera (Grape) Seed Extract] or Detoxophane [INCI: Lepidium Sativum Sprout Extract, Lecithin, Phenoxyethanol, Glycerin, Water] marketed by Mibelle Biochemistry; Bacocalmine [INCI: PEG-8, Bacopa Monniera Extract, Water (Aqua), Hydroxyethylcellulose], Kombuchka [INCI: Saccharomyces/Xylinum Black Tea Ferment, Glycerin, Hydroxyethyl cellulose], Citystem [INCI: Glycerin, Marrubium Vulgare Extract] or Prodizia [INCI: Albizia Julibrissin Extract, Glycerin] marketed by Sederma/Croda; Extramel C [INCI: Hydroxypropyltrimonium Maltodextrin Crosspolymer, Cucumis Melo (Melon) Fruit Extract] marketed by Seppic; Defensine [INCI: Triticum Vulgare Germ Extract], Apolluskin^{®} [INCI: Taraxacum officinale (Dandelion) Extract], Detoxyl^{®} [INCI: Water, Butylene Glycol, Butyrospermum parkii (Shea Butter) Seedcake Extract] or Antiglyskin [INCI: Aqua, Helianthus Annuus Seed Extract] marketed by Silab; and combinations thereof.

The compositions of the invention may be for use in any of the applications or uses discussed above under the heading "Applications".

In another aspect, the invention provides kit for use in a cosmetic, non-therapeutic method of treatment and/or care of the skin comprising:
(i) a composition comprising Botulinum toxin;
(ii) optionally, a composition comprising Ac-Glu-Glu-Met-Gln-Arg-Arg-NH₂, or H-Tyr-D-Ala-Gly-Phe-Leu-OH or combinations thereof; and
(iii) a cosmetic composition comprising a compound of formula (I).

The (i) composition comprising Botulinum toxin, (ii) composition comprising Ac-Glu-Glu-Met-Gln-Arg-Arg-NH₂, or H-Tyr-D-Ala-Gly-Phe-Leu-OH or combinations thereof, if present, and the (iii) composition comprising the compound of the invention according to the first aspect, can be in the same or separate containers. In one embodiment, the kit can further comprise means to apply the compositions to the skin. For instance, the kit can comprise means such as syringes or microneedles.

The invention is illustrated by the following non-limiting examples.

### EXAMPLES

### General methods

### Abbreviations

The abbreviations used for amino acids follow the 1983 IUPAC-IUB Joint Commission on Biochemical Nomenclature recommendations outlined in Eur. J. Biochem. (1984) 138:9-37*.*

(R), resin; 2-CITrt-(R), 2-chlorotrityl resin; Ac, acetyl; AcOH, acetic acid; Ala, alanine; AM, 2-[4-aminomethyl-(2,4-dimethoxyphenyl)] phenoxyacetic acid; Arg, arginine; Asn, asparagine; Asp, aspartic acid; Boc, *tert*-butyloxycarbonyl; DCM, dichloromethane; DIEA, *N*,*N*'-diisopropylethylamine; DIPCDI, *N*,*N*'-diisopropylcarbodiimide; DMF, *N*,*N-*dimethylformamide; ESI-MS, electrospray ionization mass spectrometry; Fmoc, 9-flluorenylmethyloxycarbonyl; Gln, glutamine; Glu, glutamic acid; Gly, Glycine; His, histidine; HOBt, 1-hydroxybenzotriazole; HPLC, high performance liquid chromatography; Ile, isoleucine; KOH, potassium hydroxide; Leu, leucine; Lys, lysine; MBHA, *p*-methylbenzhydrylamine; MeCN, acetonitrile; MeOH, methanol; Met, Methionine; Myr, myristoyl; Palm, palmitoyl; Pbf, 2,2,4,6,7-pentamethyldihydrobenzofuran-5-sulfonyl; Pro, proline; Ser, serine; tBu, *tert-butyl;* TFA, trifluoroacetic acid; Thr threonine; Trt, trithyl; Val, valine.

### Chemical Synthesis

All synthetic processes are carried out in polypropylene syringes fitted with porous polyethylene discs. The coupling protocol is carried out following the standard protocols founded in bibliography and the solvents and soluble reagents are removed by suction. The Fmoc group is removed with piperidine-DMF (2:8, v/v) (1 x 1 min, 1 x 5 min, 5 ml/g resin) *[*Lloyd-Williams P. et al. (1997) "Chemical Approaches to the Synthesis of Peptides and Proteins" CRC, Boca Raton (FL, USA*)].* Washes between stages of deprotection, coupling and, again, deprotection, are carried out with DMF (3 x 1 min) each time using 10 ml solvent/g resin. Coupling reactions are performed with 3 ml solvent/g resin. The control of the couplings is performed by carrying out the ninhydrin test *[*Kaiser E. et al., Anal. Biochem. (1970), 34: 595-598*]* or chloranil test *[*Christensen T., Acta Chem. Scand., (1979), 33B, 763-766*]*. The coupling reactions are repeated as the desired peptide is synthesized. All synthetic reactions and washes are carried out at 25°C.

It is known by the skilled person that some amino acids are used with their functional groups in side chains protected. For instance, non-limiting examples of protecting groups are:
- tBu, *tert*-butyl for amino acid Glu; and
- Pbf, 2,2,4,6,7-pentamethyldihydrobenzofuran-5-sulfonyl for aminoacid Arg; and
- Trt, trithyl for amino acids Gln.

HPLC chromatographic analysis is carried out with Shimadzu equipment (Kyoto, Japan) using a reversed-phase column thermostatized at 30°C (50 x 4.6 mm, Kromasil C18, 3.5 µm, Akzo Nobel, Sweden). The elution is carried out using a gradient of acetonitrile (+0.07% TFA) in water (+0.1% TFA) at a flow rate of 1.6 mL/min and detection is carried out at 220 nm. The electrospray ionization mass spectrometry is carried out in a WATERS Alliance ZQ 2000 detector using a mixture of MeCN:H₂O 4:1 (+0.1% TFA) as the mobile phase and a flow rate of 0.3 ml/min.

### EXAMPLE 1

*Obtaining Fmoc-Wₘ-Xₙ-AA₁-AA₂-AA₃-AA₄-AA₅-AA₆₋-Yₚ-Z_{q}-AM-MBHA-(R), wherein AA₁ is L-Arg; AA₂ is L-Arg; AA₃ is L-Gln or D-Gln; AA₄ is L-Met or D-Met; AA₅ is L-Glu; AA₆ is L-Glu; and n, m, p and q are each 0 or 1.*

Weights have been normalized. Fmoc-AM-pMBHA resin is treated with piperidine:DMF according to the described general protocol in order to remove the Fmoc group. 5 equiv of Fmoc-L-Glu(tBu)-OH (Fmoc-AA₆-OH) is incorporated onto the deprotected resin in the presence of 5.5 equiv of DIPCDI and 5 equiv of HOBt using DMF as a solvent for 1 hour.

The resin is then washed as described in the general methods and the deprotection treatment of the Fmoc group is repeated to couple the next amino acid: 5 equiv of Fmoc-Glu(tBu)-OH (Fmoc-AA₅-OH); and subsequently 5 equiv of Fmoc-D-Met-OH (Fmoc-AA₄-OH); 5 equiv of Fmoc-L-Gln(Trt)-OH (Fmoc-AA₃-OH); 5 equiv of Fmoc-L-Arg(Pbf)-OH (Fmoc-AA₂-OH); and finally 5 equiv of Fmoc-L-Arg(Pbf)-OH (Fmoc-AA₁-OH) are sequentially coupled in the presence of 5 equiv of HOBt and 5.5 equiv of DIPCDI in each coupling step.

After the synthesis, the peptidyl resin is washed with DCM (3 x 1 min).

Non-limiting examples of peptides synthesized by this method are showed in Table 4:

**Table 4**

| **Peptides** |
|---|
| Fmoc-Arg-Arg-Dgln-Met-Glu-Glu-AM-M BHA-(R) |
| Fmoc-Arg-Arg-Gln-Dmet-Glu-Glu-AM-MBHA-(R) |
| Fmoc-Arg-Arg-Gln-Met-Glu-Glu-AM-MBHA-(R) |
| Fmoc-Ala-Arg-Arg-Gln-Dmet-Glu-Glu-AM-MBHA-(R) |
| Fmoc-Arg-Arg-Gln-Dmet-Glu-Glu-Ala-AM-MBHA-(R) |

By following the described method is possible to obtain different sequences changing the desired amino acids to be coupled.

### EXAMPLE 2

### General process for removal of Fmoc N-terminal protective group.

The N-terminal Fmoc groups of the peptidyl resins obtained in Example 1 are deprotected as described in the general methods (20% piperidine in DMF, 1 x 1 min + 1 x 5 min). The peptidyl resins are washed with DMF (5 x 1 min), DCM (3 x 1 min), diethyl ether (3 x 1 min) and dried under vacuum.

### EXAMPLE 3

### Process for introducing the R₁ palmitoyl group onto the peptidyl resins obtained in Example 1.

5 equiv of palmitic acid pre-dissolved in DMF (1 ml) is added respectively of each of the peptidyl resins obtained in Example 2, in the presence of HOBt and DIPCDI. The mixture is allowed to react for 3 hours, after which the resin is washed with DMF (3 x 1 min), DCM (3 x 1 min), diethyl ether (3 x 1 min) and is dried under vacuum.

### EXAMPLE 4

### Process for introducing the R₁ acetyl group onto the peptidyl resins obtained in Example 1.

Each of the peptidyl resins obtained in Example 2 is treated with acetic anhydride in the presence of DIEA using DMF as a solvent. The mixture is allowed to react for 30 min, after which the resin is washed with DMF (3 x 1 min), DCM (3 x 1 min), diethyl ether (3 x 1 min) and is dried under vacuum.

### EXAMPLE 5

### Cleavage process from the polymeric support of the peptidyl resins obtained in Examples 2, 3 and 4.

Each of the dried peptidyl resins obtained in Examples 2, 3 and 4 are treated with 3 ml of TFA:H₂O (95:5, v/v) for 2 hours at room temperature under stirring. Then it is filtered through a polypropylene syringe fitted with porous polyethylene discs. The filtrate is collected onto cold diethyl ether, and washed 5 times with diethyl ether. The final precipitate is dried under vacuum.

Peptides of general formula R₁-Wₘ-Xₙ-AA₁-AA₂-AA₃₋AA₄-AA₅-AA₆-Yₚ-Z_{q}-OH or R₁-Wₘ-Xₙ-AA₁-AA₂-AA₃₋AA₄-AA₅-AA₆-Yₚ-Z_{q}-NH₂, wherein R₁ is H, acetyl or palmitoyl are obtained following this method.

HPLC analyses of the obtained peptides in gradients of MeCN (+0.07% TFA) in H₂O (+0.1% TFA) show a purity exceeding 80% in all cases. The identity of the peptides obtained is confirmed by ESI-MS.

### EXAMPLE 6

### Inhibition of noradrenaline release from human neuroblastoma cells.

Release of neurotransmitters by exocytosis in neuromuscular junctions from peripheral neurons to skeletal muscle allows muscle contraction. Face muscles are subject to said contraction and facial muscle contraction is more frequent around eyes, mouth and forehead than elsewhere in the face. With age, continuous release of neurotransmitters into neuromuscular junctions and decrease of elasticity contribute to the increase of facial wrinkles and permanent expression lines. Compounds able to block or decrease exocytosis on neuromuscular junction sites are thus good candidates for the cosmetic treatment of un-aesthetic wrinkles. *In vitro* induction of the neurotransmitter Noradrenaline (NA) release in a human neuroblastoma cell line (SH-SY5Y) is considered a direct and reliable method for exocytosis measurement. The aim of this study is to evaluate the efficacy of the peptides of the invention on the inhibition of neuronal exocytosis by means of measuring the levels of NA release in SH-SY5Y cells by Enzyme-linked Immunosorbent Assay (ELISA).

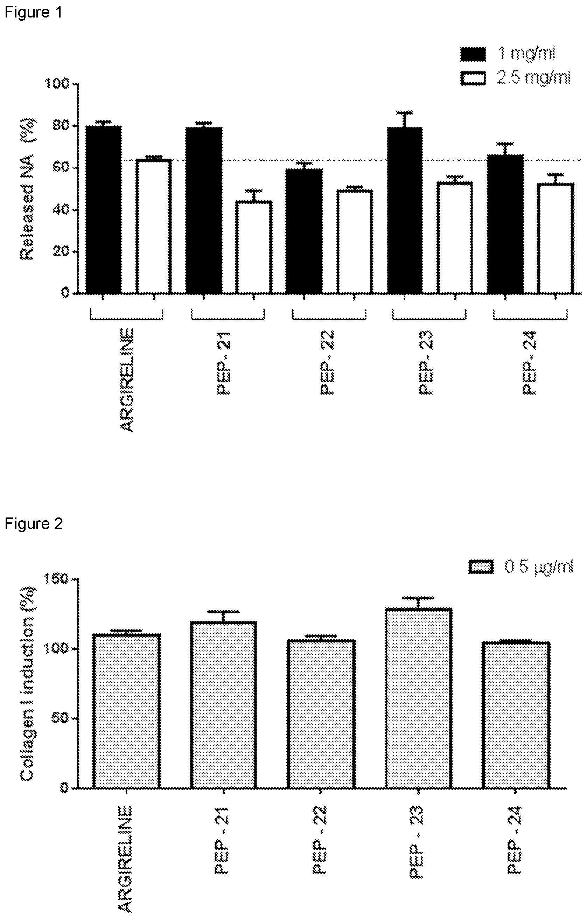

SH-SY5Y (ECACC) cells are seeded in 12-well plates at a density of 1x10⁶ cells/well. After a 6-day culture, medium is removed from wells and cells are treated for 60 minutes with the peptides of the invention dissolved in Hank's Balanced Salt Solution (HBSS, Life Technologies) at 1 and 2.5 mg/ml. Cells treated with HBSS alone are used as a basal control. To mobilize NA vesicles, supernatants are removed and 100 nM Tetradecanoylphorbol-13-acetate (TPA, Sigma) is dissolved in the presence of test items in HBSS or dissolved in HBSS alone in the case of the basal control. After this, solutions with TPA are removed and NA release is induced by the addition of 10 µM lonomycin (Epica) with 100 nM TPA dissolved in the presence of test items in HBSS or dissolved in HBSS alone in the case of the basal control. Afterwards, the medium in the wells, containing released NA, is collected and centrifuged. Supernatants obtained are used for quantification of NA by ELISA with noradrenalin ELISA kit (IBL International) following manufacturer's instructions. In brief, direct sandwich ELISA is performed with a plate pre-coated with an anti-NA antibody. The color obtained after substrate addition is directly proportional to the amount of NA present in each condition tested. Absorbance is read in a microplate absorbance reader (Clariostar, BMG) at 405 nm. The percentage of NA release for each condition is calculated respect to basal control. Results of percentage of NA release versus non-treated cells (basal control) are shown in FIG. 1 and are also provided in the table below.

**Table 4a**

| | | % Released NA |
|---|---|---|
| ARGIRELINE | 2.5 mg/ml | 63.5 |
| | 1 mg/ml | 79.6 |
| PEP-21 | 2.5 mg/ml | 43.8 |
| | 1 mg/ml | 78.9 |
| PEP-22 | 2.5 mg/ml | 49.0 |
| | 1 mg/ml | 58.9 |
| PEP-23 | 2.5 mg/ml | 52.8 |
| | 1 mg/ml | 78.9 |
| PEP-24 | 2.5 mg/ml | 52.3 |
| | 1 mg/ml | 65.8 |

The results demonstrate that the peptides of the invention inhibit NA release respect to basal conditions in SH-SY5Y at tested concentration(s). They also demonstrate that the peptides of the invention are able to decrease NA release to values even lower than those achieved with Argireline^{®}.

An increase of exocytosis release in neuromuscular junction is related with aging and with an increase of facial wrinkles and expression lines. The peptides of the invention reduce exocytosis levels as they reduce NA release in SH-SY5Y. Furthermore, the peptides of the invention decrease said release even more than Argireline^{®}.

### EXAMPLE 7

### In vitro study of type I collagen synthesis on human dermal fibroblasts by Enzyme-Linked Immunosorbent Assay.

Collagen type I is the principal collagen of skin. This molecule, which is mainly produced by fibroblasts, is responsible for the strength and resiliency of this tissue. For this reason, in vitro quantification of collagen induction by cosmetic products on human dermal fibroblasts provides information about their potential anti-aging effects on the skin. Collagen induction by the peptides of the invention is evaluated with an Enzyme-Linked Immunosorbent Assay (ELISA). The aim of this study is to investigate the ability of a product to induce collagen type I synthesis in primary human dermal fibroblasts isolated from neonatal skin (HDFn).

HDFn (Cascade) are seeded at a density of 5x10⁴ in 48-well plates. After 24-hour incubation, fresh medium with scalar dilutions of the test items a 0.5 µg/ml are added. Non-treated cells are used as basal control. The cells are treated for 48 hours. Then, the medium of the wells is collected and transferred into a 96-well plate. A standard calibration curve prepared with calf skin type I collagen (Sigma) is transferred into the plate as well. The plate is left overnight in order to coat type I collagen into its surface. After that, collagen I is detected with an anti-collagen type I antibody (Sigma). The primary antibody is then recognized by a secondary antibody IgG-HRP (molecular probes). 3,3,5,5-tetramethylbenzidine liquid substrate (TMB, Sigma) is added to measure the amount of secondary antibody attached. The colour produced by this reaction is measured in a microtiter plate reader (Clariostar, BMG) and type I collagen concentration is determined using a linear regression of the standard curve. Results of collagen synthesis versus non-treated cells are shown in FIG. 2 and are also provided in the table below.

**Table 4b**

| | % Collagen induction |
|---|---|
| ARGIRELINE | 109.8 |
| PEP-21 | 119.3 |
| PEP-22 | 106.0 |
| PEP-23 | 114.6 |
| PEP-24 | 104.7 |

The results demonstrate that the peptides of the invention boost type I collagen production respect to basal conditions in HDFn at tested concentration. They also demonstrate that the peptides of the invention have similar or increased collagen type I production with respect to Argireline^{®}.

During the process of skin aging type I collagen is decreased. The peptides of the invention increase collagen type I produced by HDFn. Particularly, the efficacy of peptides PEP-21 and PEP-23 in increasing collagen type I is even higher than that found for the Argireline^{®} peptide.

### EXAMPLE 8

### In vitro quantification of Musclebind like protein 1 in human skeletal muscle cells by Time resolved fluorescence resonance energy transfer.

Aging is linked with a muscle mass loss due to the activation of muscle atrophy-related events. Among the processes related with muscle atrophy, loss of function of Musclebind like protein 1 (MBNL1) is associated with muscle mass loss involved in the ageing process of facial muscles. For this reason, compounds able to enhance MBNL1 can be used as a good approach for cosmetic treatment of sagging facial appearance due to muscle mass loss.

MBNL1 induction by the peptides of the invention is evaluated by a Time resolved fluorescence resonance energy transfer (TR-FRET) assay. The aim of this study is to investigate the capacity of peptide candidates to increase MBNL-1 in human skeletal muscle cells (hSkMc).

hSkMc (Innoprot) are seeded in 12-well plates at a density of 1,5x10⁴ cells/well in Skeletal Muscle Cell Growth Medium (Promocell). After 72 hours incubation, medium is removed and replaced with Skeletal Muscle Cell Differentiation Medium (Promocell). 48 hours after beginning cell differentiation, fresh differentiation medium with 0.5 mg/ml of test items is added. Treatment is continued for 48 hours and non-treated cells are used as basal control. After 48 hours of treatment, cell medium is removed and added cell lysis buffer to wells. Immediately, plates are kept at -80°C in order to improve protein extraction during the defrosting process. After 72 hours, cells are lysated by shaking the plates in an orbital rotor at room temperature for 45 minutes. Next, cell lysates are collected and assayed to quantify the level of MBNL-1 protein and total protein concentration.

MBNL-1 protein levels measurement is performed with Human MBNL1 Assay Kit (Cisbio) according to the manufacturer's protocol. Briefly, the kit is used to perform a TR-FRET for MBNL1 quantification. The protein is detected with the antibodies provided in the kit and quantified by a fluorescence measurement. The quantification is carried out by using a microplate reader (ClarioStar, BMG) set to 665nm and 620nm.

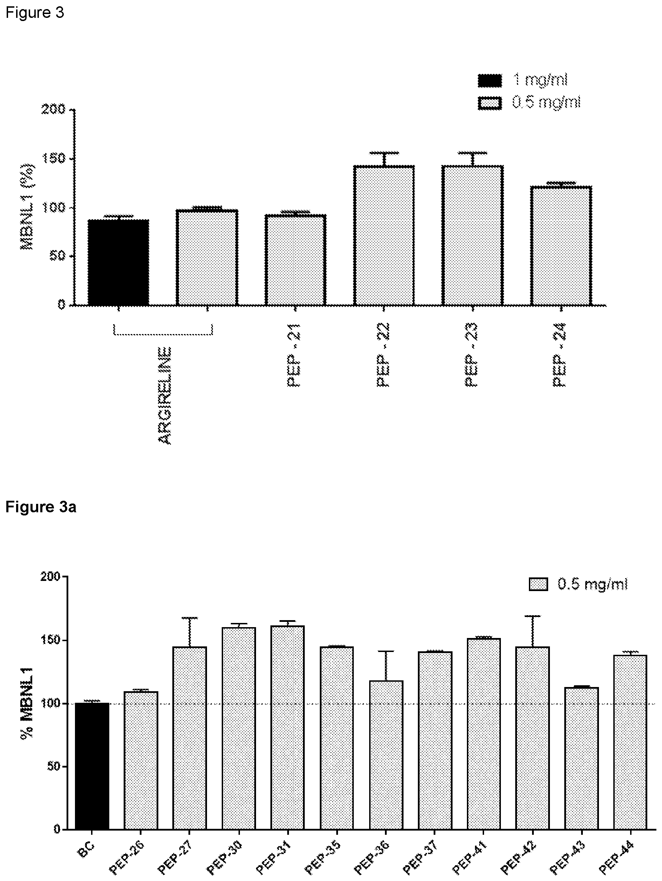

Total protein concentration of cell lysate is determined by using Pierce BCA Protein Assay Kit (Thermo Scientific) according to manufacturer's protocol. In brief, after adding Working Reagent to the samples and the standards, the samples are incubated. Afterwards, color change is measured with an absorbance microplate reader (Clariostar, BMG) at 562 nm. The total protein amount is used to normalize the level of MBNL1 protein concentration obtained by the TR-FRET test in the samples. Results of % MBNL1 protein induced versus a basal control are shown in FIG. 3 and FIG. 3a and are also provided in the table below.

**Table 4c**

| | | | % MBNL1 vs basal level |
|---|---|---|---|
| ARGIRELINE | | 1 mg/ml | 86.9 |
| | | 0.5 mg/ml | 96.6 |
| PEP-21 | | 0.5 mg/ml | 91.9 |
| PEP-22 | Ac-L-Arg-L-Arg-D-Gln-L-Met-L-Glu-L-Glu | 0.5 mg/ml | 141.4 |
| PEP-23 | Ac-L-Arg-L-Arg-L-Gln-D-Met-L-Glu-L-Glu-NH₂ | 0.5 mg/ml | 142.3 |
| PEP-24 | Ac-L-Arg-L-Arg-D-Gln-D-Met-L-Glu-L-Glu-NH₂ | 0.5 mg/ml | 120.9 |
| PEP-26 | Ac-L-Arg-L-Arg-L-Gln-D-Met-L-Glu-L-Glu-OH | 0.5 mg/ml | 109.6 |
| PEP-27 | H-L-Arg-L-Arg-L-Gln-D-Met-L-Glu-L-Glu-NH₂ | 0.5 mg/ml | 144.2 |
| PEP-41 | Ac-L-Arg-L-Arg-L-Asp-D-Met-L-Glu-L-Glu-NH₂ | 0.5 mg/ml | 151.3 |
| PEP-30 | Ac-L-Arg-L-Arg-L-Gln-D-Met-L-Asp-L-Glu-NH₂ | 0.5 mg/ml | 160.0 |
| PEP-31 | Ac-L-Arg-L-Arg-L-Gln-D-Met-L-Glu-L-Gln-NH₂ | 0.5 mg/ml | 161.3 |
| PEP-35 | Ac-L-Arg-L-Lys-L-Gln-D-Met-L-Glu-L-Glu-NH₂ | 0.5 mg/ml | 144.3 |
| PEP-36 | Ac-L-Lys-L-Arg-L-Gln-D-Met-L-Glu-L-Glu-NH₂ | 0.5 mg/ml | 117.6 |
| PEP-42 | Ac-L-Ala-L-Arg-L-Arg-Gln-D-Met-L-Glu-L-Glu-L-Ala-NH₂ | 0.5 mg/ml | 144.4 |
| PEP-43 | Ac-L-Arg-L-Gln-D-Met-L-Glu-L-Glu-NH₂ | 0.5 mg/ml | 112.4 |
| PEP-44 | Ac-L-Arg-L-Arg-L-Gln-D-Met-L-Glu-NH₂ | 0.5 mg/ml | 137.8 |
| PEP-37 | Ac-L-Arg-L-Arg-L-Gln-D-Leu-L-Glu-L-Glu-NH₂ | 0.5 mg/ml | 140.3 |

The results demonstrate that peptides PEP-22, PEP-23, PEP-24, PEP-26, PEP-27, PEP-41, PEP-30, PEP-31, PEP-35, PEP-36, PEP-42, PEP-43, PEP-44 and PEP-37 of the invention increase MBNL1 levels when compared to the basal control and to Argireline^{®}. In fact, the results show that Argireline^{®} has no effect on MBNL1 even at a concentration of twice (1 mg/mL) that of the peptides of the invention (0-5 mg/mL). It is believed that the enhancement of MBNL1 protein slows the muscle mass loss during aging, thus avoiding the appearance of sagging face skin in older people.

### EXAMPLE 9

### Preparation of a cream containing peptide PEP-21 Ac-L-Arg-L-Arg-L-Gln-L-Glu-L-Glu-NH₂ peptide solution

In a suitable vessel, the ingredients of phase A: water [INCI: WATER (AQUA)], Zemea^{™} [INCI: PROPANEDIOL], Hydrolite^{®} 5 [INCI: PENTYLENE GLYCOL], Phenoxetol^{™} [INCI: PHENOXYETHANOL] and Dissolvine^{®} NA2 [INCI: DISODIUM EDTA] are dissolved.

Phase A1 ingredient:Carbopol^{®} Ultrez 10 Polymer [INCI: CARBOMER] is added to the previous mixture. Once dispersed, phase A2: Cola^{®}Fax CPE-K [INCI: POTASSIUM CETYL PHOSPHATE] is introduced. Then the mixture is heated up to 70-75°C.

In a separate vessel, phase B ingredients: Schercemol^{™} DIS Ester [INCI: DIISOPROPYL SEBACATE], Phytocream^{®} 2000 [INCI: GLYCERYL STEARATE, CETEARYL ALCOHOL, POTASSIUM PALMITOYL HYDROLYZED WHEAT PROTEIN], Massocare^{®} EC [INCI: ETHYLHEXYL COCOATE], Astro-sil 2C 350 [INCI: DIMETHICONE] and Tocopheryl Acetate [INCI: TOCOPHERYL ACETATE] are mixed and the resulting mixture is heated at 70-75°C.

The emulsion is made by adding slowly phase B into phase A under conditions of fast stirring with a turbine.

Once the mixture is cooled to 40°C, the components of phase C: Novemer^{™} EC-1 polymer [INCI: MINERAL OIL (PARAFFINUM LIQUIDUM); WATER (AQUA); ACRYLATES/ACRYLAMIDE CROSSPOLYMER; POLYSORBATE 85] are added to the previous mixture under stirring and mixed until dispersion.

Phase D: Peptide PEP-21 solution [INCI: GLYCERIN; WATER (AQUA); peptide PEP-21) is added to the previous mixture.

Phase E: Fragrance [INCI: FRAGANCE (PARFUM)], is added.
pH is adjusted to 6.0 - 6.5 with phase F ingredient sodium hydroxide 20% w/w [INCI: WATER (AQUA); SODIUM HYDROXIDE])

**Table 5**

| Phase | INGREDIENT (INCI name) | % weight |
|---|---|---|
| A | WATER | 69.10 |
| A | PROPANEDIOL | 10.00 |
| A | PENTYLENE GLYCOL | 2.00 |
| A | PHENOXYETHANOL | 0.50 |
| A | DISODIUM EDTA | 0.20 |
| A1 | CARBOMER | 0.50 |
| A2 | POTASSIUM CETYL PHOSPHATE | 0.50 |
| B | DIISOPROPYL SEBACATE [GLYCERYL STEARATE; CETEARYL ALCOHOL; | 5.00 |
| B | POTASSIUM PALMITOYL HYDROLYZED WHEAT PROTEIN] | 5.00 |
| B | ETHYLHEXYL COCOATE | 2.50 |
| B | DIMETHICONE | 1.00 |
| B | TOCOPHERYL ACETATE [MINERAL OIL (PARAFFINUM LIQUIDUM); | 0.50 |
| C | WATER (AQUA); ACRYLATES/ACRYLAMIDE CROSSPOLYMER; POLYSORBATE 85] | 1.00 |
| D | [GLYCERIN; WATER (AQUA); PEP-21] | 2.00 |
| E | FRAGANCE (PARFUM) | 0.20 |
| F | [WATER (AQUA); SODIUM HYDROXIDE] | q.s. |

### EXAMPLE 10

### Preparation of a Gel-cream comprising peptide PEP-23 (Ac-L-Arg-L-Arg-L-Gln-D-Met-L-Glu-L-Glu).

In a suitable vessel, the ingredients of phase A: water [INCI: WATER (AQUA)], Zemea^{™} [INCI: PROPANEDIOL], Phenoxetol^{®} [INCI: PHENOXYETHANOL], Dissolvine^{®} NA2 [INCI: DISODIUM EDTA] and Potassium Sorbate Granular [POTASSIUM SORBATE] are dispersed.

Phase A1 ingredient: Carbopol^{®} Ultrez 21 Polymer [INCI: ACRYLATES/ C10/30 ALKYL ACRYLATE CROSSPOLYMER] is added to the previous mixture under stirring. Once dispersed, phase A2: Xanthan Gum [INCI: XANTHAN GUM] is introduced to the previous mixture and stirred until complete dispersion.

In a separate vessel, phase B ingredients: Schercemol^{™} 1818 Ester [INCI: ISOSTEARYL ISOSTEARATE], is weighed.

The emulsion is made by adding slowly phase B into phase A under conditions of fast stirring with a turbine.

Phase C: Peptide PEP-23 solution [INCI: WATER (AQUA); CAPRYLYL GLYCOL; Peptide PEP-23] is added to the previous mixture.
pH is adjusted to 6.0 - 6.5 with phase D ingredient: Sodium Hydroxide 20% w/w [INCI: WATER (AQUA); SODIUM HYDROXIDE])

**Table 6**

| Phase | INGREDIENT (INCI name) | % weight |
|---|---|---|
| A | WATER | 83.54 |
| A | PROPANEDIOL | 10.00 |
| A | PHENOXYETHANOL | 0.35 |
| A | DISODIUM EDTA | 0.20 |
| A | POTASSIUM SORBATE | 0.10 |
| A1 | ACRYLATES/ C10/30 ALKYL ACRYLATE CROSSPOLYMER | 0.65 |
| A2 | XANTHAN GUM | 0.20 |
| B | ISOSTEARYLISOSTEARATE | 2.00 |
| C | [WATER (AQUA); CAPRYLYL GLYCOL; PEPTIDE PEP-23] | 2.00 |
| D | [WATER (AQUA); SODIUM HYDROXIDE] | 0.96 |

### EXAMPLE 11

### Preparation of a gel comprising 2% of peptide PEP-23 (Ac-L-Arg-L-Arg-L-Gln-D-Met-L-Glu-L-Glu-NH₂) solution

In a suitable vessel, the ingredients of phase A: water [INCI: WATER (AQUA)], Zemea^{™} [INCI: PROPANEDIOL], Glucam^{™}E-20 Humectant [INCI: METHYL GLUCETH-20], Dissolvine^{®} NA2 [INCI: DISODIUM EDTA], Phenoxetol^{®} [INCI: PHENOXYETHANOL] are dissolved.

Phase A1: Carbopol^{®} ultrez 10 polymer [INCI: CARBOMER] is added to the previous mixture and mixed until complete dispersion.

Phase B: Peptide PEP-23 solution [INCI: WATER (AQUA); CAPRYLYL GLYCOL; PEP-23] is added to previous mixture and mixed.

Phase C: EUMULGIN^{®} CO 40 [INCI: PEG-40 HYDROGENATED CASTOR OIL], Fragrance [INCI: FRAGRANCE (PARFUM)], is added to previous mixture and mixed.

The pH is adjusted to 6.0-6.5 with the ingredient of phase D: Sodium Hydroxide 20% w/w [INCI: WATER (AQUA); SODIUM HYDROXIDE].

**Table 7**

| Phase | INGREDIENT (INCI name) | % weight |
|---|---|---|
| A | WATER | 80.8 |
| A | PROPANEDIOL | 10 |
| A | METHYL GLUCETH-20 | 5 |
| A | PHENOXYETHANOL | 0.5 |
| A | DISODIUM EDTA | 0.2 |
| A1 | CARBOMER | 0.7 |
| B | [WATER (AQUA); CAPRYLYL GLYCOL; Peptide PEP-23] | 2 |
| C | PEG-40 HYDROGENATED CASTOR OIL | 0.6 |
| C | FRAGRANCE (PARFUM) | 0.2 |
| D | [WATER (AQUA); SODIUM HYDROXIDE] | q.s. |

### EXAMPLE 12

### Preparation of a lotion comprising 2% of peptide PEP-23 (Ac-L-Arg-L-Arg-L-Gln-D-Met-L-Glu-L-Glu-NH₂) solution

In a suitable vessel, the ingredients of phase A1: water [INCI: WATER (AQUA)], Zemea^{™} [INCI: PROPANEDIOL], glycerin [INCI: GLYCERIN], potassium sorbate [INCI: POTASSIUM SORBATE] and Dissolvine^{®} NA2 [INCI: DISODIUM EDTA] are dissolved.

Phase A2 ingredient: Carbopol^{®} Ultrez 30 Polymer [INCI: CARBOMER] is added in the previous mixture. Once dispersed, phase A3: xanthan gum [INCI: XANTHAN GUM] is introduced. Then the mixture is heated at 70-75°C.

In a separate vessel, phase B ingredients: Fancor^{®} Meadowfoam seed oil [INCI: LIMNANTHES ALBA (MEADOWFOAM) SEED OIL], Kodasil 600 IDD Gel [INCI: ISODODECANE; VINYL DIMETHICONE/LAURYL DIMETHICONE CROSSPOLYMER; DIMETHICONE; LAURYL DIMETHICONE], Astro-sil 2C 350 [INCI: DIMETHICONE], Schercemol^{™} CATC ester [INCI: COCOYL ADIPIC ACID/TRIMETHYLOLPROPANE COPOLYMER; TRIMETHYLOLPROPANE], Schercemol^{™} DIS ester [INCI: DIISOPROPYL SEBACATE], Tocopheryl Acetate [INCI: TOCOPHERYL ACETATE] and Phenoxetol^{™} [INCI: PHENOXYETHANOL] are mixed and the resulting mixture is heated at 70-75°C.

The emulsion is made by adding slowly phase B into phase A under conditions of fast stirring with a turbine.

Once the mixture is cooled to 40°C, the components of phase C: Novemer^{™} EC-2 polymer [INCI: WATER (AQUA); SODIUM ACRYLATES/BEHENETH-25 METHACRYLATE CROSSPOLYMER; HYDROGENATED POLYDECENE, LAURYL GLUCOSIDE], SA-SB-300 (7%) [INCI: SILICA; DIMETHICONE], Fragrance [INCI: FRAGANCE (PARFUM)], and peptide PEP-23 solution [INCI: WATER (AQUA); CAPRYLYL GLYCOL; peptide PEP-23) are added to the previous mixture.
pH is adjusted to 6.0 - 6.5 with phase D ingredient sodium hydroxide 20% w/w [INCI: WATER (AQUA); SODIUM HYDROXIDE])

**Table 8**

| Phase | INGREDIENT (INCI name) | % weight |
|---|---|---|
| A1 | WATER | 63.60 |
| A1 | PROPANEDIOL | 10.00 |
| A1 | GLYCERIN | 5.00 |
| A1 | POTASSIUM SORBATE | 0.10 |
| A1 | DISODIUM EDTA | 0.20 |
| A2 | CARBOMER | 0.30 |
| A3 | XANTHAN GUM | 0.20 |
| B | LIMNANTHES ALBA (MEADOWFOAM) SEED OIL [ISODODECANE; VINYL DIMETHICONE/LAURYL | 5.00 |
| B | DIMETHICONE CROSSPOLYMER; DIMETHICONE; LAURYL DIMETHICONE] | 3.00 |
| B | DIMETHICONE [COCOYL ADIPIC ACID/TRIMETHYLOLPROPANE COPOLYMER; TRIMETHYLOLPROPANE] | 3.00 |
| B | | 2.00 |
| B | DIISOPROPYL SEBACATE | 2.00 |
| B | TOCOPHERYL ACETATE | 0.50 |
| B | PHENOXYETHANOL [WATER (AQUA); SODIUM | 0.50 |
| C | ACRYLATES/BEHENETH-25 METHACRYLATE CROSSPOLYMER; HYDROGENATED POLYDECENE, LAURYL GLUCOSIDE] | 1.50 |
| C | [SILICA; DIMETHICONE] | 1.00 |
| C | [WATER (AQUA); CAPRYLYL GLYCOL; Peptide PEP-23] | 2.00 |
| C | FRAGANCE (PARFUM) | 0.10 |
| D | [WATER (AQUA); SODIUM HYDROXIDE] | q.s. |

### EXAMPLE 13

### Preparation of a fluid emulsion comprising 2% of peptide PEP-23 (Ac-L-Arg-L-Arg-L-Gln-D-Met-L-Glu-L-Glu-NH₂) solution

In a suitable vessel, the ingredients of phase A1: water [INCI: WATER (AQUA)], Zemea^{™} [INCI: PROPANEDIOL], glycerin [INCI: GLYCERIN], Genencare^{™} OSMS BA [INCI: BETAINE], Dissolvine^{®} NA2 [INCI: DISODIUM EDTA], potassium sorbate [INCI: POTASSIUM SORBATE] are dissolved.

Phase A2: Carbopol^{®} ultrez 10 polymer [INCI: CARBOMER] is added to the previous mixture. Once dispersed, phase A3: Cola^{®}Fax CPE-K [INCI: POTASSIUM CETYL PHOSPHATE] is added. The resulting mixture is heated at 70-75°C.

In another vessel, the components of phase B: Massocare^{®} HD [INCI: ISOHEXADECANE], Lincol BAS [INCI: C12-15 ALKYL BENZOATE], Gandak C [INCI: CETYL ALCOHOL], Sorbital T 20 P [INCI: POLYSORBATE 20], 2-phenoxyethanol [INCI: PHENOXYETHANOL], Vegetable stearic acid 50/50 [INCI: STEARIC ACID; PALMITIC ACID] are mixed and heated at 70-75°C. Phase B is slowly introduced over phase A under conditions of intense stirring with a turbine.

The mixture is cooled at 40°C, and phase C: BRB CM 56-S [INCI: CYCLOMETHICONE], peptide PEP-23 solution [INCI: WATER (AQUA); CAPRYLYL GLYCOL; peptide PEP-23], Fragrance [INCI: FRAGRANCE (PARFUM)] is added. The pH is adjusted to 6.0-6.5 with the ingredient of phase D: Sodium Hydroxide 20% w/w [INCI: WATER (AQUA); SODIUM HYDROXIDE].

**Table 9**

| Phase | INGREDIENT (INCI name) | % weight |
|---|---|---|
| A1 | WATER | 71.2 |
| A1 | PROPANEDIOL | 10 |
| A1 | GLYCERIN | 3 |
| A1 | BETAINE | 3 |
| A1 | DISODIUM EDTA | 0.2 |
| A1 | POTASSIUM SORBATE | 0.1 |
| A2 | CARBOMER | 0.4 |
| A3 | POTASSIUM CETYL PHOSPHATE | 0.4 |
| B | ISOHEXADECANE | 2 |
| B | C12-15 ALKYL BENZOATE | 2 |
| B | CETYL ALCOHOL | 1.8 |
| B | POLYSORBATE 20 | 0.8 |
| B | PHENOXYETHANOL | 0.5 |
| B | [STEARIC ACID; PALMITIC ACID] | 0.5 |
| C | CYCLOMETHICONE | 2 |
| C | [WATER (AQUA); CAPRYLYL GLYCOL; PEP-23] | 2 |
| C | FRAGRANCE (PARFUM) | 0.1 |
| D | [WATER (AQUA); SODIUM HYDROXIDE] | q.s. |

### Example 14

### In vivo study for the assessment of antiwrinkle effect of the peptide of the invention after long-term application in Caucasian skin type female volunteers.

The study is carried out for 28 days. Thirthy (30) Caucasian female volunteers, aged between 35 and 50 years old showing skin wrinkledness on the face, are included. Subjects apply the composition described in Example 10 (Active Cream) on one side of the face (left or right) and a Placebo Cream having the same composition except the peptide of the invention. Active and Placebo Cream are applied for 28 days twice a day (morning and evening). The subjects serve as their own reference and results obtained at time 14 and 28 days are compared with those obtained at initial time. Moreover, results obtained with the Active Cream are compared with those obtained with Placebo Cream.

The antiwrinkle efficacy of the compositions on volunteer's face is assessed by:
- Wrinkle depth: Images of volunteer's crow's feet area are taken with a 3D microtopography imaging system. Wrinkle depth measurements are taken at initial time, at day 14 and after 28 days of product application. Results are shown in Table 10.

**Table 10. Wrinkle depth decrease after 14 days and 28 days of product application. Statistical significance respect initial time: * p<0.05 calculated using a paired Student's t test vs initial time.**

| | % of change at 14 days | % of change at 28 days |
|---|---|---|
| Active Cream | -14.4 * | -20.3 * |
| Placebo Cream | -1.6 | -2.3 |

Results demonstrate that, after 14 and 28 days of application of the composition of the invention, there is a statistically significant decrease of wrinkle depth compared to initial time. Moreover, the decrease in wrinkle depth is higher with the active cream than with placebo cream.
- Wrinkle volume: Images of volunteer's crow's feet area are taken with a 3D microtopography imaging system. Wrinkle volume measurements are taken at initial time, at day 14 and after 28 days of product application. Results are shown in Table 11.

**Table 11. Wrinkle volume decrease after 14 days and 28 days of product application. Statistical significance respect initial time: * p<0.05 calculated using a paired Student's t test vs initial time.**

| | % of change at 14 days | % of change at 28 days |
|---|---|---|
| Active Cream | -11.7 * | -14.3 * |
| Placebo Cream | -1.0 | -2.2 |

Results demonstrate that, after 14 days and 28 days of product application, there is a statistically significant decrease of wrinkle volume compared to initial time. Moreover, the decrease in wrinkle volume is higher with the active cream than the placebo.
- Wrinkle length: Images of volunteer's crow's feet area are taken with a 3D microtopography imaging system. Wrinkle length measurements are taken at initial time, at day 14 and after 28 days of product application. Results are shown in Table 12.

**Table 12. Wrinkle length decrease after 14 days and 28 days of product application. Statistical significance respect initial time: * p<0.05 calculated using a paired Student's t test vs initial time.**

| | % of change at 14 days | % of change at 28 days |
|---|---|---|
| Active Cream | -5.9 * | -8.0 * |
| Placebo Cream | +0.3 | +0.5 |

Results demonstrate that, after 14 days and 28 days of product application, there is a statistically significant decrease of wrinkle length compared to initial time. Moreover, the decrease in wrinkle length is higher with active cream than with the placebo.
- Skin wrinkledness clinical evaluation: by means of a clinical scale skin wrinkledness is evaluated by a dermatologist at initial time, at day 14 and after 28 days of product application. Results are shown in Table 13.

**Table 13. % of subjects showing an improvement at clinical evaluation after 14 and 28 days of product application.**

| | % of subjects showing an improvement at 14 days | % of subjects showing an improvement at 28 days |
|---|---|---|
| Active Cream | 36.7 | 56.7 |
| Placebo Cream | 10.0 | 16.7 |

Results demonstrate that, after 28 days of product application of the composition of the active cream, there is a higher percentage of subjects showing and improvement of skin wrinkledness than with placebo cream
- Self-questionnaire: after 28 days of active cream and placebo volunteers answered a self-questionnaire to evaluate the efficacy of both products. Results are shown in Table 14.

**Table 14. Percentage of positive responses to wrinkle-related questions after 28 days of product application.**

| | % of positive responses Active cream | % of positive responses Placebo cream |
|---|---|---|
| The product reduces skin roughness | 76.7 | 56.7 |
| After the use of the product my expression lines have reduced | 73.3 | 60.0 |
| After the use of the product I have noticed an improvement in my crow's feet area | 76.7 | 60.0 |
| After the use of the product the skin arround treated eye area seems smoothed | 80.0 | 73.3 |
| After the use of the product wrinkles are visibly reduced | 76.7 | 56.7 |
| The product has an anti-wrinkle efficacy | 80.0 | 60.0 |

Results demonstrate that, after 28 days, the active cream showed a higher improvement than placebo, according to the volunteers' answers.

### EXAMPLE 15

### Preparation of a Gel-cream comprising peptide PEP-22 (Ac-L-Arg-L-Arg-D-Gln-L-Met-L-Glu-L-Glu).

In a suitable vessel, the ingredients of phase A: water [INCI: WATER (AQUA)], Zemea^{™} [INCI: PROPANEDIOL], Phenoxetol^{®} [INCI: PHENOXYETHANOL], Dissolvine^{®} NA2 [INCI: DISODIUM EDTA] and Potassium Sorbate Granular [POTASSIUM SORBATE] are dispersed.

Phase A1 ingredient: Carbopol^{®} Ultrez 21 Polymer [INCI: ACRYLATES/ C10/30 ALKYL ACRYLATE CROSSPOLYMER] is added in the previous mixture under stirring. Once dispersed, phase A2: Xanthan Gum [INCI: XANTHAN GUM] is introduced to the previous mixture and stirred until complete dispersion.

In a separate vessel, phase B ingredients: Schercemol^{™} 1818 Ester [INCI: ISOSTEARYL ISOSTEARATE], is weighed.

The emulsion is made by adding slowly phase B onto phase A under fast stirring with a turbine.

Phase C: Peptide PEP-22 peptide solution [INCI: WATER (AQUA); CAPRYLYL GLYCOL; Peptide PEP-22] is added to the previous mixture.
pH is adjusted to 6.0 - 6.5 with phase D ingredient: Sodium Hydroxide 20% w/w [INCI: WATER (AQUA); SODIUM HYDROXIDE])

**Table 15**

| Phase | INGREDIENT (INCI name) | % weight |
|---|---|---|
| A | WATER | 83.54 |
| A | PROPANEDIOL | 10.00 |
| A | PHENOXYETHANOL | 0.35 |
| A | DISODIUM EDTA | 0.20 |
| A | POTASSIUM SORBATE | 0.10 |
| A1 | ACRYLATES/ C10/30 ALKYL ACRYLATE CROSSPOLYMER | 0.65 |
| A2 | XANTHAN GUM | 0.20 |
| B | ISOSTEARYL ISOSTEARATE | 2.00 |
| C | [WATER (AQUA); CAPRYLYL GLYCOL; PEPTIDE PEP-22] | 2.00 |
| D | [WATER (AQUA); SODIUM HYDROXIDE] | 0.96 |

### EXAMPLE 16

### Preparation of a lotion comprising 2% PEP-23 (Ac-L-Arg-L-Arg-L-Gln-D-Met-L-Glu-L-Glu-NH₂) and 2% ARGIRELINE^{®} peptide solution

In a suitable vessel, the ingredients of phase A1: water [INCI: WATER (AQUA)], Zemea^{™} [INCI: PROPANEDIOL], glycerin [INCI: GLYCERIN], potassium sorbate [INCI: POTASSIUM SORBATE] and Dissolvine^{®} NA2 [INCI: DISODIUM EDTA] are dissolved.

Phase A2 ingredient: Carbopol^{®} Ultrez 30 Polymer [INCI: CARBOMER] is added in the previous mixture. Once dispersed, phase A3: xanthan gum [INCI: XANTHAN GUM] is introduced. Then the mixture is heated at 70-75°C.

In a separate vessel, phase B ingredients: Fancor^{®} Meadowfoam seed oil [INCI: LIMNANTHES ALBA (MEADOWFOAM) SEED OIL], Kodasil 600 IDD Gel [INCI: ISODODECANE; VINYL DIMETHICONE/LAURYL DIMETHICONE CROSSPOLYMER; DIMETHICONE; LAURYL DIMETHICONE], Astro-sil 2C 350 [INCI: DIMETHICONE], Schercemol^{™} CATC ester [INCI: COCOYL ADIPIC ACID/TRIMETHYLOLPROPANE COPOLYMER; TRIMETHYLOLPROPANE], Schercemol^{™} DIS ester [INCI: DIISOPROPYL SEBACATE], Tocopheryl Acetate [INCI: TOCOPHERYL ACETATE] and Phenoxetol^{™} [INCI: PHENOXYETHANOL] are mixed and the resulting mixture is heated at 70-75°C.

The emulsion is made by adding slowly phase B onto phase A under conditions of fast stirring with a turbine.

Once the mixture is cooled to 40°C, phase C: Novemer^{™} EC-2 polymer [INCI: WATER (AQUA); SODIUM ACRYLATES/BEHENETH-25 METHACRYLATE CROSSPOLYMER; HYDROGENATED POLYDECENE, LAURYL GLUCOSIDE], SA-SB-300 (7%) [INCI: SILICA; DIMETHICONE], Fragrance [INCI: FRAGANCE (PARFUM)], ARGIRELINE^{®} peptide [INCI: WATER (AQUA); ACETYL HEXAPEPTIDE-8; CAPRYLYL GLYCOL] and PEP-23 peptide solution [INCI: WATER (AQUA); CAPRYLYL GLYCOL; PEP-23 peptide]) are added to the previous mixture.
pH is adjusted to 6.0 - 6.5 with phase D ingredient sodium hydroxide 20% w/w [INCI: WATER (AQUA); SODIUM HYDROXIDE])

**Table 16**

| Phase | INGREDIENT (INCI name) | % weight |
|---|---|---|
| A1 | WATER | 61.60 |
| A1 | PROPANEDIOL | 10.00 |
| A1 | GLYCERIN | 5.00 |
| A1 | POTASSIUM SORBATE | 0.10 |
| A1 | DISODIUM EDTA | 0.20 |
| A2 | CARBOMER | 0.30 |
| A3 | XANTHAN GUM | 0.20 |
| B | LIMNANTHES ALBA (MEADOWFOAM) SEED OIL | 5.00 |
| B | [ISODODECANE; VINYL DIMETHICONE/LAURYL DIMETHICONE CROSSPOLYMER; DIMETHICONE; LAURYL DIMETHICONE] | 3.00 |
| B | DIMETHICONE | 3.00 |
| B | [COCOYL ADIPIC ACID/TRIMETHYLOLPROPANE COPOLYMER; TRIMETHYLOLPROPANE] | 2.00 |
| B | DIISOPROPYL SEBACATE | 2.00 |
| B | TOCOPHERYL ACETATE | 0.50 |
| B | PHENOXYETHANOL | 0.50 |
| C | [WATER (AQUA); SODIUM ACRYLATES/BEHENETH-25 METHACRYLATE CROSSPOLYMER; HYDROGENATED POLYDECENE, LAURYL GLUCOSIDE] | 1.50 |
| C | [SILICA; DIMETHICONE] | 1.00 |
| C | [WATER (AQUA); CAPRYLYL GLYCOL; PEP-23] | 2.00 |
| C | [WATER (AQUA); ACETYL HEXAPEPTIDE-8; CAPRYLYL GLYCOL] | 2.00 |
| C | FRAGANCE (PARFUM) | 0.10 |
| D | [WATER (AQUA); SODIUM HYDROXIDE] | q.s. |

### EXAMPLE 17

### In vitro alleviation of the effects of muscle mass loss induced in human skeletal muscle cells by immunofluorescence.

Muscle mass loss correction by the peptides of the invention is evaluated by an immunofluorescence assay. The aim of this study is to investigate the capacity of peptide candidates to alleviate muscular mass loss after treatment with TNF-α in human muscular skeletal cells (hSKMC), i.e. to reverse the adverse effects induced by TNF-α (a model of muscular ageing). Myosin heavy chain (MHyC) is used as a morphologic marker of differentiated myotubes allowing the measurement of their diameters, and this can be used to ascertain a degree of mass loss. Tumor necrosis factor α (TNF-α) has been described in the literature as a muscle mass loss inductor.

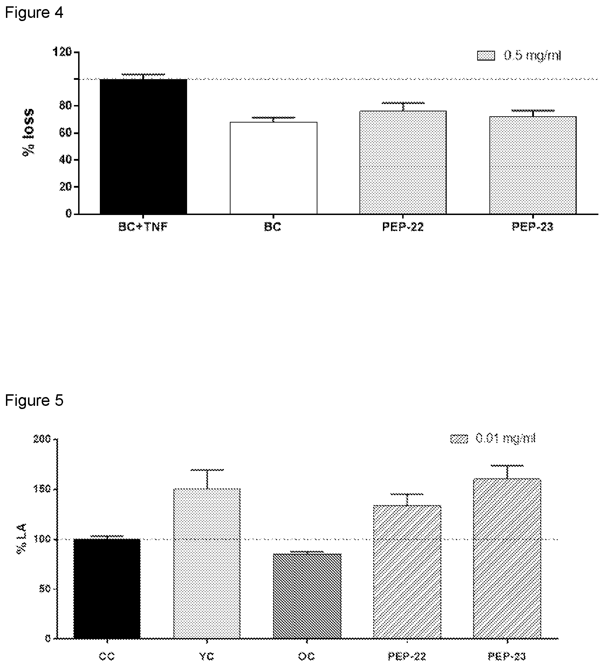

hSKMC (Tebu-Bio) are seeded in 96-well plates at a density of 1.5x10⁴ cells/well in Skeletal Muscle Cell Growth Medium (Promocell). After 24 hours incubation, the medium is replaced with Skeletal Muscle Cell Differentiation Medium (Promocell) and cells are differentiated for 6 days. After this, muscular cells mass loss is induced by adding fresh differentiation medium with 20 ng/ml TNFα (Sigma). Non-TNFα treated cells are used as a basal control (BC). After 24 hours of mass loss induction, the culture medium is replaced in some of the well plates with fresh differentiation medium comprising 0.01 mg/ml of the peptides of the invention. TNFα treated cells which are not treated with peptides are used as a mass loss control (BC + TNF) and cells which are treated with neither the peptides of the invention nor TNFα are used as a basal mass loss control (BC). Treatment is renewed 24 hours later. After 24 hours after the second treatment, immunofluorescence is determined. Cells are fixed 15 minutes with 4% Paraformaldehyde (Sigma), permeated 15 minutes with 1% Triton X-100 (Sigma) and then blocked with 5% Bovine Serum Albumin (Sigma). Next, primary antibody Anti-MHyC mouse (1:100, Vitro) is added and incubated overnight at 4°C. After that, secondary antibody Alexa Fluor 488 goat anti-mouse IgG (1:250, Life Technologies) is added and incubated for 1 hour. Finally, myotube diameters are determined by imaging MHyC staining by Operetta (Perkin Elmer). Percentage of mass loss is automatically calculated with Harmony (Perkin Elmer) software by classifying myotubes according to their diameter using a mass loss threshold of 20 µm. Results of the % muscle mass loss versus the non-treated cells of the basal control are shown in FIG. 4 and Table 17. The results are normalized to the mass loss control (BC + TNF).

**Table 17**

| | % Loss |
|---|---|
| Basal control (BC) + TNF-α | 100.0 |
| Basal control (BC) | 68.3 |
| PEP-22 | 76.2 |
| PEP-23 | 71.9 |

The results demonstrate that peptides PEP-22 and PEP-23 of the invention alleviate mass loss when compared to basal control treated with TNF-α.

### EXAMPLE 18

### In vitro effect of PEP-22 and PEP-23 in lipid accumulation in human subcutaneous pre-adipocytes.

Skin experiences a loss of adipose tissue, leading to an unaesthetic reduction in facial volume, with ageing. This loss of adipose tissue is mainly caused by adipocyte senescence, i.e. when adipocyte cells are no longer able to differentiate thus causing a decrease in normal lipid accumulation. In addition, old senescent adipocytes express a Senescence-associated secretory phenotype (SASP) that induces senescence on young adipocytes.

Measurement of lipid accumulation in a coculture of young and old adipose cells indirectly evaluates the inhibition of SASP. A pool of old adipose cells has a higher number of senescent cells. When cocultured with young cells, lipid accumulation observed is below the theoretically expected levels due to a senescence induction effect of SASP from old to young adipocytes. For this reason, compounds that are able to increase lipid accumulation can be used as a good approach for facial volume loss prevention caused by senescent of adipocytes.

Induction of lipid accumulation by the peptides of the invention is evaluated by lipid fluorescent staining with Adipored^{®} Assay Reagent. The aim of this study is to investigate the capacity of peptide candidates to induce lipid accumulation in human subcutaneous adipocytes, and therefore prevent adipocytes senescence.

Human subcutaneous pre-adipocytes (26-year-old and 60-year-old donors, Cell applications) cells are cocultured in 96-well plates at a density of 4x10³ cells/well each age in Human Preadipocyte Growth Medium (Sigma). Monoculture of each age are seeded at a density of 8x10³ cells/well as age lipid accumulation controls. After 24 hours incubation, differentiation of pre-adipocytes into adipocytes is induced by changing medium to fresh Human Preadipocyte Differentiation Medium (Promocell). At the same time, cocultured cells are treated with 0.01 mg/ml of peptides of the invention. Non-treated cocultured cells are used as basal control and monocultured young and old cells as age lipid accumulation controls. After 12 days of treatment and differentiation, cells are stained with Adipored^{®} Assay Reagent (Lonza) following the manufacturer's instructions. In brief, the plate is washed with Phosphate Buffer Saline (Sigma) and then Adipored^{®} reagent and Hoechst 33342 (Life technologies) are added and incubated for 15 minutes at 37°C. Finally, fluorescence intensity and nuclei number are measured by Operetta (Perkin Elmer). Fluorescence is normalized by cell nuclei and results are represented respect to cocultured basal control. Results of percentage of lipid content versus non-treated cells (basal control) is showed in FIG. 5 and Table 18. Abbreviations: % LA = Percentage of lipid accumulation; CC = Coculture control; YC = young control; OC = old control

**Table 18**

| | %LA |
|---|---|
| YC | 150.5 |
| OC | 85.29 |
| CC | 100.0 |
| PEP-22 | 133.9 |
| PEP-23 | 159.9 |

The results demonstrate that peptides PEP-22 and PEP-23 of the invention increase lipid accumulation in adipocytes when compared to basal control (co-culture, CC) not treated with peptides. The percentage of lipid accumulation in cells treated with PEP-22 and PEP-23 resembles that of young cells.

During the process of aging, lipid accumulation in adipocytes is decreased due to senescence. The peptides of the invention prevent senescence of adipocyte cells as they increase lipid accumulation in cocultured HPAd cells.

### EXAMPLE 19

### In vivo study for the assessment of antiwrinkle effect of the peptide PEP-23 of the invention after long-term application in Caucasian skin type female volunteers.

The study is carried out for 28 days. Forty-one (41) Caucasian female volunteers, aged between 35 and 60 years old showing skin wrinkledness on the face were included in the study. Subjects apply the composition described in Example 10 (Active Cream) on one side of the face (left or right) and a Placebo Cream having the same composition except the peptide of the invention on the other side of the face. Active and Placebo Cream are applied for 28 days twice a day (morning and evening). The subjects serve as their own reference and results obtained at time 5, 14 and 28 days are compared with those obtained at initial time. Moreover, results obtained with the Active Cream are compared with those obtained with Placebo Cream.

The skin isotropy is assessed by analysing the images of volunteer's crow's feet area taken with PRIMOS 3D microtopography imaging system. The isotropy is a determination of how the collagen fibers of the skin are structured and oriented. High levels of isotropy correspond to collagen fibers homogeneously oriented in different directions and are characteristic of young skin. Low levels of isotropy (i.e. anisotropy) are characteristic of aged skin. Thus, increasing the levels of isotropy of the skin is desirable to provide a younger-looking aspect.

Images taken with PRIMOS 3D microtopography imaging system at days 5, 14 and after 28 days of product application are analysed to measure skin isotropy. Results are shown in Table 19.

**Table 19. Skin's isotropy increase after 5 days, 14 days and 28 days of product application.**

| | Placebo Cream | Active Cream |
|---|---|---|
| % of increase at 5 days | -1.6 | 6.3 |
| % of increase at 14 days | -0.8 | 3.1 |
| % of increase at 28 days | 2.0 | 9.9 |

Results demonstrate that, after 5, 14 and 28 days of application of the Active Cream, there is an increase of skin's isotropy compared to initial time (i.e. at the start of day 1). Moreover, the increase in skin's isotropy is higher with the Active Cream and with the Placebo Cream.

### EXAMPLE 20

### In vivo study for the assessment of effect of peptide of PEP-23 on the volume of volunteer's faces after long-term application in Caucasian skin type female volunteers.

During the ageing process there is a natural process of facial volume decrease and increase of sagginess appearance of the skin. To assess the effect of PEP-23 on facial volume a study is carried out for 28 days and the efficacy of the product is evaluated in nineteen (19) Caucasian female volunteers, aged between 35 and 45 years. Subjects apply the composition described in Example 10 (Active Cream) on one side of the face (left or right) and a Placebo Cream having the same composition except the peptide of the invention on the other half face. Active and Placebo Cream are applied for 28 days twice a day (morning and evening). The subjects serve as their own reference and results obtained at 14 and 28 days are compared with those obtained at the initial time (beginning of day 1). Moreover, results obtained with the Active Cream are compared with those obtained with Placebo Cream.

The increase of volume on volunteer's face is assessed after the acquisition of the facial topography of the whole face with a 3D device. A dedicated software was used to analyse the volume in the cheek area.

Measurements at days 5, 14 and after 28 days of product application are analysed to obtain the volume of the face in the cheek area. Results are shown in Table 20.

**Table 20. Facial volume increase after 14 days and 28 days of product application.**

| | Placebo Cream | Active Cream |
|---|---|---|
| | | |
| % of increase at 14 days | 0.0 | 2.2 |
| % of increase at 28 days | 1.1 | 5.5 |

Results demonstrate that, after 5, 14 and 28 days of application of the composition of the invention, there is an increase of facial volume in the cheek area compared to initial time. Moreover, the increase is higher with Active Cream than with Placebo Cream.

## Claims

1. A compound of formula (I)
R₁-Wₘ-Xₙ-AA₁-AA₂-AA₃-AA₄-AA₅-AA₆-Yₚ-Z_{q}-R₂ (I),
a stereoisomer and/or cosmetically acceptable salt thereof, wherein:
AA₁ is Arg, Lys or no amino acid;
AA₂ is Arg or Lys;
AA₃ is Gln or Asp;
AA₄ is Met or Leu;
AA₅ is Glu, Asp or Gln;
AA₆ is Glu, Asp, Gln or no amino acid;
AA₁ is different from AA₆;
W, X, Y and Z are each independently an amino acid selected from the group consisting of Ala, Gly, Val and Ile;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl;
R₁ and R₂ are not amino acids; and
either (i) each of AA₁, AA₂, AA₃, AA₄, AA₅ and AA₆ is an L-amino acid, or (ii) 1, 2 or 3 of AA₃, AA₄ and AA₅ is a D-amino acid and the remaining of AA₁ to AA₆ are L-amino acids.

2. A compound according to claim 1, wherein AA₅ is selected from the group consisting of Glu and Asp; and AA₆ is selected from the group consisting of Glu, Gln or no amino acid.

3. A compound according to claim 1 or claim 2, wherein AA₁ is selected from the group consisting of Arg and Lys; and AA₆ is selected from the group consisting of Glu and Gln.

4. A compound according to any of the preceding claims, wherein AA₁ is Arg and/or AA₂ is Arg.

5. A compound according to any one of the preceding claims, wherein 1 or 2 of AA₃, AA₄ and AA₅ is a D-amino acid and the remaining of AA₁ to AA₆ are L-amino acids and, preferably, AA₄ is a D-amino acid and the remaining of AA₁ to AA₆ are L-amino acids.

6. A compound according to claim 5, wherein the compound has a formula of:
R₁-Wₘ₋Xₙ-L-Arg-L-Arg-D-Gln-L-Met-L-Glu-L-Glu-Yₚ-Z_{q}-R₂ (II);
R₁-Wₘ-Xₙ-L-Arg-L-Arg-L-Gln-D-Met-L-Glu-L-Glu-Yₚ-Z_{q}-R₂ (III);
R₁-Wₘ-Xₙ-L-Arg-L-Arg-D-Gln-D-Met-L-Glu-L-Glu-Yₚ-Z_{q}-R₂ (IV);
R₁-Wₘ-Xₙ-L-Arg-L-Arg-L-Asp-D-Met-L-Glu-L-Glu-Yₚ-Z_{q}-R₂ (V);
R₁-Wₘ-Xₙ-L-Arg-L-Arg-L-Gln-D-Met-L-Asp-L-Glu-Yₚ-Z_{q}-R₂ (VI);
R₁-Wₘ-Xₙ-L-Arg-L-Arg-L-Gln-D-Met-L-Glu-L-Gln-Yₚ-Z_{q}-R₂ (VII);
R₁-Wₘ-Xₙ-L-Arg-L-Lys-L-Gln-D-Met-L-Glu-L-Glu-Yₚ-Z_{q}-R₂ (VIII);
R₁-Wₘ-Xₙ-L-Lys-L-Arg-L-Gln-D-Met-L-Glu-L-Glu-Yₚ-Z_{q}-R₂ (IX);
R₁-Wₘ-L-Ala-L-Arg-L-Arg-L-Gln-D-Met-L-Glu-L-Glu-L-Ala-Z_{q}-R₂ (X);
R₁-Wₘ-Xₙ-L-Arg-L-Gln-D-Met-L-Glu-L-Glu-Yₚ-Z_{q}-R₂ (XI);
R₁-Wₘ-Xₙ-L-Arg-L-Arg-L-Gln-D-Met- L-Glu-Yₚ-Z_{q}-R₂ (XII)
; or
R₁-Wₘ-Xₙ-L-Arg-L-Arg-L-Gln-D-Leu-L-Glu-L-Glu-Yₚ-Z_{q}-R₂ (XIII).

7. A compound according to claim 6, wherein the compound has a formula of:
R₁-Wₘ-Xₙ-L-Arg-L-Arg-D-Gln-L-Met-L-Glu-L-Glu-Yₚ-Z_{q}-R₂ (II).

8. A compound according to claim 6, wherein the compound has a formula of:
R₁-Wₘ-Xₙ-L-Arg-L-Arg-L-Gln-D-Met-L-Glu-L-Glu-Yₚ-Z_{q}-R₂ (III).

9. A compound according to claim 6, wherein the compound has a formula of:
R₁-Wₘ-Xₙ-L-Arg-L-Arg-D-Gln-D-Met-L-Glu-L-Glu-Yₚ-Z_{q}-R₂ (IV).

10. A compound according to any of the previous claims wherein m, n, p and q are each 0.

11. A compound according to any one of the preceding claims, wherein R₁ is selected from the group consisting of H and R₅-CO-, wherein R₅ is selected from the group consisting of C₁-C₁₈ alkyl, C₂-C₂₄ alkenyl, C₃-C₂₄ cycloalkyl; and R₂ is -NR₃R₄ or -OR₃ wherein R₃ and R₄ are independently selected from the group consisting of H and C₁-C₁₆ alkyl.

12. A compound according to claim 11, wherein R₁ is selected from the group consisting of H, acetyl, palmitoyl, lauroyl or myristyl; and R₂ is -NR₃R₄ or -OR₃, wherein R₃ and R₄ are independently selected from the group consisting of H and C₁-C₁₆ alkyl.

13. A compound according to claim 12, wherein R₁ is selected from the group consisting of H, acetyl, or palmitoyl; and R₂ is -NH₂ or -OH and, preferably R₁ is acetyl and R₂ is NH₂.

14. A compound according to claim 1, wherein the compound is:
Ac-L-Arg-L-Arg-L-Gln-L-Met-L-Glu-L-Glu-NH₂ (PEP-21);
Ac-L-Arg-L-Arg-D-Gln-L-Met-L-Glu-L-Glu-NH₂ (PEP-22);
Ac-L-Arg-L-Arg-L-Gln-D-Met-L-Glu-L-Glu-NH₂ (PEP-23);
Ac-L-Arg-L-Arg-D-Gln-D-Met-L-Glu-L-Glu-NH₂ (PEP-24);
Ac-L-Arg-L-Arg-L-Gln-D-Met-L-Glu-L-Glu-OH (PEP-26);
H-L-Arg-L-Arg-L-Gln-D-Met-L-Glu-L-Glu-NH₂ (PEP-27);
Ac-L-Arg-L-Arg-L-Asp-D-Met-L-Glu-L-Glu-NH₂ (PEP-41);
Ac-L-Arg-L-Arg-L-Gln-D-Met-L-Asp-L-Glu-NH₂ (PEP-30);
Ac-L-Arg-L-Arg-L-Gln-D-Met-L-Glu-L-Gln-NH₂ (PEP-31);
Ac-L-Arg-L-Lys-L-Gln-D-Met-L-Glu-L-Glu-NH₂ (PEP-35);
Ac-L-Lys-L-Arg-L-Gln-D-Met-L-Glu-L-Glu-NH₂ (PEP-36);
Ac-L-Ala-L-Arg-L-Arg-L-Gln-D-Met-L-Glu-L-Glu-L-Ala-NH₂ (PEP-42);
Ac-L-Arg-L-Gln-D-Met-L-Glu-L-Glu-NH₂ (PEP-43);
Ac-L-Arg-L-Arg-L-Gln-D-Met-L-Glu-NH₂ (PEP-44);
or
Ac-L-Arg-L-Arg-L-Gln-D-Leu-L-Glu-L-Glu-NH₂ (PEP-37).

15. A compound according to claim 1, wherein the compound is:
Ac-L-Arg-L-Arg-L-Gln-L-Met-L-Glu-L-Glu-NH₂ (PEP-21).

16. A compound according to claim 1, wherein the compound is:
Ac-L-Arg-L-Arg-D-Gln-L-Met-L-Glu-L-Glu-NH₂ (PEP-22).

17. A compound according to claim 1, wherein the compound is:
Ac-L-Arg-L-Arg-L-Gln-D-Met-L-Glu-L-Glu-NH₂ (PEP-23).

18. A combination of a compound according to any one of claims 1 to 17, or cosmetically acceptable salt thereof, and Botulinum toxin and/or the peptide of sequence Ac-Glu-Glu-Met-Gln-Arg-Arg-NH₂.

19. A composition comprising a cosmetically effective quantity of a compound of formula (I) according to any one of claims 1 to 17, or cosmetically acceptable salt thereof, or a combination according to claim 18, and at least one cosmetically acceptable excipient or adjuvant.

20. Use of a compound according to any one of claims 1 to 17, or a cosmetically acceptable salt thereof, for the cosmetic, non-therapeutic treatment and/or care of the skin, hair, nails and/or mucous membranes.

21. The use according to claim 20, wherein the cosmetic, non-therapeutic treatment and/or care is: the treatment and/or prevention of skin aging; the reduction and/or prevention of skin wrinkles; the stimulation of collagen synthesis and/or prevention of collagen loss; the improvement or maintenance of skin firmness; the treatment and/or prevention of sagging appearance of the skin; the reduction and/or prevention of facial asymmetry; the increase of the volume of adipose tissue and/or the prevention and/or alleviation of the effects of adipose tissue loss.

22. A method of cosmetic, non-therapeutic treatment and/or care of the skin, hair, nails and/or mucous membranes of a subject comprising administering a cosmetically effective amount of a compound according to any one of claims 1 to 17, a combination according to claim 18 or a composition according to claim 19, to the subject.

23. A method of cosmetic, non-therapeutic treatment and/or care of the skin, hair, nails and/or mucous membranes of a subject according to claim 22, wherein cosmetic, non-therapeutic treatment and/or care is: the treatment and/or prevention of skin aging; the reduction and/or prevention of skin wrinkles; the stimulation of collagen synthesis and/or prevention of collagen loss; the improvement or maintenance of skin firmness; the treatment and/or prevention of sagging appearance of the skin; the reduction and/or prevention of facial asymmetry; the increase of the volume of adipose tissue and/or the prevention and/or alleviation of the effects of adipose tissue loss.

## Patentansprüche

1. Verbindung von Formel (I)
R₁-Wₘ-Xₙ-AA₁-AA₂-AA₃-AA₄-AA₅-AA₆-Yₚ-Z_{q}-R₂ (I),
ein Stereoisomer und/oder kosmetisch verträgliches Salz davon, wobei:
AA₁ Arg, Lys oder keine Aminosäure ist;
AA₂ Arg oder Lys ist;
AA₃ Gln oder Asp ist;
AA₄ Met oder Leu ist;
AA₅ Glu, Asp oder Gln ist;
AA₆ Glu, Asp, Gln oder keine Aminosäure ist;
AA₁ sich von AA₆ unterscheidet;
W, X, Y und Z jeweils unabhängig voneinander eine Aminosäure sind, die aus der Gruppe ausgewählt ist, bestehend aus Ala, Gly, Val und Ile;
m, n, p und q jeweils unabhängig 0 oder 1 sind;
m+n+p+q kleiner als oder gleich 2 ist;
R₁ aus der Gruppe ausgewählt ist, bestehend aus H, einem Polymer, das von Polyethylenglycol abgeleitet ist, einer nicht cyclischen aliphatischen Gruppe, Alicyclyl, Heterocyclyl, Heteroarylalkyl, Aryl, Aralkyl und R₅-CO-, wobei R₅ aus der Gruppe ausgewählt ist, bestehend aus H, einer nicht cyclischen aliphatischen Gruppe, Alicyclyl, Aryl, Aralkyl, Heterocyclyl und Heteroarylalkyl;
R₂ aus der Gruppe ausgewählt ist, bestehend aus -NR₃R₄, -OR₃, -SR₃, wobei R₃ und R₄ unabhängig aus einer Gruppe ausgewählt sind, bestehend aus H, einem Polymer, das von Polyethylenglycol abgeleitet ist, einer nicht cyclischen aliphatischen Gruppe, Alicyclyl, Heterocyclyl, Heteroarylalkyl, Aryl und Aralkyl;
R₁ und R₂ keine Aminosäuren sind; und
entweder (i) jedes von AA₁, AA₂, AA₃, AA₄, AA₅ und AA₆ eine L-Aminosäure ist, oder (ii) 1, 2 oder 3 von AA₃, AA₄ und AA₅ eine D-Aminosäure ist und die restlichen von AA₁ bis AA₆ L-Aminosäuren sind.

2. Verbindung nach Anspruch 1, wobei AA₅ aus der Gruppe ausgewählt ist, bestehend aus Glu und Asp; und AA₆ aus der Gruppe ausgewählt ist, bestehend aus Glu, Gln oder keiner Aminosäure.

3. Verbindung nach Anspruch 1 oder 2, wobei AA₁ aus der Gruppe ausgewählt ist, bestehend aus Arg und Lys; und AA₆ aus der Gruppe ausgewählt ist, bestehend aus Glu und Gln.

4. Verbindung nach einem der vorstehenden Ansprüche, wobei AA₁ Arg ist und/oder AA₂ Arg ist.

5. Verbindung nach einem der vorstehenden Ansprüche, wobei 1 oder 2 von AA₃, AA₄ und AA₅ eine D-Aminosäure sind und die übrigen von AA₁ bis AA₆ L-Aminosäuren sind und vorzugsweise AA₄ eine D-Aminosäure ist und die übrigen von AA₁ bis AA₆ L-Aminosäuren sind.

6. Verbindung nach Anspruch 5, wobei die Verbindung eine Formel aufweist:
R₁-Wₘ-Xₙ-L-Arg-L-Arg-D-GIn-L-Met-L-Glu-L-Glu-Yₚ-Z_{q}-R₂ (II);
R₁-Wₘ-Xₙ-L-Arg-L-Arg-L-Gln-D-Met-L-Glu-L-Glu-Yₚ-Z_{q}-R₂ (III);
R₁-Wₘ-Xₙ-L-Arg-L-Arg-D-GIn-D-Met-L-Glu-L-Glu-Yₚ-Z_{q}-R₂ (IV);
R₁-Wₘ-Xₙ-L-Arg-L-Arg-L-Asp-D-Met-L-Glu-L-Glu-Yₚ-Z_{q}-R₂ (V);
R₁-Wₘ-Xₙ-L-Arg-L-Arg-L-GIn-D-Met-L-Asp-L-Glu-Yₚ-Z_{q}-R₂ (VI);
R₁-Wₘ-_{Xn}-L-Arg-L-Arg-L-GIn-D-Met-L-Glu-L-Gln-Yₚ-Z_{q}-R₂ (VII);
R₁-Wₘ-Xₙ-L-Arg-L-Lys-L-GIn-D-Met-L-Glu-L-Glu-Yₚ-Z_{q}-R₂ (VIII);
R₁-Wₘ-Xₙ-L-Lys-L-Arg-L-GIn-D-Met-L-Glu-L-Glu-Yₚ-Z_{q}-R₂ (IX);
R₁-Wₘ-L-Ala-L-Arg-L-Arg-L-Gln-D-Met-L-Glu-L-Glu-L-Ala-Z_{q}-R₂ (X);
R₁-Wₘ-Xₙ-L-Arg-L-GIn-D-Met-L-Glu-L-Glu-Yₚ-Z_{q}-R₂ (XI);
R₁-Wₘ-Xₙ-L-Arg-L-Arg-L-Gln-D-Met- L-Glu-Yₚ-Z_{q}-R₂ (XII)
; oder
R₁-Wₘ-Xₙ-L-Arg-L-Arg-L-GIn-D-Leu-L-Glu-L-Glu-Yₚ-Z_{q}-R₂ (XIII).

7. Verbindung nach Anspruch 6, wobei die Verbindung eine Formel aufweist:
R₁-Wₘ-Xₙ-L-Arg-L-Arg-D-GIn-L-Met-L-Glu-L-Glu-Yₚ-Z_{q}-R₂ (II).

8. Verbindung nach Anspruch 6, wobei die Verbindung eine Formel aufweist:
R₁-Wₘ-Xₙ-L-Arg-L-Arg-L-GIn-D-Met-L-Glu-L-Glu-Yₚ-Z_{q}-R₂ (III).

9. Verbindung nach Anspruch 6, wobei die Verbindung eine Formel aufweist:
R₁-Wₘ-Xₙ-L-Arg-L-Arg-D-Gln-D-Met-L-Glu-L-Glu-Yₚ-Z_{q}-R₂ (IV).

10. Verbindung nach einem der vorstehenden Ansprüche, wobei m, n, p und q jeweils 0 sind.

11. Verbindung nach einem der vorstehenden Ansprüche, wobei R₁ aus der Gruppe ausgewählt ist, bestehend aus H und R₅-CO-, wobei R₅ aus der Gruppe ausgewählt ist, bestehend aus C₁-C₁₈-Alkyl, C₂₋C₂₄-Alkenyl, C₃-C₂₄-Cycloalkyl; und R₂ -NR₃R₄ oder -OR₃ ist, wobei R₃ und R₄ unabhängig voneinander aus der Gruppe ausgewählt sind, bestehend aus H und C₁₋C₁₆-Alkyl.

12. Verbindung nach Anspruch 11, wobei R₁ aus der Gruppe ausgewählt ist, bestehend aus H, Acetyl, Palmitoyl, Lauroyl oder Myristyl; und R₂ -NR₃R₄ oder - OR₃ ist, wobei R₃ und R₄ unabhängig voneinander aus der Gruppe ausgewählt sind, bestehend aus H und C₁-C₁₆-Alkyl.

13. Verbindung nach Anspruch 12, wobei R₁ aus der Gruppe ausgewählt ist, bestehend aus H, Acetyl oder Palmitoyl; und R₂ -NH₂ oder -OH ist und vorzugsweise R₁ Acetyl ist und R₂ NH₂ ist.

14. Verbindung nach Anspruch 1, wobei die Verbindung ist:
Ac-L-Arg-L-Arg-L-Gln-L-Met-L-Glu-L-Glu-NH₂ (PEP-21);
Ac-L-Arg-L-Arg-D-Gln-L-Met-L-Glu-L-Glu-NH₂ (PEP-22);
Ac-L-Arg-L-Arg-L-Gln-D-Met-L-Glu-L-Glu-NH₂ (PEP-23);
Ac-L-Arg-L-Arg-D-Gln-D-Met-L-Glu-L-Glu-NH₂ (PEP-24);
Ac-L-Arg-L-Arg-L-GIn-D-Met-L-Glu-L-Glu-OH (PEP-26);
H-L-Arg-L-Arg-L-Gln-D-Met-L-Glu-L-Glu-NH₂ (PEP-27);
Ac-L-Arg-L-Arg-L-Asp-D-Met-L-Glu-L-Glu-NH₂ (PEP-41);
Ac-L-Arg-L-Arg-L-Gln-D-Met-L-Asp-L-Glu-NH₂ (PEP-30);
Ac-L-Arg-L-Arg-L-Gln-D-Met-L-Glu-L-Gln-NH₂ (PEP-31);
Ac-L-Arg-L-Lys-L-Gln-D-Met-L-Glu-L-Glu-NH₂ (PEP-35);
Ac-L-Lys-L-Arg-L-Gln-D-Met-L-Glu-L-Glu-NH₂ (PEP-36);
Ac-L-Ala-L-Arg-L-Arg-L-Gln-D-Met-L-Glu-L-Glu-L-Ala-NH₂ (PEP-42);
Ac-L-Arg-L-Gln-D-Met-L-Glu-L-Glu-NH₂ (PEP-43);
Ac-L-Arg-L-Arg-L-Gln-D-Met-L-Glu-NH₂ (PEP-44); oder
Ac-L-Arg-L-Arg-L-Gln-D-Leu-L-Glu-L-Glu-NH₂ (PEP-37).

15. Verbindung nach Anspruch 1, wobei die Verbindung ist:
Ac-L-Arg-L-Arg-L-Gln-L-Met-L-Glu-L-Glu-NH₂ (PEP-21).

16. Verbindung nach Anspruch 1, wobei die Verbindung ist:
Ac-L-Arg-L-Arg-D-Gln-L-Met-L-Glu-L-Glu-NH₂ (PEP-22).

17. Verbindung nach Anspruch 1, wobei die Verbindung ist:
Ac-L-Arg-L-Arg-L-Gln-D-Met-L-Glu-L-Glu-NH₂ (PEP-23).

18. Kombination aus einer Verbindung nach einem der Ansprüche 1 bis 17 oder einem kosmetisch verträglichen Salz davon und Botulinumtoxin und/oder dem Peptid der Sequenz Ac-Glu-Glu-Met-Gln-Arg-Arg-NH₂.

19. Zusammensetzung, umfassend eine kosmetisch wirksame Menge einer Verbindung von Formel (I) nach einem der Ansprüche 1 bis 17 oder eines kosmetisch verträglichen Salzes davon oder eine Kombination nach Anspruch 18 und mindestens einen kosmetisch verträglichen Hilfsstoff oder Zusatzstoff.

20. Verwendung einer Verbindung nach einem der Ansprüche 1 bis 17 oder ein kosmetisch verträgliches Salz davon für die kosmetische, nicht therapeutische Behandlung und/oder Pflege der Haut, der Haare, der Nägel und/oder der Schleimhäute.

21. Verwendung nach Anspruch 20, wobei die kosmetische, nicht therapeutische Behandlung und/oder Pflege ist: die Behandlung und/oder die Vorbeugung von Hautalterung; die Verringerung und/oder die Vorbeugung von Hautfalten; die Stimulation von Kollagensynthese und/oder die Vorbeugung von Kollagenverlust; die Verbesserung oder die Erhaltung von Hautfestigkeit; die Behandlung und/oder die Vorbeugung eines schlaffen Aussehens der Haut; die Verringerung und/oder die Vorbeugung von Gesichtsasymmetrie; die Vergrößerung des Volumens von Fettgewebe und/oder die Vorbeugung und/oder Linderung der Auswirkungen von Fettgewebeverlust.

22. Verfahren für eine kosmetische, nicht therapeutische Behandlung und/oder Pflege der Haut, der Haare, der Nägel und/oder der Schleimhäute eines Subjekts, umfassend ein Verabreichen einer kosmetisch wirksamen Menge einer Verbindung nach einem der Ansprüche 1 bis 17, einer Kombination nach Anspruch 18 oder einer Zusammensetzung nach Anspruch 19 an das Subjekt.

23. Verfahren für die kosmetische, nicht therapeutische Behandlung und/oder Pflege der Haut, der Haare, der Nägeln und/oder der Schleimhäute eines Subjekts nach Anspruch 22, wobei die kosmetische, nicht therapeutische Behandlung und/oder Pflege ist: die Behandlung und/oder die Vorbeugung von Hautalterung; die Verringerung und/oder die Vorbeugung von Hautfalten; die Stimulation von Kollagensynthese und/oder die Vorbeugung von Kollagenverlust; die Verbesserung oder die Erhaltung von Hautfestigkeit; die Behandlung und/oder die Vorbeugung eines schlaffen Aussehens der Haut; die Verringerung und/oder die Vorbeugung von Gesichtsasymmetrie; die Vergrößerung des Volumens von Fettgewebe und/oder die Vorbeugung und/oder Linderung der Auswirkungen von Fettgewebeverlust.

## Revendications

1. Composé de formule (I)
R₁-Wₘ-Xₙ-AA₁-AA₂-AA₃-AA₄-AA₅-AA₆-Yₚ-Z_{q}-R₂ (I),
un stéréoisomère et/ou sel de celui-ci acceptable sur le plan cosmétique, où :
AA₁ est Arg, Lys ou aucun acide aminé ;
AA₂ est Arg ou Lys ;
AA₃ est Gln ou Asp ;
AA₄ est Met ou Leu ;
AA₅ est Glu, Asp ou Gln ;
AA₆ est Glu, Asp, Gln ou aucun acide aminé ;
AA₁ est différent de AA₆ ;
W, X, Y et Z sont chacun indépendamment un acide aminé choisi dans le groupe constitué d'Ala, Gly, Val et Ile ;
m, n, p et q valent chacun indépendamment 0 ou 1 ;
m + n + p + q est inférieur ou égal à 2 ;
R₁ est choisi dans le groupe constitué de H, un polymère dérivé de polyéthylène glycol, un groupe aliphatique non cyclique, alicyclyle, hétérocyclyle, hétéroarylalkyle, aryle, aralkyle et R₅-CO-, où R₅ est choisi dans le groupe constitué de H, un groupe aliphatique non cyclique, alicyclyle, aryle, aralkyle, hétérocyclyle et hétéroarylalkyle ;
R₂ est choisi dans le groupe constitué de -NR₃R₄, -OR₃, -SR₃, où R₃ et R₄ sont choisis indépendamment dans un groupe constitué de H, un polymère dérivé de polyéthylène glycol, un groupe aliphatique non cyclique, alicyclyle, hétérocyclyle, hétéroarylalkyle, aryle et aralkyle ;
R₁ et R₂ ne sont pas des acides aminés ; et
soit (i) chacun de AA₁, AA₂, AA₃, AA₄, AA₅ et AA₆ est un acide aminé L, soit (ii) 1, 2 ou 3 de AA₃, AA₄ et AA₅ est un acide aminé D et le reste de AA₁ à AA₆ sont des acides aminés L.

2. Composé selon la revendication 1, où AA₅ est choisi dans le groupe constitué de Glu et Asp ; et AA₆ est choisi dans le groupe constitué de Glu, Gln ou aucun acide aminé.

3. Composé selon la revendication 1 ou la revendication 2, où AA₁ est choisi dans le groupe constitué d'Arg et Lys ; et AA₆ est choisi dans le groupe constitué de Glu et Gln.

4. Composé selon l'une quelconque des revendications précédentes, où AA₁ est Arg et/ou AA₂ est Arg.

5. Composé selon l'une quelconque des revendications précédentes, dans lequel 1 ou 2 de AA₃, AA₄ et AA₅ sont des acides aminés D et le reste de AA₁ à AA₆ sont des acides aminés L et, de préférence, AA₄ est un acide aminé D et le reste de AA₁ à AA₆ sont des acides aminés L.

6. Composé selon la revendication 5, où le composé a une formule de :
R₁-Wₘ-Xₙ-L-Arg-L-Arg-D-Gln-L-Met-L-Glu-L-Glu-Yₚ-Z_{q}-R₂ (II) ;
R₁-Wₘ-Xₙ-L-Arg-L-Arg-L-Gln-D-Met-L-Glu-L-Glu-Yₚ-Z_{q}-R₂ (III) ;
R₁-Wₘ-Xₙ-L-Arg-L-Arg-D-GIn-D-Met-L-Glu-L-Glu-Yₚ-Z_{q}-R₂ (IV) ;
R₁-Wₘ-Xₙ-L-Arg-L-Arg-L-Asp-D-Met-L-Glu-L-Glu-Yₚ-Z_{q}-R₂ (V) ;
R₁-Wₘ-Xₙ-L-Arg-L-Arg-L-GIn-D-Met-L-Asp-L-Glu-Yₚ-Z_{q}-R₂ (VI) ;
R₁-Wₘ-Xₙ-L-Arg-L-Arg-L-GIn-D-Met-L-Glu-L-Gln-Yₚ-Z_{q}-R₂ (VII) ;
R₁-Wₘ-Xₙ-L-Arg-L-Lys-L-GIn-D-Met-L-Glu-L-Glu-Yₚ-Z_{q}-R₂ (VIII) ;
R₁-Wₘ-Xₙ-L-Lys-L-Arg-L-GIn-D-Met-L-Glu-L-Glu-Yₚ-Z_{q}-R₂ (IX) ;
R₁-Wₘ-L-Ala-L-Arg-L-Arg-L-Gln-D-Met-L-Glu-L-Glu-L-Ala-Z_{q}-R₂ (X) ;
R₁-Wₘ-Xₙ-L-Arg-L-GIn-D-Met-L-Glu-L-Glu-Yₚ-Z_{q}-R₂ (XI) ;
R₁-Wₘ-Xₙ-L-Arg-L-Arg-L-Gln-D-Met- L-Glu-Yₚ-Z_{q}-R₂ (XII)
; ou
R₁-Wₘ-Xₙ-L-Arg-L-Arg-L-GIn-D-Leu-L-Glu-L-Glu-Yₚ-Z_{q}-R₂ (XIII).

7. Composé selon la revendication 6, où le composé a une formule de :
R₁-Wₘ-Xₙ-L-Arg-L-Arg-D-GIn-L-Met-L-Glu-L-Glu-Yₚ-Z_{q}-R₂ (II).

8. Composé selon la revendication 6, où le composé a une formule de :
R₁-Wₘ-Xₙ-L-Arg-L-Arg-L-GIn-D-Met-L-Glu-L-Glu-Yₚ-Z_{q}-R₂ (III).

9. Composé selon la revendication 6, où le composé a une formule de :
R₁-Wₘ-Xₙ-L-Arg-L-Arg-D-Gln-D-Met-L-Glu-L-Glu-Yₚ-Z_{q}-R₂ (IV).

10. Composé selon l'une quelconque des revendications précédentes, où m, n, p et q valent chacun 0.

11. Utilisation selon l'une quelconque des revendications précédentes, où R₁ est choisi dans le groupe constitué de H et R₅-CO-, où R₅ est choisi dans le groupe constitué d'alkyle en C₁-C₁₈, alcényle en C₂-C₂₄, cycloalkyle en C₃-C₂₄ ; et R₂ est -NR₃R₄ ou -OR₃, où R₃ et R₄ sont choisis indépendamment dans le groupe constitué de H et alkyle en C₁-C₁₆.

12. Composé selon la revendication 11, où R₁ est choisi dans le groupe constitué de H, acétyle, palmitoyle, lauroyle ou myristyle ; et R₂ est -NR₃R₄ ou - OR₃, où R₃ et R₄ sont choisis indépendamment dans le groupe constitué de H et alkyle en C₁-C₁₆.

13. Composé selon la revendication 12, où R₁ est choisi dans le groupe constitué de H, acétyle, ou palmitoyle ; et R₂ est -NH₂ ou -OH et, de préférence, R₁ est acétyle et R₂ est NH₂.

14. Composé selon la revendication 1, dans lequel ledit composé est :
Ac-L-Arg-L-Arg-L-Gln-L-Met-L-Glu-L-Glu-NH₂ (PEP-21) ;
Ac-L-Arg-L-Arg-D-Gln-L-Met-L-Glu-L-Glu-NH₂ (PEP-22) ;
Ac-L-Arg-L-Arg-L-Gln-D-Met-L-Glu-L-Glu-NH₂ (PEP-23) ;
Ac-L-Arg-L-Arg-D-Gln-D-Met-L-Glu-L-Glu-NH₂ (PEP-24) ;
Ac-L-Arg-L-Arg-L-GIn-D-Met-L-Glu-L-Glu-OH (PEP-26) ;
H-L-Arg-L-Arg-L-Gln-D-Met-L-Glu-L-Glu-NH₂ (PEP-27) ;
Ac-L-Arg-L-Arg-L-Asp-D-Met-L-Glu-L-Glu-NH₂ (PEP-41) ;
Ac-L-Arg-L-Arg-L-Gln-D-Met-L-Asp-L-Glu-NH₂ (PEP-30) ;
Ac-L-Arg-L-Arg-L-Gln-D-Met-L-Glu-L-Gln-NH₂ (PEP-31) ;
Ac-L-Arg-L-Lys-L-Gln-D-Met-L-Glu-L-Glu-NH₂ (PEP-35) ;
Ac-L-Lys-L-Arg-L-Gln-D-Met-L-Glu-L-Glu-NH₂ (PEP-36) ;
Ac-L-Ala-L-Arg-L-Arg-L-Gln-D-Met-L-Glu-L-Glu-L-Ala-NH₂ (PEP-42) ;
Ac-L-Arg-L-Gln-D-Met-L-Glu-L-Glu-NH₂ (PEP-43) ;
Ac-L-Arg-L-Arg-L-Gln-D-Met-L-Glu-NH₂ (PEP-44) ; ou
Ac-L-Arg-L-Arg-L-Gln-D-Leu-L-Glu-L-Glu-NH₂ (PEP-37).

15. Composé selon la revendication 1, dans lequel ledit composé est :
Ac-L-Arg-L-Arg-L-Gln-L-Met-L-Glu-L-Glu-NH₂ (PEP-21).

16. Composé selon la revendication 1, dans lequel ledit composé est :
Ac-L-Arg-L-Arg-D-Gln-L-Met-L-Glu-L-Glu-NH₂ (PEP-22).

17. Composé selon la revendication 1, dans lequel ledit composé est :
Ac-L-Arg-L-Arg-L-Gln-D-Met-L-Glu-L-Glu-NH₂ (PEP-23).

18. Combinaison d'un composé selon l'une quelconque des revendications 1 à 17, ou d'un sel de celui-ci acceptable sur le plan cosmétique, et de toxine botulique et/ou du peptide de séquence Ac-Glu-Glu-Met-Gln-Arg-Arg-NH₂.

19. Composition comprenant une quantité efficace sur le plan cosmétique d'un composé de formule (I) selon l'une quelconque des revendications 1 à 17, ou d'un sel de celui-ci acceptable sur le plan cosmétique, ou d'une combinaison selon la revendication 18, et au moins un excipient ou adjuvant acceptable sur le plan cosmétique.

20. Utilisation d'un composé selon l'une quelconque des revendications 1 à 17, ou d'un sel de celui-ci acceptable sur le plan cosmétique, pour le traitement et/ou le soin cosmétique et non thérapeutique de la peau, des cheveux, des ongles et/ou des muqueuses.

21. Utilisation selon la revendication 20, où le traitement et/ou le soin cosmétique et non thérapeutique est : le traitement et/ou la prévention du vieillissement cutané ; la réduction et/ou la prévention des rides de la peau ; la stimulation de la synthèse de collagène et/ou la prévention de la perte de collagène ; l'amélioration ou le maintien de la fermeté de la peau ; le traitement et/ou la prévention de l'aspect relâché de la peau ; la réduction et/ou la prévention de l'asymétrie faciale ; l'augmentation du volume de tissu adipeux et/ou la prévention et/ou l'atténuation des effets de la perte de tissu adipeux.

22. Procédé de traitement et/ou de soin cosmétique et non thérapeutique de la peau, des cheveux, des ongles et/ou des muqueuses d'un sujet, comprenant l'administration d'une quantité efficace sur le plan cosmétique d'un composé selon l'une quelconque des revendications 1 à 17, d'une combinaison selon la revendication 18 ou d'une composition selon la revendication 19, au sujet.

23. Procédé de traitement et/ou de soin cosmétique et non thérapeutique de la peau, des cheveux, des ongles et/ou des muqueuses d'un sujet selon la revendication 22, où ledit traitement et/ou le soin cosmétique et non thérapeutique est : le traitement et/ou la prévention du vieillissement cutané ; la réduction et/ou la prévention des rides de la peau ; la stimulation de la synthèse de collagène et/ou la prévention de la perte de collagène ; l'amélioration ou le maintien de la fermeté de la peau ; le traitement et/ou la prévention de l'aspect relâché de la peau ; la réduction et/ou la prévention de l'asymétrie faciale ; l'augmentation du volume de tissu adipeux et/ou la prévention et/ou l'atténuation des effets de la perte de tissu adipeux.
